(19) Europäisches Patentamt
European Patent Office
Office européen des brevets

(11) Numéro de publication: **0 262 188**
**B1**

(12) # FASCICULE DE BREVET EUROPEEN

(45) Date de publication du fascicule du brevet: **14.11.90**

(21) Numéro de dépôt: **87902108.7**

(22) Date de dépôt: **26.03.87**

(86) Numéro de dépôt international:
**PCT/FR87/00096**

(87) Numéro de publication internationale:
**WO 87/05908 08.10.87 Gazette 87/22**

(51) Int. Cl.$^5$: **C 07 J 21/00,** C 07 J 31/00, C 07 J 33/00, C 07 J 41/00, C 07 J 43/00, C 07 J 71/00, A 61 K 31/58

(54) **STEROIDES COMPORTANT UN CYCLE SPIRANNIQUE EN POSITION 17, LEUR PROCEDE DE PREPARATION ET LEUR UTILISATION.**

(30) Priorité: **26.03.86 FR 8604355**

(43) Date de publication de la demande:
**06.04.88 Bulletin 88/14**

(45) Mention de la délivrance du brevet:
**14.11.90 Bulletin 90/46**

(84) Etats contractants désignés:
**AT BE CH DE FR GB IT LI LU NL SE**

(56) Documents cités:
**EP-A-0 057 115**
**EP-A-0 097 572**
**EP-A-0 156 284**
**US-A-3 509 135**
**US-A-3 764 596**

**Le dossier contient des informations techniques présentées postérieurement au dépôt de la demande et ne figurant pas dans le présent fascicule.**

(73) Titulaire: **ROUSSEL-UCLAF**
**35, boulevard des Invalides**
**F-75007 Paris (FR)**

(72) Inventeur: **NIQUE, François**
**7, allée Pierre Brossolette**
**F-93320 Les Pavillons sous Bois (FR)**
Inventeur: **NEDELEC, Lucien**
**45, boulevard de l'Ouest**
**F-93340 Le Raincy (FR)**
Inventeur: **PHILIBERT, Daniel**
**16, rue Chevalier**
**F-94210 La Varenne Saint Hilaire (FR)**
Inventeur: **MOGUILEWSKY, Martine**
**37, rue Lamartine**
**F-75009 Paris (FR)**

(74) Mandataire: **Fritel, Hubert et al**
**111, route de Noisy B.P. no 9**
**F-93230 Romainville (FR)**

**EP 0 262 188 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne de nouveaux stéroïdes comportant un cycle spirannique en position 17, leur procédé et des intermédiaires de préparation, leur application comme médicaments et les compositions pharmaceutiques les renfermant.

Dans le brevet européen EP 0.097.572 sont décrits des produits ne comportant pas, en position 17, un radical spirannique caractéristique des produits de la présente demande.

Dans le brevet européen EP 0.156.284 sont décrits des produits comportant en position 11, un atome d'hydrogène ou un radical alkyle, alkényle ou cycloalkyle ce que ne comportant pas les produits de la présente demande.

Dans le brevet européen EP 0.057.115 sont décrits des produits pouvant comporter de très nombreux substituants en position 17. Cependant les substituant spiranniques que comportent les produits de la présente demande ne sont pas mentionnés dans le brevet cité et confèrent aux produits de la présente demande une activité supérieure à celle des produits dudit brevet cité.

Dans les brevets américains USP 3,509,135 et 3,764,596 sont décrits des produits ne comportant pas de substituants en position 11.

L'invention a pour objet les produits de formule (I):

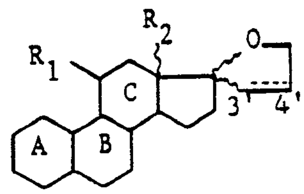

(I)

dans laquelle $R_1$ représente soit un radical phényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle, chacun de ces radicaux étant éventuellement substitué par un ou plusieurs radicaux choisis parmi:

les radicaux alkyle ayant de 1 à 8 atomes de carbone;

les radicaux alkoxy ayant de 1 à 8 atomes de carbone;

les radicaux alkényloxy choisis parmi les radicaux vinyloxy ou allyloxy;

les atomes d'halogène;

les radicaux hydroxyle, trifluorométhyle, acyle ayant de 1 à 6 atomes de carbone, carboxy éventuellement estérifié;

les radicaux alkylthio ayant de 1 à 8 atomes de carbone éventuellement oxydé sous forme de sulfoxyde ou de sulfone;

les radicaux amino, amino mono ou disubstitués par des radicaux alkyles ayant de 1 à 8 atomes de carbone et éventuellement oxydés en N-oxyde et le radical bis (chloroéthyl) amino;

les radicaux diméthylamino méthyle, diméthylamino éthyle, méthyl (diméthylamino éthyl) amino, diméthylamino éthoxy, morpholino ou pipéridinyle;

soit $R_1$ représente les radicaux:

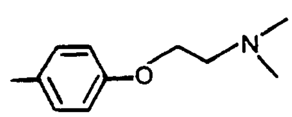
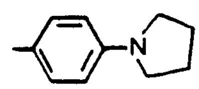
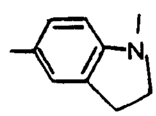
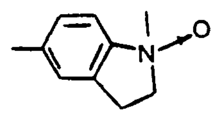

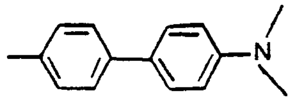
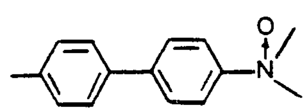
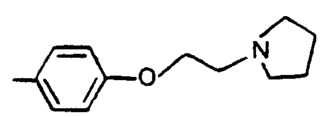

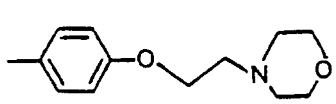
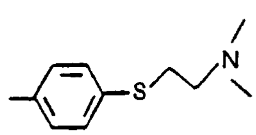
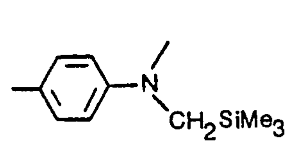

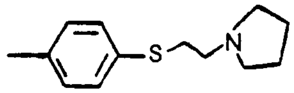
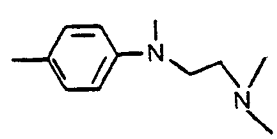
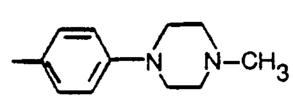

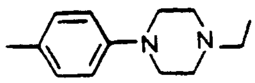 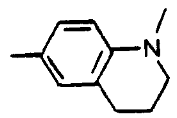 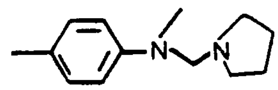 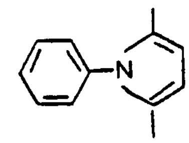

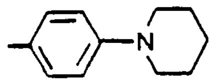 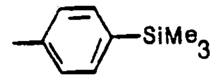 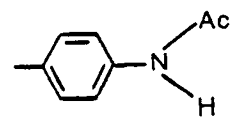

$R_2$ en position alpha ou béta, représente un radical hydrocarboné renfermant de 1 à 18 atomes de carbone, le trait ondulé du spiro éther indique que l'atome d'oxygène peut se trouver en position alpha ou béta, le trait pointillé en position 3', 4' indique la présence éventuelle d'une seconde liaison entre les carbones qui le portent, les cycles A et B ayant l'une des structures suivantes:

a) — soit A et B représentent le groupement:

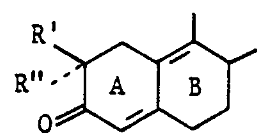

dans lequel R' et R'' identiques ou différents représentent un atome d'hydrogène, ou un radical alkyle ayant de 1 à 4 atomes de carbone;

b) — soit A et B représentent le groupement:

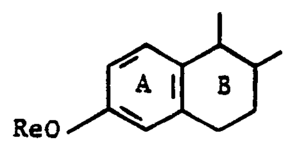

dans lequel Re représente un atome d'hydrogène, un radical alkyle ayant de 1 à 6 atomes de carbone éventuellement substitué ou un radical acyle;

c) — soit A, B et C représentent le groupement:

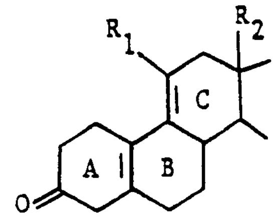

d) — soit A et B représentent le groupement:

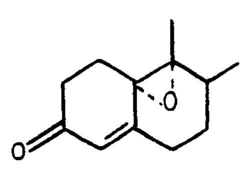

e) — soit A et B représentent le groupement:

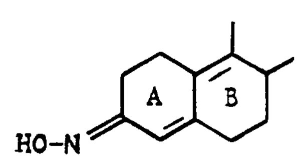

ainsi que leurs sels.

L'invention a notamment pour objet, parmi les produits de formule (I), les produits répondant à la formule (I'):

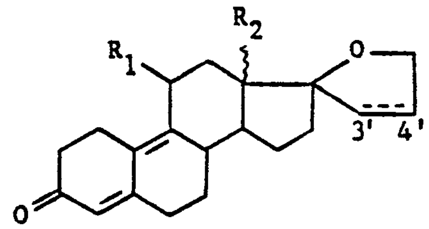

(I')

dans laquelle $R_1$ a la signification précédente , $R_2$ en position alpha ou béta, représente un radical hydrocarboné renfermant de 1 à 18 atomes de carbone, l'atome d'oxygène du spiro éther est en position béta, le trait pointillé en position 3', 4' indique la présence éventuelle d'une seconde liaison entre les carbones qui le portent ainsi que leurs sels.

Lorsque $R_1$ représente un radical aryle ou aralkyle carbocyclique éventuellement substitué, il s'agit de préférence du radical phényle ou benzyle. Ces radicaux aromatiques peuvent être substitués en ortho, méta ou para par un ou plusieurs radicaux alkyles renfermant de préférence de 1 à 8 atomes de carbone; par un ou plusieurs radicaux alkoxy ayant préférentiellement de 1 à 8 atomes de carbone tels que les radicaux méthoxy, éthoxy, propyloxy, isopropyloxy, butyloxy, isobutyloxy, tert-butyloxy; alkényloxy tel que vinyloxy ou allyloxy, tous ces radicaux étant éventuellement substitués; par un ou plusieurs atomes d'halogène, tels que fluor, chlore, brome, iode, de préférence chlore ou fluor; par un ou plusieurs radicaux choisis parmi les radicaux hydroxyle, trifluorométhyle, acyle ayant de 1 à 6 atomes de carbone tel que acétyle, propionyle, carboxy éventuellement estérifié tel que méthoxycarbonyl, éthoxycarbonyl, propoxycarbonyl, alkylthio ayant de 1 à 8 atomes de carbone tel que méthylthio, éthylthio éventuellement oxydé sous forme de sulfoxyde ou de sulfone; par un ou plusieurs radicaux amino ou amino mono ou disubstitués par des radicaux alkyles comportant de 1 à 8 atomes de carbone, eux-mêmes éventuellement substitués tels que les radicaux méthylamino, diméthylamino et bis (chloroéthyl) amino, les radicaux amino ou amino mono ou disubstitués étant éventuellement oxydés en N-oxyde, les radicaux amino incorporés dans un hétérocycle comportant éventuellement un hétéroatome choisi dans le groupe formé par l'oxygène, l'azote et le soufre tel que les radicaux morpholino ou pipéridinyle; bien entendu les radicaux aryle ou aralkyle peuvent être substitués par une combinaison de ces différents radicaux tel que par exemple 2-méthylthioéthoxy, 3-fluoro, 4-diméthylamino; $R_1$ peut également représenter un radical aryle hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridinyle ou pipéridinyle et les hétérocycles connus de l'homme de métier.

Comme substituant sur le noyau arylique, on peut également envisager un radical amino (substitué) alkyle tel que le radical diméthylamino méthyle, diméthylamino éthyle, méthyl (diméthylaminoéthyl) amino; un radical amino (substitué) alkoxy tel que le radical diméthylamino éthyloxy.

On peut également citer les radicaux comportant un atome de silicium tel que le radical triméthylsilyl phényle.

Les radicaux prédécemment cités comportant un atome d'azote ou de soufre peuvent être oxydés.

De manière générale, on préfère les produits dans lesquels le substituant $R_1$ comporte un hétéroatome de préférence l'azote ou le soufre.

Parmi les différents radicaux $R_1$, on peut citer les radicaux suivants:

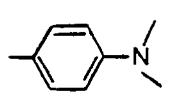 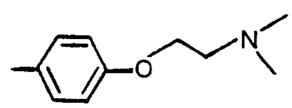 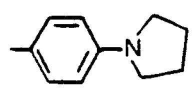 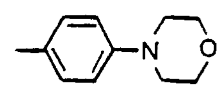

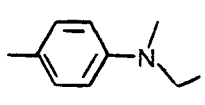 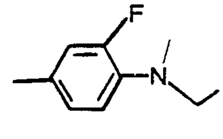 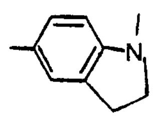 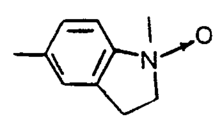

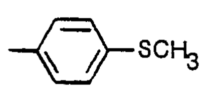 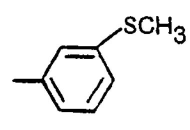 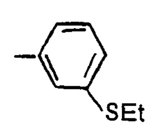 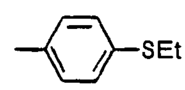 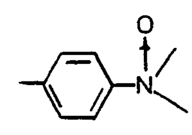

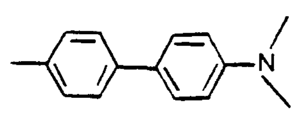 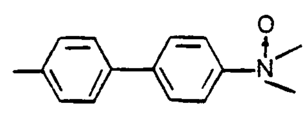 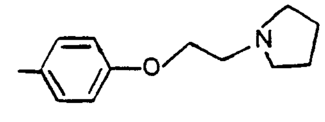

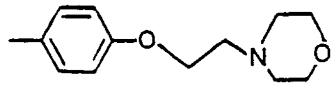 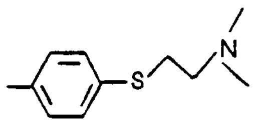 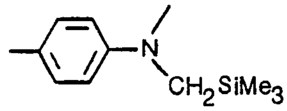

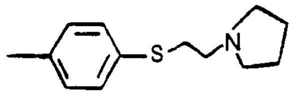 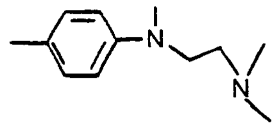 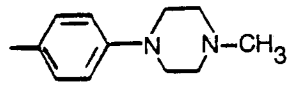

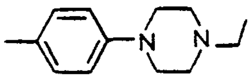 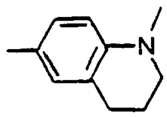 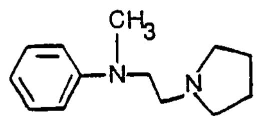 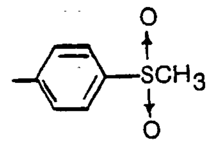

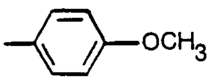 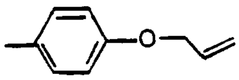 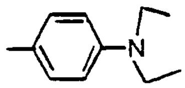 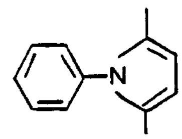

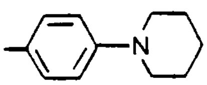 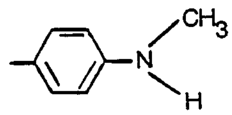 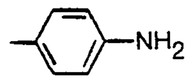 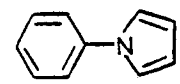

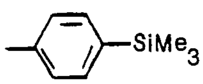 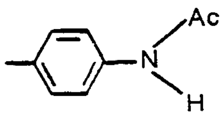 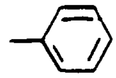

Le radical $R_2$ représente de préférence un radical alkyle saturé, linéaire ou ramifié, renfermant de 1 à 4 atomes de carbone, par exemple un radical méthyle, éthyle, propyle ou butyle.

Préférentiellement $R_2$ représente un radical méthyle ou éthyle. Plus préférentiellement $R_2$ représente un radical méthyle.

Le radical $R_2$ peut être en position alpha ou béta. On préfère les produits dans lesquels $R_2$ est en position béta.

Lorsque $R_1$ comporte une fonction carboxy, celle-ci peut être salifiée. Parmi les sels possibles, on peut citer, par exemple, les sels de sodium, de potassium, de lithium, de calcium, de magnésium ou d'ammonium. On peut citer, par les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Lorsque $R_1$ comporte une fonction salifiable par un acide et notamment une fonction amino, on obtient des sels d'addition avec les acides.

L'invention s'étend naturellement aux sels d'addition avec les acides des composés de formule (I) salifiables, comme par exemple les sels formés avec les acides chlorhydrique, bromhydride, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits de formule (I), on préfère les produits dans lesquels l'atome d'oxygène du spiroéther est en position 17 béta et les cycles A et B représentent le groupement:

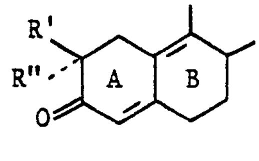

dans lequel R' et R'' représentent un atome d'hydrogène.

Parmi les produits de formule I, on préfère les produits dans lesquels $R_1$ représente soit un radical aryle ou aralkyle portant une fonction amine

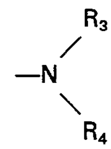

dans laquelle $R_3$ et $R_4$ représentent chacun un radical alkylé primaire, secondaire ou tertiaire renfermant de 1 à 8 atomes de carbone ainsi que leurs sels.

$R_3$ et $R_4$ peuvent être identiques ou différents et représenter notamment les valeurs méthyle, éthyle, propyle, isopropyle, butyle, terbutyle, ou $R_3$ et $R_4$ peuvent notamment représenter avec l'atome qui les porte les radicaux morpholinyle, imadazolidinyle, pyrrolidinyle, pipéridinyle, pipérazinyle.

On retient également particulièrement les produits de formule (I) dans laquelle le radical aryle est un radical phényle et le substituant porté par le radical phényle est en position para, ainsi que leurs sels.

Parmi les valeurs de $R_1$, on préfère les valeurs suivantes:

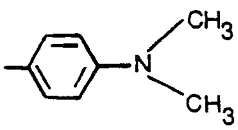 ; 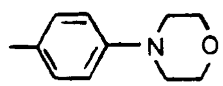 ; 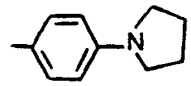 ; 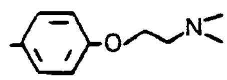

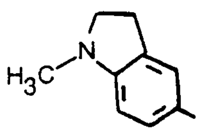 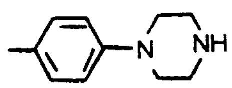 ; 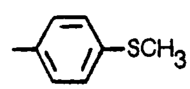 et 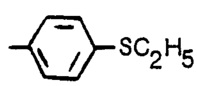

et plus particulièrement encore les valeurs:

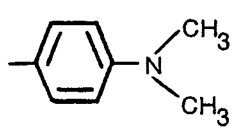 et 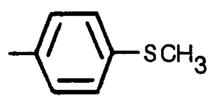

Parmi les produits de formule I, on préfère les produits dans lesquels le substituant $R_2$ représente un radical méthyle en position alpha ou béta ou un radical éthyle en position béta.

L'invention a plus particulièrement pour objet les produits décrits ci-après dans les exemples dont les noms suivent:

(17 R) 4', 5'-dihydro 11 béta-[4-(diméthylamino) phényl] spiro (estra-4,9-dièn-17,2'(3 H) furan) 3-one,

(17 R) 11 béta- [4-(diméthylamino) phényl] spiro (estra-4,9-dièn-17, 2'-(5 H) furan) 3-one,

(17 R) 11 béta- [4-(méthylthio) phényl] spiro (estra-4,9-dièn-17, 2'-(5 H) furan) 3-one,

(17 R) 4', 5'-dihydro 11 béta-[4-(méthylthio) phényl] spiro (estra-4,9-dièn-17, 2'-(5 H) furan) 3-one,

(17 R) 11 béta- [4-(1-pyrrolidinyl) phényl] spiro (estra-4,9-dièn-17, 2'-(5 H) furan) 3-one,

(17 R) 4', 5'-dihydro 11 béta-[4-(1-pyrrolidinyl) phényl] spiro (estra-4,9-dièn-17,2'-3 H) furan) 3-one,

(17 R) 4', 5'-dihydro 11 béta-[ (2,3-dihydro 1-méthyl) (1H) indol-5-yl spiro (estra-4,9-dièn-17,2'-(3 H) furan) 3-one,

(17 R) 11 béta- [4-(éthylthio) phényl] spiro (estra-4,9-dièn-17, 2'-(5H) furan) 3-one, ainsi que leurs sels.

L'invention a également pour objet un procédé de préparation des produits de formule I telle que définie ci-dessus caractérisé en ce que

a) pour préparer les produits de formule ($I_A$):

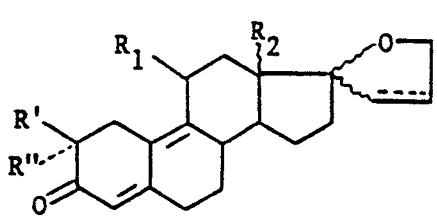

($I_A$)

dans laquelle $R_1$ et $R_2$ conservent la même signification que précédemment et R' et R'' représentent chacun un atome d'hydrogène ou un radical alkyle ou bien l'un représente un atome d'hydrogène et l'autre représente un radical alkyle, l'on soumet soit un produit de formule (II):

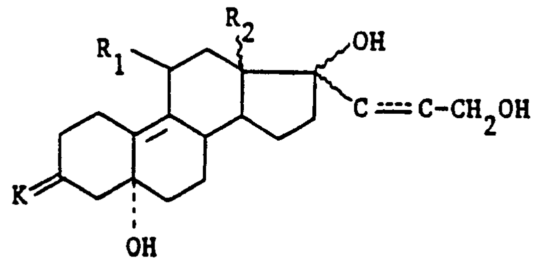

(II)

*soit* un produit de formule (III):

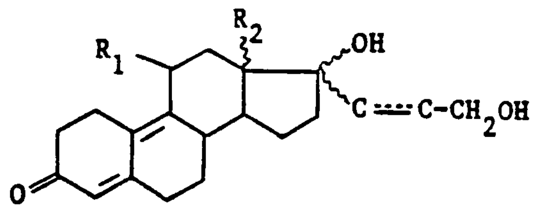

(III)

produits dans lesquels $R_1$ et $R_2$ ont la signification indiquée précédemment et K représente un groupement protecteur du radical cétone, à l'action d'un réactif de cyclisation pour obtenir respectivement, *soit* les produits de formule (IV):

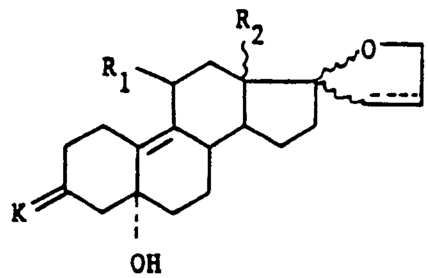

(IV)

les produits de formule (IV) que l'on soumet à l'action d'un réactif de déshydratation également susceptible de libérer la fonction cétone pour obtenir les produits de formule ($I_A$), dans laquelle R′ et R″ représentent un atome d'hydrogène, *soit* lesdits produits de formule ($I_A$) et que, si désiré, l'on soumet à une oxydation les produits de formule ($I_A$) dans laquelle $R_1$ comporte un atome de soufre ou d'azote pour obetenir les produits dans lesquels $R_1$ comporte un atome de soufre oxydé en sylfoxyde ou en sulfone ou un atome d'azote oxydé en N-oxyde et que, si désiré l'on soumet les produits de formule ($I_A$) à une salification, ou si désiré soumet les produits de formule ($I_A$) à l'action d'une base forte puis d'un halogénure d'alkyle pour obtenir un produit de formule ($I_A$) dans laquelle R′ et/ou R″ représentent un radical alkyle ayant de 1 à 4 atomes de carbone;

b) pour préparer les produits de formule ($I_B$):

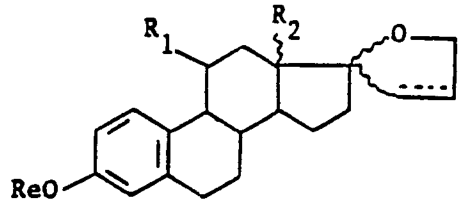

($I_B$)

dans laquelle $R_1$, $R_2$ et Re conservent la même signification que ci-dessus, ou soumet un produit de formule ($I'_A$):

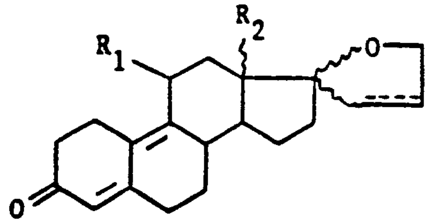

($I'_A$)

dans laquelle $R_1$ et $R_2$ ont la signification déjà indiquée, à l'action d'un agent d'aromatisation puis le cas échéant à l'action d'un agent de saponification et enfin si désiré soumet le produit de formule ($I_B$) dans laquelle Re est un atome d'hydrogène à un réactif d'alkylation ou d'acylation;

c) pour préparer les produits de formule ($I_C$):

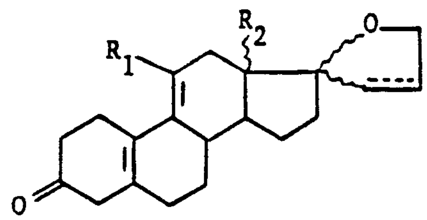

($I_C$)

dans laquelle $R_1$ et $R_2$ conservent la même signification que ci-dessus, on soumet *soit* un produit de formule (V):

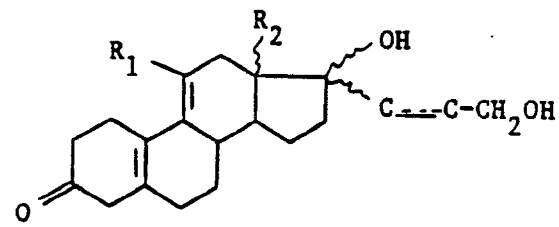

(V)

à l'action d'un agent de cyclisation, *soit* un produit de formule ($I'_A$) à l'action d'un agent d'acylation puis de saponification;

d) pour préparer les produits de formule ($I_D$):

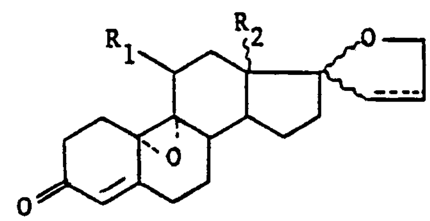

($I_D$)

dans laquelle $R_1$ et $R_2$ conservent la même signification que ci-dessus, on soumet un produit de formule ($I'_A$) à l'action d'un agent d'époxydation)

e) pour préparer les produits de formule ($I_E$):

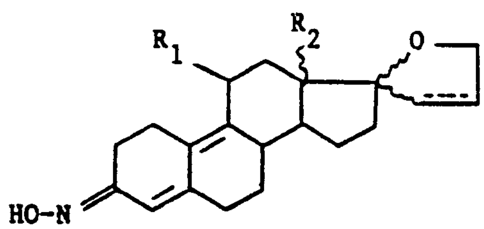

($I_E$)

dans laquelle $R_1$ et $R_2$ conservent la même signification que ci-dessus, on fait agir l'hydroxylamine sur un produit de formule ($I'_A$).

L'invention a également pour objet un procédé de préparation des produits de formule (I) dans laquelle $R_1$ représente un radical aryle ou aralkyle substitué par un radical carboxy éventuellement estérifié ou salifié, caractérisé en ce que l'on soumet un produit de formule ($I_F$):

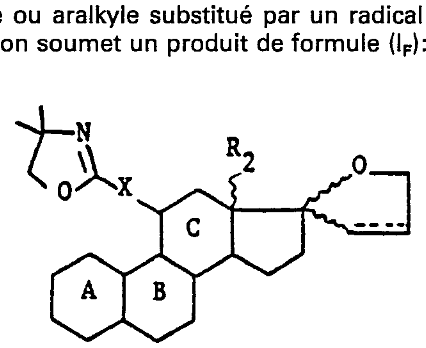

($I_F$)

dans laquelle A, B, C et $R_2$ ont la signification déjà indiquée et X représente un radical aryle ou aralkyle carbocyclique ou hétérocyclique à une hydrolyse acide pour obtenir un produit de formule ($I_G$):

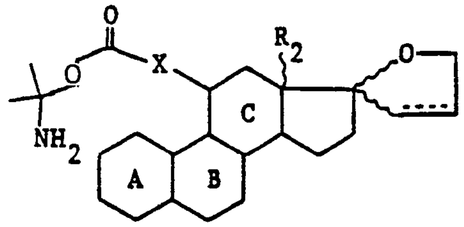

($I_G$)

que le cas échéant, l'on soumet à l'action d'un agent basique pour obtenir un produit de formule ($I_H$):

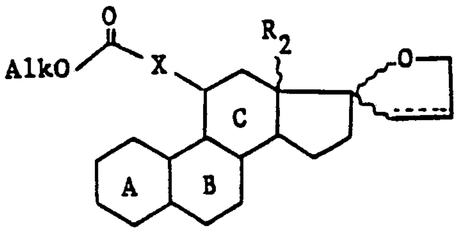

($I_H$)

dans laquelle alk représente un radical alkyle ayant de 1 à 6 atomes de carbone, que le cas échéant, l'on saponifie pour obtenir un produit de formule ($I_J$):

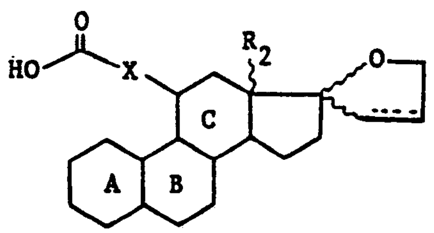

($I_J$)

que le cas échéant, l'on estérifie ou salifie.

Dans un mode préférentiel d'exécution des procédés ci-dessus décrits, le réactif de cyclisation que l'on fait agir de préférence sur les produits de formule (II), (III) ou (V), est le chlorure de tosyle en présence de pyridine; on peut également utiliser le chlorure de méthylsulfonyle.

La transformation des produits de formule IV, à l'aide d'un réactif de déshydratation également susceptible de libérer la fonction cétone, est effectuée de préférence à l'aide d'une résine sulfonique (forme acide) par exemple, une résine sulfonique du commerce à support de polystyrène ou à support de polymère styrène/divinyl/benzène. On peut cependant utiliser un acide minéral tel que l'acide chlorhydrique ou l'acide sulfurique dans un alcanol inférieur ou l'acide perchlorique dans l'acide acétique ou un acide sulfonique comme l'acide paratoluène sulfonique.

L'agent d'oxydation que l'on fait agir sur les produits de formule ($I_A$) ou l'agent d'époxydation que l'on fait agir pour obtenir les produits de formule ($I_D$) est de préférence un peracide tel que l'acide métachloroperbenzoïque, l'acide peracétique ou l'acide perphtalique. On peut également utiliser l'eau oxygénée seule ou en présence d'hexachloro ou d'hexafluoro acétone.

Bien entendu selon le nombre de fonctions pouvant faire l'objet d'une oxydation, on peut utiliser un ou plusieurs équivalents d'agent oxydant.

C'est ainsi, par exemple, que si l'on veut oxyder l'atome de soufre que comporte $R_1$ en sulfone, on doit bien entendu utiliser au moins deux équivalents d'agent oxydant.

La base forte que l'on utilise sur les produits de formule ($I_A$) peut être un amidure de métal alcalin, tel que l'amidure de sodium ou de lithium éventuellement préparé in situ;

l'halogénure d'alkyle que l'on utilise est de préférence un iodure comme l'iodure de méthyle;

l'agent d'aromatisation utilisé pour préparer les produits de formule ($I_B$) est de préférence un halogénure d'acyle tel que le bromure d'acétyle ou un anhydride d'acide tel que l'anhydride acétique ou un mélanane des deux;

l'acylation éventuelle des produits de formule ($I_B$) et l'acylation conduisant aux produits de formule ($I_C$) est réalisée selon les méthodes usuelles, on utilise de préférence un halogénure d'acyle;

l'alkylation éventuelle des produits de formule ($I_B$) est effectuée selon les méthodes usuelles. On utilise par exemple un halogénure d'alkyle;

l'agent de saponification que l'on utilise pour obtenir les produits de formule ($I_B$), ($I_C$) ou ($I_J$) est de préférence une base alcaline comme la soude ou la potasse et la réaction est réalisée au sein d'un alcool inférieur tel que le méthanol ou l'éthanol;

l'oximation des produits de formule ($I'_A$) est effectuée en utilisant l'hydroxylamine sous forme de sel de

préférence le chlorhydrate au sein d'un alcool à la température du reflux;

l'hydrolyse acide à laquelle on soumet les produits de formule ($I_F$) est effectuée selon les conditions usuelles; on peut par exemple utiliser un acide minéral l'acide chlorhydrique de préférence en solution aqueuse;

l'agent basique utilisé pour obtenir les produits ($I_H$) est de préférence un alcoolate alcalin tel que l'éthylate de sodium.

La salification est effectuée dans des conditions usuelles. On peut opérer, par exemple, en présence de soude éthanolique. On peut également utiliser un sel de sodium tel que le carbonate ou le carbonate acide de sodium ou de potassium.

De même, la salification par un acide est réalisée dans les conditions usuelles. On opère de préférence avec l'acide chlorhydrique, par exemple en solution éthérée.

Les produits de formule (I) ainsi que leurs sels pharmaceutiquement acceptables sont des produits particulièrement intéressants du point de vue pharmacologique.

L'étude des produits sur les récepteurs hormonaux a permis de mettre en évidence des activités progestomimétiques ou anti progestomimétiques, androgènes ou antiandrogènes.

Les produits de formule (I) possèdent en particulier une remarquable activité antiprogestomimétique.

Les produits de formule (I) possèdent également une activité antiglucocorticoide comme le montrent les résultats des tests exposés ci-après.

Certains produits montrent cependant une activité antiprogestomimétique supérieure à leur propriété antiglucocorticoïde.

Les produits de formule (I) ainsi que leurs sels pharmaceutiquement acceptables qui possèdent des propriétés antiprogestomimétiques peuvent être utilisés comme contraceptifs; ils peuvent être utilisés contre les dérèglements hormonaux.

Certains produits de formule (I) ansi que leurs sels pharmaceutiquement acceptables peuvent également présenter des propriétés progestomimétiques et peuvent ainsi être employées dans le traitement des aménorrhées, des dysménorrhées et des insuffisances lutéales.

Les produits de formule (I) ainsi que leurs sels pharmaceutiquement acceptables peuvent donc être utilisés comme médicaments pour lutter principalement contre les effets secondaires des glucocorticoïdes, ils permettent de lutter également contre les troubles dus à une hypersécrétion de glucocorticoïdes et notamment contre le vieillisement en général et plus particulièrement contre l'hypertension, l'athérosclérose, l'ostéoporose, le diabète, l'obésité ainsi que l'immunodépression et l'insomnie.

Les produits de formule (I) ainsi que leurs sels pharmaceutiquement acceptables qui présentent des propriétés antiandrogènes peuvente être utilisés dans le traitement des hypertrophies et du cancer de la prostate, de l'hyperandrogènie, de l'anémie, de l'hirsutisme et de l'acné.

Les produits de formule (I) ainsi que leurs sels pharmaceutiquement acceptables possèdent également des propriétés anti-prolifératives qui les rendent utilisables dans le traitement des cancers hormono-dépendants notamment les carcinomes mammaires et leurs métastases. Ces propriétés les rendent également utilisables dans le traitement des tumeurs bénignes.

Certains des produits de formule (I) ainsi que leurs sels pharmaceutiquement acceptables possèdent des propriétés estrogènes et/ou anti-estrogènes. Les propriétés anti-estrogènes les rendent utilisables dans le traitement des cancers estrogéno dépendants.

Les propriétés estrogènes que peuvent également présenter lesdits produits de formule (I) ainsi que leurs sels pharmaceutiquement acceptables les rendent utilisables également dans le traitement des troubles liés à une hypofolliculinie, par exemple les aménorrhées, les dysménorrhées, les avortements répétés, les troubles prémenstruals ainsi que le traitement de la ménopause.

L'invention a donc pour objet à titre de médicament les produits de formule I pharmaceutiquement acceptables, c'est-à-dire non toxiques aux doses utilisées, ainsi que leurs sels pharmaceutiquement acceptables.

L'invention a particulièrement pour objet, à titre de médicaments, les produits de formule (I) préférés mentionnés plus haut et tout particulièrement les produits dont les noms suivent:

(17 R) 4', 5'-dihydro 11 béta-[4-(diméthylamine) phényl] spiro (estra-4,9-dièn 17,2'(3H) furan) 3-one,

(17 R) 11 béta- [4-(diméthylamino) phényl] spiro (estra-4,9-dièn 17, 2'(5H) furan) 3-one,

(17 R) 11 béta- [4-(méthylthio) phényl] spiro (estra-4,9-dièn-17, 2'(5H) furan) 3-one,

(17 R) 4', 5'-dihydro 11 béta-[4-(méthylthio) phényl] spiro (estra-4,9-dièn 17, 2'(5H) furan) 3-one,

(17 R) 11 béta- [4-(1-pyrrolidinyl) phényl] spiro (estra-4,9-dièn 17, 2'(5H) furan) 3-one,

(17 R) 4', 5'-dihydro 11 béta-(4-(1-pyrrolidinyl) phényl] spiro (estra-4,9-dièn 17,2'(3H) furan) 3-one,

(17 R) 4', 5'-dihydro 11 béta-(4-(2,3-dihydro 1-méthyl) (1H) indol-5-yl spiro (estra-4,9-dièn-17,2'(3 H) furan) 3-one,

(17 R) 11 béta- (4-(éthylthio) phényl] spiro (estra 4,9-dièn 17, 2' (5H) furan) 3-one, ainsi que leurs sels pharmaceutiquement acceptables.

La posologie utile varie en fontion de l'affection à traiter et de la voie d'administration; elle peut varier par exemple de 10 mg à 1 g par jour chez l'adulte par voie orale.

Les nouveaux produits de formule I, et leurs sels, tels que définis ci-dessus peuvent être employés pour préparer des compositions pharmaceutiques renfermant, à titre de principe actif, l'un au moins desdits produits.

Les produits de formule (I) et leurs sels sont utilisés par voie digestive, parentérale ou locale. Ils peuvent être prescrits sous forme de comprimés simples ou dragéifiés, de gélules, de granulés, de suppositories et de préparation injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agent mouillants, dispersants ou émulsifiants, les conservateurs.

L'invention a donc pour objet les compositions pharmaceutiques renfermant comme principe actif au moins un produit de formule (I), ou au moins un de leurs sels pharmaceutiquement acceptables.

Les produits de formules (II), (III) et (V) sont connus ou peuvent être préparés par les méthodes usuelles telles que celles décrites dans le brevet européen EP 0.147.361.

Les produits de formule (II), (III) et (V) dont l'atome d'oxygène du spiroéther est en position alpha peuvent être préparés selon la méthode indiquée dans le brevet européen EP 0.129.499.

L'invention a également pour objet à titre de produits industriels nouveaux, les produits de formule (IV):

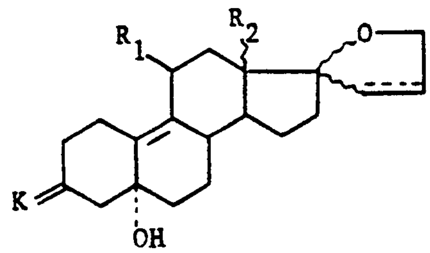

(IV)

dans laquelle $R_1$, $R_2$ et K conservent la même signification que précédemment.

En plus des exemples suivants qui illustrent l'invention sans toutefois la limiter, les produits suivants constituent des produits pouvant être obtenus dans le cadre de la présente invention.

Les produits de formule (I):

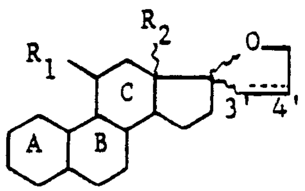

(I)

dans laquelle A, B, C on la valeur indiquée précédemment et $R_1$, $R_2$ et la liaison 3'—4' ont la valeur suivante:

| $R_1$ | $R_2$ | 3′ 4′ |
|---|---|---|
| 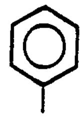 | β $CH_3$ | C–C |
| " | " | C=C |
| 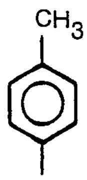 | " | C–C |
| " | " | C=C |
| 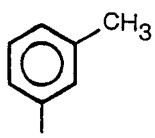 | " | C–C |
| " | " | C=C |
| 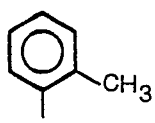 | " | C–C |
| " | " | C=C |
| 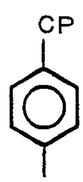 | " | C–C |
| " | " | C=C |
| 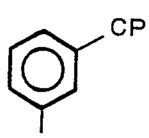 | " | C–C |
| " | " | C=C |
| 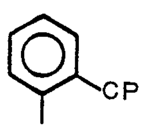 | " | C–C |
| " | " | C=C |
| 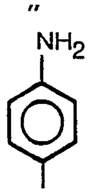 | " | C–C |

| $R_1$ | $R_2$ | 3′ 4′ |
|---|---|---|
| 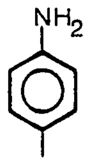 | β $CH_3$ | C=C |
| 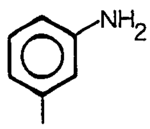 | " | " |
| " | " | C=C |
| 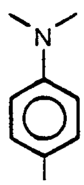 | β $C_2H_5$ | C–C |
| " | " | C=C |
| " | α $CH_3$ | C–C |
| " | " | C=C |
| 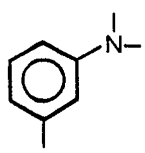 | β $CH_3$ | C–C |
| " | " | C=C |
| 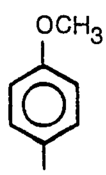 | " | C–C |
| " | " | C=C |
| 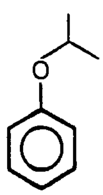 | " | C–C |
| " | " | C=C |
| 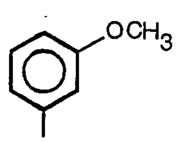 | " | C–C |
| " | " | C=C |
| 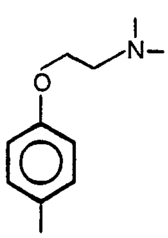 | " | C–C |

| $R_1$ | $R_2$ | 3′ 4′ |
|---|---|---|
| 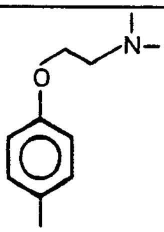 | β $CH_3$ | C=C |
| 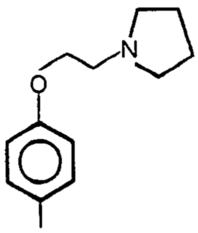 | " | C–C |
| " | " | C=C |
| 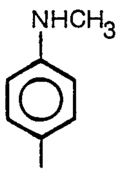 | " | C–C |
| " | " | C=C |
| 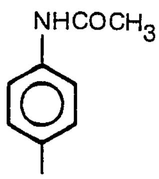 | " | C–C |
| " | " | C=C |
| 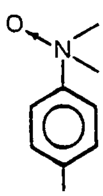 | " | C–C |
| " | " | C=C |
| 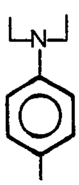 | " | C–C |
| " | " | C=C |
| 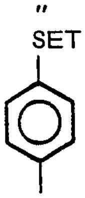 | " | C–C |
| " | " | C=C |
| 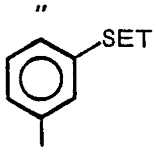 | " | C–C |

| $R_1$ | $R_2$ | 3′ 4′ |
|---|---|---|
| 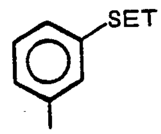 | β $CH_3$ | C=C |
| 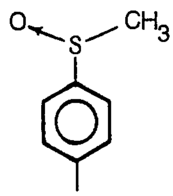 | " | C–C |
| " | " | C=C |
| 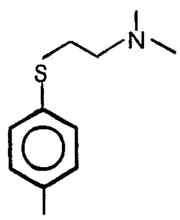 | " | C–C |
| " | " | C=C |
| 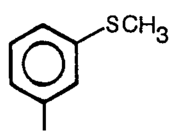 | " | C–C |
| " | " | C=C |
| 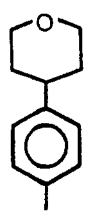 | " | C–C |
| " | " | C=C |
| 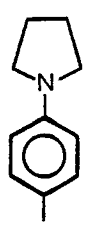 | " | C–C |
| " | " | C=C |
| 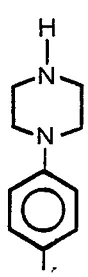 | " | C–C |
| " | " | C=C |

| $R_1$ | $R_2$ | 3′ 4′ |
|---|---|---|
| 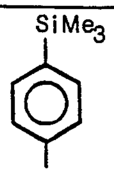 | β $CH_3$ | C–C |
| " | " | C=C |
| 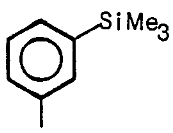 | " | C–C |
| " | " | C=C |
| 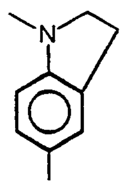 | " | C–C |
| " | " | C=C |
| 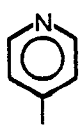 | " | C–C |
| " | " | C=C |
| 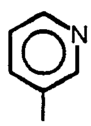 | " | C–C |
| " | " | C=C |
| 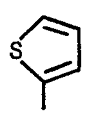 | " | " |
| 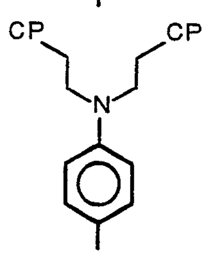 | " | C–C |
| " | " | C=C |
| 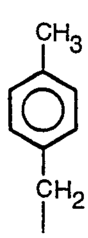 | " | C–C |
| " | " | C=C |

| $R_1$ | $R_2$ | 3' 4' |
|---|---|---|
| 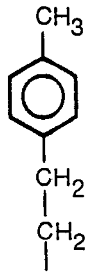 | β $CH_3$ | C−C |
| " | " | C=C |
| 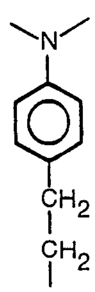 | " | C−C |
| " | " | C=C |

Exemple 1

(17 R) 4', 5'-dihydro 11 β-/4-(diméthylamino) phényl/spiro (estra 4,9-dién-17,2' (3 H) furan) 3-one

*Stade A:* γ-lactone de l'acide 5α, 17β-dihydroxy 11β-/4-(diméthylamino) phényl/ 3,3-/(1,2-éthane diyl) bisoxy/ 19-nor 17α-pregn-9-èn-21-carboxylique

Dans 60 $cm^3$ d'une solution de butyllithium à 15% dans l'hexane (1,6 M) on introduit à −70°C 60 $cm^3$ de tétrahydrofuranne, puis goutte à goutte à −60°C on introduit 9,2 $cm^3$de N,N,N',N'- tétraméthyl phosphoramidate d'allyle en solution dans 30 $cm^3$ de tétrahydrofuranne, agite penant 45 minutes à −10°C, ajoute 9,95 g de 3,3-(1,2-éthane diyl) acétal cyclique de 11β-/4-(diméthylamino) phényl/ 5α-hydroxy estr-9-èn 3,17-dione en solution dans 20 $cm^3$ de tétrahydrofuranne, on ajoute encore 20 $cm^3$ de tétrahydrofuranne et agite pendant 1 heure à 20°C.

On verse le mélange réactionnel dans une solution aqueuse de chlorure d'ammonium, extrait à l'acétate d'éthyle, lave à l'eau, sèche, concentre à sec par distillation sous pression réduite, chromatographie le résidu sur silice en éluant par un mélange de cyclohexane et d'acétate d'éthyle (3/7) et obtient 3,9 g de produit recherché brut que l'on dissout dans le chlorure de méthylène, on filtre, ajoute de l'éther isopropylique au filtrat, élimine le chlorure de méthylène par distillation, essore, lave et obtient 3,45 g de produit recherché, F = 198°C.

*Spectre IR* (chloroforme)
OH associé 3510$^{cm-1}$
γ lactone 1760$^{cm-1}$ cétal,
bandes aromatiques 1610$^{cm-1}$, 1560$^{cm-1}$, 1516$^{cm-1}$, 823$^{cm-1}$.

*Analyse:* $C_{31}H_{41}O_5$ N (507,67)

| | C% | H% | N% |
|---|---|---|---|
| Calculés | 73,34 | 8,14 | 2,76 |
| Trouvés | 73,1 | 8,3 | 2,8 |

*Stade B:* (1,2-éthanediyl) acétal cyclique de 5α, 17β-dihydroxy 11β-/4-(diméthylamino) phényl/ 17α-(3)-hydroxypropyl) estr 9-èn 3-one

On dissout 1,014 g de produit obtenu ci-dessus dans 30 $cm^3$ de tétrahydrofuranne, ajoute, par fractions, sous balayage d'azote, en agitant 500 mg d'hydrure de lithium aluminium, la température s'élève jusqu'à 35°C, agite pendant 1 heure et 30 minutes à 20°C, ajoute goutte à goutte de l'acétate d'éthyle puis une solution aqueuse saturée de chlorure d'ammonium, on décante alors la phase organique surnageante et extrait le résidu en l'agitant avec un mélange tétrahydrofuranne-acétate d'éthyle (1/1), lave les phases organiques à l'eau salée, sèche, concentre à sec par distillation sous pression réduite et obtient 993 mg de produit recherché brut (pF 210°C), utilisé tel quel pour le stade suivant.

L'échantillon analytique obtenu après purification chromatographique sur silice en éluant par un mélange acétate d'éthyle éthanol (95/5), puis cristallisation dans l'éthanol fond à 234°C.

*Spectre IR* (chloroforme)
OH à $3620^{cm^{-1}}$ et OH associé.
bandes aromatiques à $1613^{cm^{-1}}$, $1560^{cm^{-1}}$, $1517^{cm^{-1}}$, présence de cétal.

*Analyse:* $C_{31}H_{45}O_5N$ 511,7

| | C% | H% | N% |
|---|---|---|---|
| Calculés | 72,76 | 8,86 | 2,73 |
| Trouvés | 72,6 | 9,0 | 2,7 |

*Stade C:* Mélange de (1,2-éthanediyl) acétal cyclique de (17R) 4',5'-dihydro 11β-/4-(diméthylamino) phényl/ 5α-hydroxy spiro (estr 9-èn-17,2' (3H) furan) 3-one (composé A) et de (17 R) 4',5'-dihydro 11β-/4-(diméthylamino) phényl/ spiro (estra-4,9-dièn-17,2' (3H) furan) 3-one (composé B).

On dissout 2 g du composé ci-dessus dans 30 $cm^3$ de pyridine, ajoute, à +3°C, 1,52 g de chlorure de tosyle, abandonne pendant 40 heures à 20°C, refroidit le milieu réactionnel à +3°C, ajoute de l'eau puis une solution de bicarbonate de sodium, extrait à l'acétate d'éthyle, lave à l'eau, sèche concentre à sec par distillation sous pression réduite, élimine la pyridine par un entrainement azéotropique avec du toluène, chromatographie la résidu sur silice en éluant par un mélange cyclohexanne-acétate d'éthyle (7/3) et obtient 395 mg de composé (B) et 500 mg de composé (A).

*Contrôles du composé A:*
*Spectre IR* (chloroforme):
absence de bande CO, 50H $3512^{cm^{-1}}$
aromatiques $1613^{cm^{-1}}$, $1557^{cm^{-1}}$, $1517^{cm^{-1}}$.

*Stade D:* (17 R) 4', 5'-dihydro 11 β-/4-diméthylamino/ phényl/ spiro (estra-4,9-dièn-17,2' (3H) furan) 3-one (composé B).

Les 300 mg de composé A obtenus au stade C sont dissous dans 15 $cm^3$ d'éthanol, ajoute 10 $cm^3$ de solution aqueuse 2N d'acide chlorhydrique, on abandonne pendant 45 minutes à 20°C, ajoute une solution aqueuse de bicarbonate de sodium, extrait au chlorure de méthylène, lave, sèche, concentre à sec par distillation sous pression réduite et obtient 390 mg de composé B brut.

*Purification du composé B:*

On réunit les 395 mg de composé B obtenus au stade C et les 390 mg de composé B obtenus ci-dessus, chromatographie sur silice en éluant avec un mélange cyclohexane-acétate d'éthyle (7/3) et obtient 645 mg de composé B.

Après recristallisation dans l'éthanol aqueux, on obtient 367 mg de produit attendu F = 100°C (peu net).

*Composé B:*
*Spectre IR* (chloroforme):
absence d'OH; cétone en 3 $1653^{cm^{-1}}$;
C = C, aromatique $1612^{cm^{-1}}$, $1597^{cm^{-1}}$, $1560^{cm^{-1}}$, $1518^{cm^{-1}}$.

*Spectre UV:*
dans l'éthanol: max. 260 nm ε = 18900;
max. 302 nm ε = 22100
dans l'éthanol HCl 0,1N: max. 300 nm ε = 20500
inflexions 240, 249, 270 nm.

*Analyse:* $C_{29}H_{37}NO_2$ 431,62

| | C% | H% | N% |
|---|---|---|---|
| Calculés | 80,7 | 8,64 | 3,24 |
| Trouvés | 80,6 | 8,8 | 3,2 |

*Spectre RMN* (dentérochloroforme)
Pic à 0,59 ppm hydrogènes du méthyle 18;
Pic à 2,30 ppm hydrogènes du diméthylamino;
Pic à 3,76 ppm hydrogènes de $CH_2O$;
Pics à 4,3 ppm hydrogène en position 11;
Pic à 5,70 ppm hydrogène en position 4;
Pics à 6,64 et 7,03 ppm hydrogènes aromatiques.

Exemple 2

(17R) 11 β-/4-(diméthylamino) phényl/ spiro (estra-4,9-dièn-17,2′ (5H furan) 3-one

On dissout 1,425 g de (Z) 11 β-/4-(diméthylamino) phényl/ 17β-hydroxy 17α-(3-hydroxy 1-propényl) estra-4,9-dièn-3-one dans 30 $cm^3$ de pyridine, ajoute à +3°C, 3 g de chlorure de tosyle, agite pendant 4 heures à +20°C, refroidit à +3°C, ajoute de l'eau, agite pendant 15 minutes, extrait à l'acétate d'éthyle, lave à l'eau, sèche, concentre à sec par distillation sous pression réduite, effectue un entrainement azéotropique avec du toluène pour bien éliminer toute la pyridine, dissout le résidu dans l'éther éthylique, filtre, concentre à sec et obtient 1,37 g de produit recherché cristallisé.

Après recristallisation dans l'éther isopropylique, on obtient 1,23 g de produit recherché pur.

*Contrôles*

*Spectre IR* (chloroforme)

3 ceto $\Delta^4$ C=O $1655^{cm^{-1}}$

C=C $1612^{cm^{-1}}$

aromatique $1597^{cm^{-1}}$, $1562^{cm^{-1}}$, $1518^{cm^{-1}}$,

*Spectre UV*

Ethanol

| | | |
|---|---|---|
| max. á 260 nm | | ε = 19600 |
| max. á 302 nm | | ε = 23300 |

Ethanol/HCl 0,1N

| | | |
|---|---|---|
| infl. | 215 nm | |
| infl. | 238 nm | |
| max. | 300 nm | ε = 21600 |

*Analyse:* $C_{29}H_{35}NO_2$ 429,60

| | C% | H% | N% |
|---|---|---|---|
| Calculés | 81,08 | 8,21 | 3,26 |
| Trouvés | 81,0 | 8,3 | 3,3 |

*Spectre RMN* (deutérochloroforme):

Pic à 0,62 ppm hydrogène du méthyle 18;
Pic à 2,92 ppm hydrogène du diméthylamino;
Pic à 4,29 ppm hydrogène en position 11;
Pic à 4,60 ppm hydrogène de $CH_2O$;
Pic à 5,78 ppm hydrogène en position 4;
Pic à 5,88 ppm hydrogènes en position 3′ et 4′;
Pic à 6,69 ppm hydrogènes aromatiques
et 7,05 ppm

Exemple 3

(17R) 11 β-/4-(méthylthio) phényl/ spiro (estra-4,9-dièn-17,2′ (5H) furan) 3-one

*Stade A:* 3,3-(1,2-éthane diyl) acétal cyclique de 5α, 10α-époxy, 17 β-hydroxy 17α-/3-(tétrahydro-2H-2-pyrannyloxy) 1-propynyl/estr-9(11)-èn-3-one.

On mélange sous atmosphère inerte 5,06 $cm^3$ de réactif HC≡C—$CH_2$OTHP, 30 $cm^3$ de tétrahydrofuranne, refroidit à −5°C, ajoute goutte à goutte 20 $cm^3$ de solution de butyllithium dans l'hexane (1,65 M), agite pendant 30 minutes à 0°C introduit en 40 minutes environ à 0°C, 6,6 g de 3,3-(1,2-éthanediyl) acétal cyclique de 5α, 10α-époxy estra-9(11)-èn 3,17-dione en solution dans 55 $cm^3$ de tétrahydrofuranne, agite pendant 16 heures à 20°C, verse dans un solution aqueuse à 10% de chlorure d'ammonium, extrait à l'acétate d'éthyle, lave à l'eau, sèche, concentre à sec par distillation sous pression réduite, chromatographie le résidu obtenu sur silice en éluant par un mélange cyclohexane-acétate d'éthyle (1/1) obtient 8,3 g de composé recherché, utilisé tel quel pour le stade suivant.

*Spectre IR* (chloroforme)

OH libre à $3601^{cm^{-1}}$ + un peu d'absorption OH associé;
C≡C— à $1640^{cm^{-1}}$;
présence de OTHP.

*Stade B:* 3,3-(1,2-éthanediyl) acétal cyclique de 5α, 17β-dihydroxy 11 β/4-(méthylthio) phényl/ 17α-/3-(tétrahydro 2H 2-pyrannyloxy) 1-propynyl/estra-9-èn-3-one.

1°/. Préparation du magnésien:

On mélange sous atmosphère inerte 3 g de magnésium en tournures, 3 $cm^3$ de tétrahydrofuranne, amène à 45°C, ajoute quelques gouttes de la solution suivante: 20,2 g de parabromothioanisole en solution

dans 70 $cm^3$ de tétrahydrofuranne. Après amorçage de la réaction, on poursuit l'introduction de cette solution de façon à maintenir la température à environ 50°C, on chauffe encore 1 heure à 50°C après la fin de l'introduction.

On obtient un magnésien titrant 1,1 N.

2°/. Condensation:

On mélange sous atmosphère inerte 80 $cm^3$ de solution de magnésien, 90 $cm^3$ tetrahydrofuranne, 887 mg de chlorure cuivreux, refroidit à −15°C, introduit en 15 minutes environ une solution de 12,2 g de (1,2-éthanediyl) acétal cyclique de 5α, 10α-époxy 17β-hydroxy 17α-/3-(tétrahydro 2H 2-pyrannyloxy) 1-propynyl/ estr 9(11)-èn-3-one dans 25 $cm^3$ de tétrahydrofuranne, agite pendant une heure à 0°C, verse dans une solution aqueuse à 10% de chlorure d'ammonium, extrait à l'acétate d'éthyle; lave à l'eau, sèche, et concentre à sec par distillation sous pression réduite, chromatographie sur silice le résidu obtenu en éluant pa un mélange cyclohexane-acétate d'éthyle (1/1), obtient 15 g de composé recherché brut, on cristallise 11 g de ce produit dans l'éthanol à 50% d'eau, obtient 10,2 g de produit recherché F = 160°C.

*Contrôles:*

*Spectre IR* (chloroforme):
OH en 17 à 3600$^{cm^{-1}}$ (libre) + associé en 5 à 3510$^{cm^{-1}}$ aromatiques
à 1596$^{cm^{-1}}$; 1556$^{cm^{-1}}$; 1492$^{cm^{-1}}$; présence de OTHP.

*Spectre UV* (éthanol):
Infl. 228 nm; Max. 255 nm ε = 15000
Infl. 288 nm;
Infl. 297 nm.

*Stade C:* (Z) (1,2-éthane diyl) acétal cyclique de 5α, 17β-dihydroxy 11β-/4-(méthylthio) phényl/ 17α-/3-(tétrahydro-2H-2-pyrannyloxy) 1-propényl/ estr-9-èn-3-one.

On dissout 594 mg de 3,3-(1,2-éthane diyl) acétal cyclique de 5α, 17β-dihydroxy 11β-/4-(méthylthio) phényl/ 17α-/3-(tétrahydro-2H-2-pyrannyl oxy) 1-propynyl/ estr-9-èn-3-one obtenu ci-dessus dans 20 $cm^3$ d'acétate d'éthyle, ajoute 60 mg d'hydroxyde de palladium à 10% sur charbon actif, agite sous atmosphère d'hydrogène pendant 14 heures, élimine le catalyseur par filtration, concentre à sec par distillation sous pression réduite, chromatographie le résidu obtenu sur silice en éluant avec un mélange cyclohexane-acétate d'éthyle 1/1, obtient 151 mg de composé recherché.

*Contrôles:*

*Spectre IR* (chloroforme):
Peu d'OH libre;
surtout OH associé à 3500$^{cm^{-1}}$;
3410$^{cm^{-1}}$ épaulement;
aromatiques à 1557$^{cm^{-1}}$, 1492$^{cm^{-1}}$, 831$^{cm^{-1}}$;
présence d'OTHP.

*Spectre de RMN* (deuterochloroforme)
Pic à 0,53 ppm hydrogène du méthyle 18;
Pic à 2,47 ppm hydrogène du méthyle de $-SCH_3$;
Pic à 4,77 ppm hydrogène cétalique de THP;
Pics de 5,6 à 5,84 ppm hydrogènes éthylèniques;
Pics de 3,44 à 4,5 ppm hydrogène de $CH_2O$ et hydrogène en 11;
Pic à 7,17 ppm hydrogènes aromatiques.

*Stade D:* (Z) 17β-hydroxy 17α-(3-hydroxy 1-propényl) 11β-/4-(méthylthio) phényl/ estra 4,9-dièn 3-one.

On dissout 2,42 g de (Z) (1,2-éthane diyl) acétal cyclique de 5α, 17β-dihydroxy 11β-/4-(méthylthio) phényl/ 17α-/3-(tétrahydro-2H-2-pyrannyloxy) 1-propényl/ estr-9-èn-3-one obtenu ci-dessus dans 44 $cm^3$ de méthanol, ajoute 20 $cm^3$ de solution aqueuse 2 N d'acide chlorhydrique, agite sous atmosphère inerte, pendant 1 heure 30 minutes, dilue à l'eau, extrait à l'acétate d'éthyle, sèche, concentre à sec par distillation sous pression réduite, chromatographie le résidu obtenu sur silice en éluant par un mélange cyclohexane-acétate d'éthyle (1/1). Obtient:

904 mg de composé attendu,

567 mg de (Z) 17β-hydroxy 17α-[3-(tétrahydro-2H-2-pyrannyloxy) 1-propényl] 11-béta-/4-(méthylthio) phényl/estra 4,9-dièn-3-one que l'on remet en hydrolyse acide dans les mêmes conditions et obtient après purification chromatographique 188 mg de composé recherché.

*Contrôles:*

*Spectre IR* (chloroforme):
OH à 3609$^{cm^{-1}}$ + associé;

diènone à $1653^{cm^{-1}}$
diènone à $1601^{cm^{-1}}$
aromatique à $1555^{cm^{-1}}$ et $1493^{cm^{-1}}$

*Spectre de RMN* (deutérochloroforme):
Pic à 0,63 ppm hydrogène du méthyle 18;
Pic à 2,47 ppm hydrogène du méthyle de $-SCH_3$;
Pic à 4,33 ppm hydrogène en position 11;
Pic à 4,39 ppm hydrogènes du $CH_2O$;
Pic de 5,59 à 5,92 ppm hydrogènes éthyléniques;
Pics de 7,04 à 7,24 ppm hydrogènes aromatiques.

*Stade E:* (17R) 11β-/4-(méthylthio)phényl/ spiro (estra-4,9-dièn-17,2'(5H) furan) 3-one.

On dissout 1,04 g de (Z) 17β-hydroxy 17α-(3-hydroxy 1-propényl) 11β- /4-(méthylthio) phényl/estra-4,9-dièn-3-one obtenu précédemment dans 20 cm³ de pyridine, ajoute 2,1 g de chlorure de tosyle, agite pendant 2 heures à 20°C, dilue avec de l'eau et de la glace, extrait à l'acétate d'éthyle, lave par une solution aqueuse diluée d'acide chlorydrique, à l'eau, sèche, concentre à sec par distillation sous pression réduite, chromatographie le résidu obtenu sur silice en éluant par un mélange cyclohexane-acétate d'éthyle (6/4), obtient 820 mg de produit recherché brut que l'on cristallise dans un mélange de chlorure de méthylène et d'éther isopropylique, obtient 694 mg de produit recherché.

*Contrôles:*

*Spectre IR* (chloroforme):
— absence d'OH;
dianone $1653^{cm^{-1}}$ et $1601^{cm^{-1}}$
aromatiques à $1555^{cm^{-1}}$, $1492^{cm^{-1}}$.

*Spectre de RMN* deutérochloroforme):
Pic à 0,59 ppm hydrogènes du méthyle 18;
Pic à 2,48 ppm hydrogènes du méthyle de $-SCH_3$;
Pics de 4,30 à 4,34 ppm hydrogène en position 11;
Pic à 4,6 ppm hydrogènes du $CH_2O$;
Pic à 5,81 ppm hydrogène en position 4;
Pic à 5,89 ppm hydrogènes en position 3' et 4';
Pics de 7,04 à 7,24 ppm hydrogènes aromatiques.

*Spectre UV:*
Max. à 260 nm ε = 16100
Max. à 300 nm.

*Analyse* (PM 432,62)

| | C% | H% | S% |
|---|---|---|---|
| Calculés | 77,73 | 7,45 | 7,41 |
| Trouvés | 77,8 | 7,6 | 7,1 |

Exemple 4

(17 R) 4', 5'-dihydro 11β- /4-(méthylthio) phényl/ spiro (estra 4,9-dièn-17,2'(3H) furan) 3-one

*Stade A:* (1,2-éthanediyl) acétal cyclique de 5α, 17β-dihydroxy 11β-/4-(méthylthio) phényl/ 17α- /3-(tétrahydro 2H 2-pyrannyloxy) propyl/ estr-9-èn 3-one.

On dissout 2,1 g de (1,2-éthane diyl) acétal cyclique de 5α, 17β-dihydroxy 11β-/4-(méthylthio) phényl/ 17α-/3-(tétrahydro-2H-2-pyrannyloxy) 1-propynyl/ estr-9-èn 3-one dans 21 cm³ de benzène, 21 cm³ d'éthanol, ajoute 840 mg de réactif de Wilkinson /chlorotris (triphénylphosphine) rhodium/ et soumet à l'hydrogénation pendant 16 heyres, ajoute 420 mg de réactif de Wilkinson et soumet à l'hydrogénation pendant encore 3 heures, concentre à sec par distillation sous pression réduite, chromatographie le résidu sur silice en éluant par un mélange cyclohexane-acétate d'éthyle (6/4), obtient:

336 mg de composé éthylénique identique au produit obtenu au stade C de l'exemple 3,
185 mg d'un mélange de composé éthylénique de produit initial et de composé recherché,
1,042 g de composé recherché utilisé tel quel pour le stade suivant.

*Spectre IR* (chloroforme):
—OH à $3600^{cm^{-1}}$;
$3504^{cm^{-1}}$ aromatiques à $1600^{cm^{-1}}$, $1492^{cm^{-1}}$

*Spectre de RMN* (deutérochloroforme):
Pic à 0,50 ppm hydrogènes du méthyle 13;

Pic à 2,46 ppm hydrogènes du méthyle de —S—$CH_3$;
Pics de 3,33 à 4,55 ppm hydrogène en position 11 et hydrogènes de $CH_2O$;
Pic à 4,63 ppm hydrogène cétalique du THP;
Pic à 7,17 ppm hydrogènes aromatiques.

*Stade B:* 17β- hydroxy 17α- (3-hydroxy propyl) 11β- /4-(méthylthio)phényl/ estra 4,9-dièn 3-one.

On dissout 1,51 g de (1,2-éthane diyl) acétal cyclique de 5α, 17β-dihydroxy 11β -/4-(méthylthio) phényl/ 17α-/3-(tétrahydro-2H-2-pyrannyloxy) propyl/ estr-9-èn-3-one obtenu au stade A dans 25 $cm^3$ de méthanol, ajoute 11,5 $cm^3$ de solution aqueuse d'acide chlorhydrique dilué au 1/2, agite sous atmosphère inerte à 20°C pendant 30 minutes, dilue à l'eau, extrait au chlorure de méthylène, sèche, concentre à sec en distillant sous pression rèduite, chromatographie le résidu obtenu sur silice en éluant par un mélange éther-acétate d'éthyle (1/1), obtient:

768 mg de produit recherché.

*Spectre IR* (chloroforme):
OH libre à $3621^{cm^{-1}}$ + associé à $3410^{cm^{-1}}$
diènone à $1653^{cm^{-1}}$ et $1601^{cm^{-1}}$
aromatiques $1555^{cm^{-1}}$; $1492^{cm^{-1}}$

*Stade C:* (17 R) 4', 5'-dihydro 11β-/4-(méthyl thio) phényl/spiro (estra 4,9-dièn-17,2'-(3H) furan)-3 one.

On dissout 1,04 g de 17β-hydroxy 17α-(3-hydroxy propyl) 11β-/4-(méthylthio) phényl/ estra-4,9-dièn-3-one obtenu au stade B dans 20 $cm^3$ de pyridine, ajoute 2,1 g de chlorure de tosyle, agite pendant 1 heure à 20°C, dilue avec de l'eau et de la glace, extrait à l'acétate d'éthyle, lave par une solution aqueuse diluée d'acide chlorhydrique, à l'eau, avec une solution aqueuse de bicarbonate de sodium, sèche, concentre à sec par distillation sous pression réduite, cristallise le résidu obtenu dans un mélange chlorure de méthylène-éthanol, obtient:

818 mg de produit recherché pF = 105°C (peu net).

*Spectre IR* (chloroforme):
—C — O — C — à $1078^{cm^{-1}}$, $1055^{cm^{-1}}$, diènone à $1653^{cm^{-1}}$, $1602^{cm^{-1}}$; aromatiques $1555^{cm^{-1}}$, $1493^{cm^{-1}}$

*Spectre de RMN* (deutérochloroforme):
Pic à 0,57 ppm hydrogènes de méthyle 18;
Pic à 2,46 ppm hydrogènes du méthyle de —$SCH_3$;
Pic à 3,78 ppm hydrogènes de $CH_2$—O—;
Pics de 4,35 à 4,42 ppm hydrogènes en position 11;
Pic à 5,81 ppm hydrogène en position 4;
Pic à 7,17 ppm hydrogènes aromatiques.

*Analyse* $C_{28}H_{34}O_2S$ (434,64)

| | C% | H% |
|---|---|---|
| Calculés | 77,37 | 7,88 |
| Trouvés | 77,1 | 8,0 |

## Exemple 5

(17R) 11β-(2-méthoxy phényl) spiro (estra-4,9-dièn-17,2'-(5H) furan) 3-one

*Stade A:* (Z) diméthyl cétal de 5α, 10α-époxy 17β-hydroxy 17α-[3-(tétrahydro 2H-2-pyrannyloxy) 1-propynyl] estr-9-(11)-èn 3-one.

On refroidit à 0°C 400 $cm^3$ d'une solution de butylithium dans l'hexane (1,6 M), ajoute à cette température 98 g de réactif HC≡C—$CH_2$OTHP dans 180 $cm^3$ de tétrahydrofuranne et agite 30 minutes à 0°C.

On ajoute goutte à goutte 66,4 g de 3,3-diméthyl cétal de 5α, 10α-époxy estr-9(11)-èn-3,17-dione en solution dans 200 $cm^3$ de tétrahydrofuranne, agite 2 heures en laissant revenir à température ambiante, verse dans une solution aqueuse de chlorure d'ammonium, extrait à l'acétate d'éthyle puis au chlorure de méthylène, réunit les phases organiques, les lave à l'eau, les sèche, puis élimine les solvants sous pression réduite. On chromatographie le résidu sur silice (éluant cyclohexane-acétate d'éthyle 7—3 à 1% de triéthylamine. On obtient 53 g de produit brut que l'on purifie en effectuant une chromatographie sur silice (éluant chlorure de méthylène-acétone 95/5 à 1% de triéthylamine).

*Stade B:* (Z) diméthyl cétal de 5α, 10α-époxy 17 β-hydroxy 17α -[3-(tétrahydro-2H-2-pyrannyloxy) 1-propényl] estr-9(11)-èn-3-one.

On hydrogène pendant 30 minutes sous une pression de 1100 mb, 2,5 g de produit brut obtenu au stade A dans 400 $cm^3$ d'acétate d'éthyle en présence de 25 mg de palladium à 10% sur sulfate de baryum et 1 $cm^3$ de pyridine. On filtre le catalyseur, le lave à l'acétate d'éthyle, réunit les phases organiques, élimine les solvants sous pression réduite et récupère 2,5 g de produit brut que l'on purifie par chromatographie sur silice (éluant chlorure de méthylène-acétone 95—5).

On obtient 728 mg de produit attendu.

*Spectre IR (CHCl$_3$):*
OH 3600$^{cm^{-1}}$, 3400$^{cm^{-1}}$ (F)
présence de l'époxy.

*Stade C:* (Z) diméthyl cétal de 5α, 17β-dihydroxy 11β-(2-méthoxy phényl) 17α-[3-(tétrahydro-2H-2-pyrannyloxy) 1-propényl] estr-9-èn-3-one.

Préparation du magnésien:

On opère comme au stade B de l'exemple 3 à partir de magnésium et d'orthobromo anisole. On obtient une solution titrant 0,72 M/l.

Condensation:

On dissout 3 g du produit obtenu au stade précédent dans 60 cm$^3$ de tétrahydrofuranne sous atmosphère inerte, ajoute 187 mg de chlorure cuivreux, chauffe à 34°C ± 1°C, introduit en 20 minutes 26,2 cm$^3$ du magnésien préparé ci-dessus et agite pendant 16 heures. On laisse revenir à température ambiante, verse dans une solution de chlorure d'ammonium, agite 15 minutes, extrait à l'acétate d'éthyle, lave avec une solution aqueuse de chlorure de sodium, sèche et concentre à sec sous pression réduite. On obtient 6,58 g de produit brut que l'on purifie par chromatographie sur silice (éluant chlorure de méthylène-acétone 92—8 a 1% de triéthyamine).

*Spectre IR (CHCl$_3$):*
OH Libre 3600$^{cm^{-1}}$ asocié 3450$^{cm^{-1}}$
aromatiques 1597$^{cm^{-1}}$, 1584$^{cm^{-1}}$, 1490$^{cm^{-1}}$

*Stade D:* (Z) 11β-(2-méthoxyphényl) 17β-hydroxy 17α-(3-hydroxy 1-propényl) estra-4,9-dièn 3-one.

On dissout à température ambiante 1,08 g de produit obtenu au stade B dans 10 cm$^3$ d'éthanol, ajoute une solution de 1,08 g d'hydrogéno sulfate de potassium dans 6,5 cm$^3$ d'eau et agite 5 heures à température ambiante. On élimine l'éthanol, extrait au chlorure de méthylène, lave à l'eau la phase organique, sèche et concentre à sec sous pression réduite. Après chromatographie sur silice (éluant cyclohexane-acétate d'éthyle 1—1), on obtient 0,703 g de produit attendu.

*Spectre IR (CHCl$_3$):*
OH Libre 3612$^{cm^{-1}}$ et associé 3,415$^{cm^{-1}}$
diénone 1654$^{cm^{-1}}$, —1597$^{cm^{-1}}$
aromatique 1488$^{cm^{-1}}$.

*Stade E:* (17R) 11β-(2-méthoxy phényl) spiro (estra 4,9-dièn-17,2'-(5H) furan) 3-one.

On dissout à température ambiante 0,661 g de produit obtenu au stade précédent dans 13,2 cm$^3$ de pyridine, refroidit la solution à 0°C et ajoute en 5 minutes 1,32 g de chlorure de tosyle, laisse revenir à température ambiante, agite 1 heure, refroidit de nouveau à 0°C et ajoute 14 cm$^3$ d'acide chlorhydrique 6 N. On décante, extrait la phase aqueuse à l'acétate d'éthyle, lave à l'eau, sèche et élimine les solvants sous pression réduite. Après chromatographie sur silice (éluant cyclohexane-acétate d'éthyle 8—2), on obtient 0,459 g de produit attendu.

*Spectre IR (CHCl$_3$):*
diénone 1655$^{cm^{-1}}$, —1597$^{cm^{-1}}$
aromatique 1488$^{cm^{-1}}$.
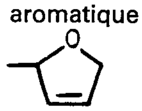 1080$^{cm^{-1}}$, 1040$^{cm^{-1}}$

Exemple 6

(17R) 11β-(4-chlorophényl) 4', 5'-dihydro spiro (estra-4,9-dièn-17, 2'(3H) furan)-3-one

*Stade A:* Diméthylcétal de 5α, 10α-époxy, 17β-hydroxy 17α-[3-tétrahydro 2H 2-pyrannyloxy) propyl] estr-9(11)-èn-3-one.

On dissout 6 g de 3,3-diméthoxy cétal de 5α, 10α-époxy 17β-hydroxy 17α-[3-tétrahydro 2H-2-pyrannyloxy) 1-propynyl/estr 9(11)èn-3-one préparé comme au stade A de l'exemple 5 dans 60 cm$^3$ de benzène puis soumet à une hydrogénation sous 1860 mbar en présence de 1,5 g de réactif de Wilkinson pendant 7 heures. On dilue le mélange à l'éther, filtre, concentre à sec le filtrat sous pression réduite et recueille 7,7 g de produit brut que l'on chromatographie sur silice (éluant éther de pétrole (Eb 40°—70°C) acétate d'éthyle 4—6). On obtient 4,95 g de produit attendu.

*Spectre IR (CHCl$_3$):*
OH Libre 3620$^{cm^{-1}}$, 3600$^{cm^{-1}}$
—C=C 1640$^{cm^{-1}}$

*Stade B:* diméthyl cétal de 11β-(4-chloro phényl) 5α, 17β-dihydroxy 17α-[3-(tétrahydro 2H-2-pyrannyloxy) propyl] estr-9(11)-èn-3-one.

Preparation du magnésien:

On opère comme au stade B de l'exemple 3 à partir de 1,22 g de manésium et 3 $cm^3$ de la solution préparée à partir de 7,65 g de parabromochlorobenzène dans 50 $cm^3$ de tétrahydrofuranne. On obtient un magnésien titrant 0,65 M/l.

Condensation:

On opère comme au stade B de l'exemple 3 en utilisant 23 $cm^3$ de la solution de magnésien, 165 mg de chlorure cuivreux et 2,46 g du produit obtenu au stade A précédent en solution dans 12 $cm^3$ de tétrahydrofuranne. Après chromatographie sur silice (éluant éther de pétrole (Eb. 40—70°C)-acétate d'éthyle 1—1), on recueille 2 g de produit brut attendu que l'on utilise tel quel pour le stade suivant.

*Spectre IR ($CDCl_3$):*

OH en 5 $3478^{cm^{-1}}$

OH en 17 max. $3620^{cm^{-1}}$, ép. $3600^{cm^{-1}}$

bandes aromatiques 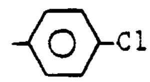 $1599^{cm^{-1}}$, $1489^{cm^{-1}}$

$—OCH_3$ $2835^{cm^{-1}}$

*Stade C:* 11β-(4-chlorophényl) 17β-hydroxy 17α-(3-hydroxy propyl) estra-4,9-dièn-3-one.

On ajoute 15 $cm^3$ d'acide chlorhydrique 2 N à 1,99 g de produit obtenu au stade précédent dans 20 $cm^3$ de méthanol.

On chauffe à 50°C pendant 45 minutes, verse dans une solution aqueuse de bicarbonate de soude, extrait à l'acétate d'éthyle, sèche et concentre à sec sous pression réduite. On obtient 1,42 g de produit attendu. F = 262°C après recristallisation dans un mélange chlorure de méthylène-éther isopropylique.

*Spectre IR ($CHCl_3$):*

OH Libre $3620^{cm^{-1}}$ + associé

diénone $1655^{cm^{-1}}$ — $1602^{cm^{-1}}$

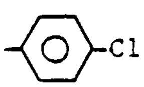 bandes aromatiques $1570^{cm^{-1}}$ — $1490^{cm^{-1}}$.

*Analyse:* $C_{27}H_{31}ClO_2$ 422,99

| | C% | H% | Cl% |
|---|---|---|---|
| Calculé | 73,53 | 7,54 | 8,03 |
| Trouvé | 73,3 | 7,8 | 8,3 |

*Stade D:* (17R) 11β-(4-chlorophényl) 4', 5'-dihydro spiro (estra-4,9-dièn-17,2'(3H) furan)-3-one.

On dissout sous atmosphère inerte 1g du produit obtenu au stade C dans 20 $cm^3$ de pyridine, refroidit à 0°/+5°C, ajoute 2,2 g de chlorure de tosyle et agite pendant 1 heure et demi à température ambiante. On verse dans l'eau glacée, agite 15 minutes, extrait à l'acétate d'éthyle, lave avec une solution aqueuse saturée en bicarbonate de sodium, essore le précipité formé, sèche et obtient 694 mg de produit attendu.

On sèche le filtrat, élimine le solvant sous pression réduite, chromatographie le résidu sur silice (éluant cyclohexane-acétate d'éthyle 6—4) et obtient le produit attendu que l'on recristallise dans un mélange éthanol-chloroforme (2—1) F = 308°C.

*Spectre IR ($CHCl_3$):*

diénone $1654^{cm^{-1}}$ — $1603^{cm^{-1}}$

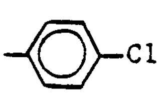 aromatiques $1572^{cm^{-1}}$ — $1490^{cm^{-1}}$

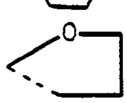 $1078^{cm^{-1}}$, $—1055^{cm^{-1}}$

*Analyse:* $C_{27}H_{31}ClO_2$: 422,99

| | C% | H% | Cl% |
|---|---|---|---|
| Calculé | 76,66 | 7,38 | 8,38 |
| Trouvé | 76,7 | 7,4 | 9 |

Exemple 7

(17R) 4',5'-dihydro 11β-(4-méthoxy phényl) spiro (estra-4,9-diène-17,2'(3H) furan)-3-one

*Stade A:* diméthyl cétal de 5α, 17β-dihydroxy 11β-(4-méthoxyphényl) 17α-[3-(tétrahydro 2H-2-pyrannyloxy) propyl] estr-9-èn-3-one.

Preparation du magnésium:

On opère comme au stade B de l'exemple 3 à partir de 1,45 g de magnésium et 9,15 g de parabromoanisole dans 45 $cm^3$ de tétrahydrofuranne. On obtient un magnésien titrant 0,9 M/l.

Condensation:

On opère comme à l'exemple 3 en utilisant 15 $cm^3$ de magnésien préparé ci-dessus, 15 $cm^3$ de tétrahydrofuranne, 165 mg de chlorure cuivreux et 2,4 g de produit préparé au stade A de l'exemple 6. Après chromatographie sur silice (éluant éther de pétrole (Eb. 40—70°C) acétate d'éthyle 1—1), on recueille 6 g de produit attendu utilisé tel quel pour le stade suivant.

*Stade B:* 17β-hydroxy 17α-(3-hydroxypropyl) 11β-(4-méthoxyphényl) estra-4,9-dièn-3-one.

On opère comme au stade C de l'exemple 6 à partir de 6 g du produit obtenu au stade B. On obtient 5 g de produit brut attendu que l'on purifie par chromatographie sur silice en éluant par le mélange chlorure de méthylène-acétone 7—3.

*Spectre IR ($CHCl_3$):*

| | |
|---|---|
| OH Libre | $3620^{cm^{-1}}$ + associé |
| C=O | $1656^{cm^{-1}}$ |
| C=C aromatique | $1608^{cm^{-1}}$ $1509^{cm^{-1}}$ |

*Stade C:* (17R) 4',5'-dihydro 11β-(4-méthoxy phényl) spiro (estra-4,9-dièn-17,2'(3H) furan)-3-one

On opère comme au stade D de l'exemple 6 à partir de 655 mg de produit obtenu au stade B et 1,4 g de chlorure de tosyle. Après chromatographie, on obtient 650 mg de produit brut que l'on recristallise dans un mélange chlorure de méthylène-éther isopropylique. On recueille 500 mg de produit attendu F = 192°C

*Spectre IR ($CHCl_3$):*

| | |
|---|---|
| C=O | 1 $655^{cm^{-1}}$ |
| C=C aromatique | 1 $608^{cm^{-1}}$(max) 1 $582^{cm^{-1}}$ (épaulement) 1 $509^{cm^{-1}}$ |
| C—O—C | 1 $078^{cm^{-1}}$ 1 $055^{cm^{-1}}$ |

*Analyse:* $C_{28}H_{34}O_3$: 418,58

| | C% | H% |
|---|---|---|
| Calculé | 80,34 | 8,18 |
| Trouvé | 80,2 | 8,2 |

$[\alpha]_D = +133{,}5° \pm 2{,}5°$ (c = 1% $CHCl_3$)

Exemple 8

(17R) 11β-(3-thiényl) spiro (estra-4,9-dièn-17,2'-(5H) furan) 3-one

*Stade A:* (Z) (1,2-éthane diyl) acétal cyclique de 5α, 10α-époxy 17α-[3-tétrahydro 2H-2-pyrannyloxy) 1-propényl] estr-9(11)-èn-3-one.

On dissout 940 mg de produit préparé au stade A de l'exemple 3 dans 20 $cm^3$ d'acétate d'éthyle en présence de 5 $cm^3$ de pyridine. On ajoute 9 mg de palladium à 10% sur sulfate de baryum et hydrogène pendant 24 minutes à pression atmosphérique. On filtre le catalyseur, le rince à l'acétate d'éthyle, et concentre à sec le filtrat. On obtient 965 mg de produit brut que l'on chromatographie sur silice (éluant chlorure de méthylène-acétate d'éthyle 8—2).

On récupère 795 mg de produit attendu.

*Spectre IR ($CHCl_3$):*

| | |
|---|---|
| OH Libre | 3 $600^{cm^{-1}}$ associé ≃ 3 $400^{cm^{-1}}$ |
| Δ 9—11 | 1 $640^{cm^{-1}}$ |
| époxy | $971^{cm^{-1}}$. |

*Stade B:* (Z) (1,2-éthane diyl) acétal cyclique de 5α,17β-dihydroxy 17α-[3-(tétrahydro 2H-2-pyrannyloxy) 1-propényl] 11β-(3-thiényl) estr-9-èn-3-one.

Préparation du magnésien:

On opère comme à l'exemple 3 (stade B) à partir de magnésium et de 3-bromothiophène. On obtient une solution titrant 0,6 M/l.

Condensation:

On refroidit à −20°C une suspension comprenant 35,2 $cm^3$ du magnésien préparé ci-dessus dans 14$cm^3$ de tétrahydrofuranne, ajoute 0,210 g de chlorure cuivreux et agite 10 minutes. On introduit goutte à goutte une solution de 2,5 g de produit obtenu au stade A dans 25 $cm^3$ de tétrahydrofuranne, en maintenant la température à −20°C et agite 1 heure. On laisse revenir à température ambiante, ajoute goutte à goutte 10 $cm^3$ de chlorure d'ammonium, en solution saturée, verse le milieu réactionnel dans 90 $cm^3$ de solution saturée en chlorure d'ammonium, agite 15 minutes, décante de extrait la phase aqueuse à l'acétate d'éthyle. On lave à l'eau la phase organique, la sèche et élimine les solvants sous pression réduite.

On obtient 4,68 g de produit brut que l'on chromatographie sur silice (éluant cyclohexane-acétate d'éthyle 6—4 à 1% de triéthylamine. On obitent 2,107 g de produit attendu F = 158°C.

*Spectre IR ($CHCl_3$):*

OH en 5 — 3 501$^{cm^{-1}}$

*Stade C:* (Z) 17β-hydroxy 17α-(3-hydroxy 1-propényl) 11β-(3-thiényl) estra-4,9-dièn-3-one.

On dissout sous atmosphère inerte 2,1 g de produit obtenu au stade précédent dans 42 $cm^3$ d'éthanol, ajoute 6,3 $cm^3$ d'acide chlorhydrique en solution aqueuse 2N et agite 3 heures à température ambiante. On refroidit à 5°C, ajoute progressivement 42 $cm^3$ d'eau, agite 30 minutes, essore le précipité, le lave à l'eau jusqu'à neutralité, le dissout dans du chlorure de méthylène, sèche et élimine les solvants sous pression réduite. On obtient 1,15 g de produit attendu. F = 240°C.

*Spectre IR ($CHCl_3$):*

OH Libre — 3 611$^{cm^{-1}}$ et associé

diénone — 1 657$^{cm^{-1}}$ −1 603$^{cm^{-1}}$

*Stade D:* (17R) 11-(3-thiényl) spiro (estra-4,9-dièn-17,2'(5H)-furan)-3-one

On opère comme au stade D de l'exemple 5 en utilisant 1,15 g de produit préparé au stade précédent, 23 $cm^3$ de pyridine, 2,3 g de chlorure de tosyle et 23 $cm^3$ d'acide chlorhydrique 6 N.

On obtient 1,03 g de produit brut que l'on purifie par chromatographie sur silice en éluant par chlorure de méthylène-acétone 98—2). On recueille 0,755 g de produit attendu que l'on recristallise dans l'isopropanol. F = 242°C.

*Spectre IR ($CHCl_3$):*

diènone — 1 654$^{cm^{-1}}$ −1 603$^{cm^{-1}}$

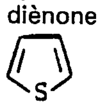 — 1 520$^{cm^{-1}}$

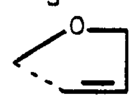 — 1 080$^{cm^{-1}}$

*Analyse:* $C_{25}H_{28}O_2S$: 392,565

| | C% | H% | S% |
|---|---|---|---|
| Calculé: | 76,49 | 7,18 | 8,16 |
| Trouvé: | 76,4 | 7,3 | 8,0 |

Exemple 9

(17R) 11β-(4-acétylphényl) spiro (estra-4,9-dièn-17,2'(5H) furan) 3-one.

*Stade A:* (Z) (1,2-éthanediyl) acétal cyclique de 5α,17β-dihydroxy 11β-[4-(2-méthyl 1,3-dioxolan-2-yl)phényl] 17α-[3-(tétrahydro-2H-2-pyrannyloxy) 1-propényl] estr-9-èn-3-one.

Prépration du magnésien.

On opère comme au stade B de l'exemple 3 à partir de 1,45 g de magnésium et 12,15 g d'éthylène cétal de parabromo acétophénone. On obtient une suspension titrant 0,8 M.

Condensation.

On ajoute sous atmosphère inerte 21 mg de chlorure cuivreux dans 8 $cm^3$ de la suspension de magnésien refroidie à 0°/+5°C, agite 15 minutes, ajoute goutte à goutte 1 g du produit préparé au stade A

de l'exemple 8 en solution 15 $cm^3$ de tétrahydrofuranne et agite 1 heure à température ambiante. On verse dans une solution aqueuse de chlorure d'ammonium, extrait à l'acétate d'éthyle, lave à l'eau, sèche et concentre à sec. On obtient 3,5 g de produit brut que l'on purifie par chromatographie sur silice (éluant: cyclohexane-acétate d'éthyle 1—1).

*Spectre IR ($CHCl_3$):*
OH en 5: $3500^{cm^{-1}}$
OH + associé: $3600^{cm^{-1}}$
aromatiques } $1605^{cm^{-1}}$
C=C } $1502^{cm^{-1}}$

*Stade B:* (Z) 11β-(4-acétylphényl) 17β-hydroxy 17α-(3-hydroxy 1-propényl) estra-4,9-dièn-3-one.

On dissout 2,07 g de produit préparé au stade précédent dans 40 $cm^3$ de méthanol, ajoute 16 $cm^3$ de solution aqueuse 2 N d'acide chlorhydrique, agite 1 heure et demise à 50°C, verse dans une solution aqueuse de bicarbonate de sodium, extrait à l'acétate d'éthyle, lave à l'eau, sèche et concentre à sec sous pression réduite. On obtient 1,63 g de produit brut que l'on chromatographie sur silice (éluant: chlorure de méthylèneacétone 7—3) puis recristallise dans l'éthanol. On récupère 1,48 g de produit attenu. F ≃ 130°C.

*Spectre IR ($CHCl_3$):*
C=O: $1678^{cm^{-1}}$
—$CH_3$: $1359^{cm^{-1}}$
C=C + aromatique: $1604^{cm^{-1}}$, $1565^{cm^{-1}}$
diénone: $1657^{cm^{-1}}$
OH libre: $3609^{cm^{-1}}$
OH associé: $3410^{cm^{-1}}$

*Analyse:* $C_{29}H_{34}O_4$: 444,57

| | C% | H% |
|---|---|---|
| Calculé: | 77,99 | 7,67 |
| Trouvé: | 78 | 7,7 |

*Stade C:* (17R) 11β-(4-acétylphényl) spiro (estra-4,9-dièn-17,2'-(5H) furan) 3-one.

On dissout sous atmosphère inerte 0,704 g de produit préparé au stade précédent dans 16 $cm^3$ de pyridine. On refroidit à 0°C, ajoute 1,49 g de chlorure de tosyle et agite 2 heures en laissant revenir à température ambiante. On verse dans de l'eau glacée, agite 15 minutes, extraite à l'acétate d'ethyle, lave avec une solution de bicarbonate de sodium, sèche et évapore à sec. On obtient 0,775 g de produit brut que l'on chromatographie sur silice (éluant: cyclohexane-acétate d'éthyle 1—1), puis recristallise dans le mélange éthanol-chlorure de méthylène. On recueille 0,555 g de produit attendu. F ≃ 125—130°C.

*Spectre IR ($CHCl_3$):*

C=O: $1678^{cm^{-1}}$
$CH_3$: $1359^{cm^{-1}}$
C=C + aromatique: $1604^{cm^{-1}}$, $1565^{cm^{-1}}$
diénone: $1657^{cm^{-1}}$
$[\alpha]_D = +231,5° \pm 3°$ (c = 1% $CHCl_3$).

*Analyse:* $C_{29}H_{32}O_3$: 428,57

| | C% | H% |
|---|---|---|
| Calculé: | 81,27 | 7,52 |
| Trouvé: | 81,1 | 7,8 |

Exemple 10

(17R) 11β-[4-(méthylthio)phényl]spiro(estra 5(10)9(11)-dièn-17,2' (5H)furan)-3-one et (17R) 11β-[4-(méthylthio)phényl]spiro (éstra-4,9-dièn-17,2' (5H) furan)-3-one.

On dissout 1,5 g du produit préparé au stade C de l'exemple 3 dans 30 $cm^3$ de méthanol et ajoute 15 $cm^3$ d'acide chlorhydrique 2 N. On ajoute de nouveau 30 $cm^3$ de méthanol, 30 $cm^3$ de dioxane et agite 2 heures à témpérature ambiante. On verse le milieu réactionnel dans l'eau, extrait au chlorure de

méthylène, concentre à sec et obtient 1,23 g de résidu que l'on redissout dans 25 $cm^3$ de pyridine. On ajoute 1,91 g de chlorure de tosyle, agite 1 heure et demie à ribbon température ambiante, verse dans l'eau glacée et extrait à l'acétate d'éthyle. On lave avec une solution aqueuse d'acide chlorhydrique, puis avec une solution aqueuse de chlorure de sodium, séche et concentre à sec. On récupère 1,12 g de produit brut que l'on chromatographie sur silice (éluant: chlorure de méthylène-acétate d'éthyle 9—1) et obtient 98 mg de produit attendu et 863 mg de l'estra-4,9-diène correspondant, (identique au produit obtenu à l'exemple 3, stade E).

*Spectre IR ($CHCl_3$)* de l'estra-5(10), 9(11)-diène.

| | |
|---|---|
| C=O: | $1712^{cm-1}$ |
| C=C + aromatique: | $1590^{cm-1}$, $1492^{cm-1}$ |
| C=C du spirocycle: | $1626^{cm-1}$ |

*Spectre IR ($CHCl_3$)* de l'estra-4,9-diène

| | |
|---|---|
| C=O: | $1653^{cm-1}$ |
| C=C conjugué à C=O: | $1600^{cm-1}$ |
| aromatique: | $1492^{cm-1}$ |

## Exemple 11

N-oxyde de (17R) 4',5'-dihydro 11β-[4-(diméthylamino)phényl] spiro (estra-4,9-dièn-17,2'-(3H) furan) 3-one.

On dissout 1,43 g de (17R) 4',5'-dihydro 11β-[4-(diméthylamino)phényl] spiro (estra-4,9-dièn-17,2'-(3H) furan) 3-one préparé à l'exemple 1 (produit B) dans 30 $cm^3$ de chlorure de méthylène, refroidit à 0°/5°C et ajoute en 15 minutes 0,666 g d'acide métachloroperbenzoïque à 85% en solution dans 15 $cm^3$ de chlorure de méthylène.

Après 1 heure et demie d'agitation à 0°/+5°C, on verse le milieu réactionnel dans 100 $cm^3$ d'une solution de thiosulfate de sodium (0,2 N) et extrait au chlorure de méthylène. On lave avec une solution aqueuse de bicarbonate de sodium, puis à l'eau, sèche et élimine les solvants. On récupère 1,8 g de produit brut que l'on chromatographie sur silice (éluant chlorure de méthylène-méthanol 7—3). On obtient 1,34 g de produit attendu (solvat contenant du chlorure de méthylène) que l'on lyophylise.

*Spectre IR ($CHCl_3$):*

| | |
|---|---|
| —C=O: | $1\,655^{cm-1}$ |
| C=C et aromatique: | $1\,602^{cm-1}$, $-1\,498^{cm-1}$ |

$[\alpha]_D = +128°C \pm 2°$ (c = 1% éthanol).

## Exemple 12

(17R) 4',5'-dihydro 11β-[4-(1-méthyléthyl) phényl] spiro (estra-4,9-dièn-17,2'-(3H) furan) 3-one

*Stade A:* (1,2-éthane diyl) acétal cyclique de 5α,10α-époxy 17β-hydroxy 17α-[3-(tétrahydro 2H-2-pyrannyloxy) propyl] estr 9(11) èn 3-one.

On opère comme à l'exemple 6 stade A, en utilisant 1 g du produit (1,2-éthane diyl) acétal cyclique de 17β-hydroxy 5α,10α-époxy 17α-[3-(tétrahydro- 2H-2-pyrannyloxy) 1-propynyl] estr 9(11)-èn-3-one préparé à l'exemple 3 stade A et 0,25 g de catalyseur de Wilkinson. On obtient 1,28 g de produit attendu.

*Spectre IR ($CHCl_3$):*

| | |
|---|---|
| OH Libre: | $3\,620^{cm-1}$ $3\,605^{cm-1}$ |
| OH associé fort: | $3\,485^{cm-1}$ |
| C=C: | $1\,643^{cm-1}$ |

*Stade B:* (1,2-éthane diyl) acétal cyclique de 5α,17β-dihydroxy 11β-[4-(1-méthyl éthyl) phényl] 17α-[3-(tétrahydro 2H-2-pyrannyloxy) propyl] estr 9-èn-3-one.

On refroidit à 0°C 2,45 g de produit préparé comme au stade A en solution dans 10 $cm^3$ de tétrahydrofuranne, ajoute 110 mg de chlorure cuivreux, agite 10 minutes, ajoute en 15 minutes 32 $cm^3$ de bromure de (4-isopropyl) phényl magnésium en solution à 0,66 M/l dans le tétrahydrofuranne. On agite 2 heures à +3°C ± 1°C, verse dans une solution aqueuse glacée de chlorure d'ammonium, extrait à l'éther puis au chlorure de méthylène, séche et élimine les solvants sous pression rèduite. On obtient 4,65 g de produit brut que l'on chromatographie sur silice (éluant cyclohexane-acétate d'éthyle 5—5) et l'utilise tel quel pur le stade suivant.

*Stade C:* 17β-hydroxy 17α-(3-hydroxypropyl) 11β-[4-(1-méthyléthyl) phényl] estra-4,9-dièn-3-one.

On agite 2 heures à température ambiante 2,58 g de produit obtenu au stade précédent dans 20 $cm^3$ d'éthanol avec 5 $cm^3$ d'acide chlorhydrique 2N. On concentre à faible volume, sous pression réduite, extrait au chlorure de méthylène, lave, sèche et élimine le solvant sous pression réduite. On obtient 2,2 g de produit attendu que l'on purifie par chromatographie sur silice (éluant n-hexane-acétate d'éthyle 3—7).

*Spectre IR ($CHCl_3$):*

| | |
|---|---|
| OH Libre: | 3 620$^{cm^{-1}}$ + associé |
| diénone: | 1 655$^{cm^{-1}}$ −1 601$^{cm^{-1}}$ |
| aromatique: | 1 590$^{cm^{-1}}$. |

*Stade D:* (17R) 4',5'-dihydro 11β-[4-(1-méthyléthyl) phényl] spiro (estra 4,9-dièn-17,2'-(3H) furan) 3-one

On agite 1 heure et demie à température ambiante 1,15 g de produit obtenu précédemment, 20 cm³ de pyridine et 2,1 g de chlorure de tosyle. On dilue avec 50 cm³ d'eau et de glace, ajoute lentement 20 cm³ d'acide chlorhydrique concentré, extrait la phase aqueuse au chlorure de méthylène, sèche et élimine les solvants sous pressions réduite. On obtient 1,686 g de produit brut que l'on chromatographie sur silice (éluant chlorure de méthylène-acétone 95—5), puis recristallise dans l'éthanol. On récupère 659 mg de produit attendu F = 114°C.

*Spectre IR ($CHCl_3$):*

| | |
|---|---|
| cétone conjugée | 1 654cm$^{cm^{-1}}$ |
| C=C<br>aromatique | } 1 601$^{cm^{-1}}$ −1 510$^{cm^{-1}}$ |

*Analyse:* $C_{30}H_{38}O_2$ 430,635

| | C% | H% |
|---|---|---|
| Calculé | 83,68 | 8,89 |
| Trouvé | 83,8 | 9,1 |

Exemple 13

(17R) 11β-[3-(méthylthio) phényl] spiro (estra-4,9-dièn-17,2'-(5H) furan) 3-one

*Stade A:* (1,2-éthane diyl) acétal cyclique de 5α,17β-dihydroxy 11β-[3-(méthylthio) phényl] 17α-[3-(1-tétrahydro 2H-2-pyrannyloxy) 1-propényl] estr-9-èn-3-one.

On opère comme au stade B de l'exemple 8 en utilisant 2,5 g de produit préparé au stade A de l'exemple 8, 110 mg de chlorure cuivreux et 18 cm³ du magnésien préparé comme à l'exemple 3 à partir de 3-bromo thioanisole et titrant 1,17 M/l. On obtient 3 g de produit attendu.

*Spectre IR ($CHCl_3$):*

| | |
|---|---|
| OH Libre ≃ | 3 600$^{cm^{-1}}$ + associé 3 498$^{cm^{-1}}$ (max)<br>3 440$^{cm^{-1}}$ (épaulement) |
| aromatique | 1 587$^{cm^{-1}}$ 1 568$^{cm^{-1}}$ |

*Stade B:* (Z) 17β-hydroxy 17α-(3-hydroxy 1 propényl) 11β-[3-(méthylyhio) phényl] estra-4,9-dièn-3-one.

On mélange à température ambiante pendant 2 heures 3 g du produit obtenu précédemment, 30 cm³ d'éthanol et 5 cm³ d'acide chlorhydrique 2N. On concentre à faible volume sous pression réduite, extrait au chlorure de méthylène, lave, sèche et élimine les solvants sous pression réduite. On chromatographie le résidu sur silice (éluant cyclohexane-acétate d'éthyle 5/5) et récupère 1,083 g de produit attendu F = 168°C.

*Spectre IR ($CHCl_3$):*

| | |
|---|---|
| OH | 3 613$^{cm^{-1}}$ + associé 3 440$^{cm^{-1}}$ |
| diénone | 1 656$^{cm^{-1}}$ −1 601$^{cm^{-1}}$ |
| aromatique | 1 587$^{cm^{-1}}$ 1 569$^{cm^{-1}}$ 1 474$^{cm^{-1}}$ |

*Stade C:* (17R) 11β-[3-(méthylthio) phényl] spiro (estra 4,9-dièn-17,2'(5H) furan) 3-one

One opère comme au stade D de l'exemple 12 à partir de 1,050 g de produit obtenu au stade B précédent et obtient 592 mg de produit pur attendu F = 150°C.

*Spectre IR ($CHCl_3$):*

| | |
|---|---|
| diénone | 1 657$^{cm^{-1}}$ −1 601$^{cm^{-1}}$ |
| aromatique | 1 587$^{cm^{-1}}$ 1 569$^{cm^{-1}}$ 1 473$^{cm^{-1}}$ |
| —C—O—C | 1 080$^{cm^{-1}}$ −1 041$^{cm^{-1}}$ |

*Analyse:* $C_{28}H_{32}SO_2$ 432,62

| | C% | H% | S% |
|---|---|---|---|
| Calculé: | 77,7 | 7,46 | 7,41 |
| Trouvé: | 77,6 | 7,4 | 7,1 |

Exemple 14

(17R) 11β-(4-chlorophényl) spiro (estra-4,9-dièn-17,2'(5H) furan) 3-one

*Stade A:* (Z) (1,2-éthanediyl)acétal cyclique de 11β-(4-chlorophényl) 5α,17β-dihydroxy 17α-[3-(tétrahydro 2H-2-pyrannyloxy) 1-propényl] estr-9-èn-3-one.

On opère comme au stade B de l'exemple 8 à partir de 2,5 g de produit préparé au stade A de l'exemple 8, 160 mg de chlorure cuivreux, et 18 $cm^3$ d'une solution de magnésien préparé comme à l'exemple 3 à partir de 4-chloro bromobenzène et titrant 0,87 M/l. On obtient 3,082 g de produit pur attendu.

*Spectre IR:*
OH 3 500$^{cm^{-1}}$ + associé vers 3 405$^{cm^{-1}}$
aromatique 1 600$^{cm^{-1}}$ 1 490$^{cm^{-1}}$

*Stade B:* (Z) 11β-(4-chlorophényl) 17β-hydroxy 17α-(3-hydroxy 1-propényl) estra-4,9-dièn-3-one.

On agite 3 heures à température ambiante 3,05 g du produit obtenu au stade précédent dans 30 $cm^3$ d'éthanol et 30 $cm^3$ d'eau en présence de 3 g d'hydrogénosulfate de potassium.

On concentre à faible volume sous pression réduite, dilue dans l'eau, extrait au chlorure de méthylène, sèche et élimine les solvants sous pression réduite. On obtient 2,54 g de produit brut que l'on chromatographie sur silice (éluant cyclohexane-acétate d'éthyle 5—5), puis cristallise dans l'acétate d'éthyle F = 214°C.

*Spectre IR ($CHCl_3$):*
OH ≈ 3 611$^{cm^{-1}}$ + associé
diénone 1 657$^{cm^{-1}}$ −1 602$^{cm^{-1}}$
aromatique 1 013$^{cm^{-1}}$

*Stade C:* (17R) 11β-(4-chlorophényl) spiro (estra-4,9-dièn-17,2'(5H) furan) 3-one.

On opère comme au stade D de l'exemple 12 à partir de 1,03 g de produit obtenu au stade B précédent 20 $cm^3$ de pyridine, 3 g de chlorure de tosyle et 25 $cm^3$ d'acide chlorhydrique concentré. On obtient 726 mg de produit cristallisé attendu F >260°C.

*Spectre IR ($CHCl_3$):*
diénone 1 654$^{cm^{-1}}$ −1 602$^{cm^{-1}}$
aromatique 1 572$^{cm^{-1}}$ (épaulement) 1 490$^{cm^{-1}}$
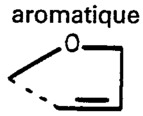 1 081$^{cm^{-1}}$ −1 039$^{cm^{-1}}$

*Analyse:* $C_{27}H_{29}ClO_2$ 420,98

| | C% | H% | S% |
|---|---|---|---|
| Calculé: | 77,03 | 6,94 | 8,42 |
| Trouvé: | 77,0 | 6,9 | 8,2 |

Exemple 15

(17R) 11β-[4-(1-méthyléthoxy) phényl] spiro (estra-4,9-dièn-17,2'-(5H) furan) 3-one.

*Stade A:* (Z) (1,2-éthane diyl) acétal cyclique de 5α,17β-dihydroxy 11β-[4-(1-méthyl éthoxy) phényl] 17α-[3-(tétrahydro-2H-2-pyrannyloxy) 1-propényl] estr-9-èn 3-one.

On opère comme au stade B de l'exemple 8 ein utilisant 2,5 g de produit obtenu comme indiqué au stade A de l'exemple 8, 110 mg de chlorure cuivreux et 35 $cm^3$ du magnésien prépare comme à l'exemple 3 à partir de 1-bromo 4-(1-méthyléthoxy) benzène. On obtient 6,764 g de produit brut que l'on chromatographie sur silice (éluant cyclohexane acétate d'éthyle 7—3 à 1% de triéthylamine) et récupère 3,173 g de produit attendu.

*Spectre IR ($CHCl_3$):*
OH en 5 3 500$^{cm^{-1}}$
autres OH 3 602$^{cm^{-1}}$ −3 458$^{cm^{-1}}$ (épaulement)
aromatique 1 608$^{cm^{-1}}$ 1 571$^{cm^{-1}}$ 1 505$^{cm^{-1}}$

*Stade B:* (Z) 17β-hydroxy 17α-(3-hydroxy 1-propényl) 11β-[4-(1-méthyléthoxy) phényl] estra-4,9-dièn-3-one.

On opére comme au stade B de l'exemple 13 à partir de 3,14 g de produit obtenu au stade A précédent, 20 $cm^3$ d'éthanol et 2 $cm^3$ d'acide chlorhydrique 2N. On obtient 1,257 g de produit pur attendu.

*Spectre IR ($CHCl_3$):*

OH 3 612$^{cm^{-1}}$ + associé

\
= O 1 656$^{cm^{-1}}$
/

\ /
C=C 1 608$^{cm^{-1}}$
/ \
aromatique 1 585$^{cm^{-1}}$ (épaulement)
1 506$^{cm^{-1}}$

*Stade C:* (17R) 11β-[4-(1-méthyléthoxy) phényl] spiro (estra-4,9-dièn-17,2'-(5H) furan) 3-one

On opère comme au stade D de l'exemple 12 au départ de 1,22 g de produit obtenu au stade B précédent 20 $cm^3$ de pyridine, 2,1 g de chlorure de tosyle et 25 $cm^3$ d'acide chlorhydrique concentré. On obtient 849 mg de produit pur que l'on cristallise dans l'étanol F = 155°C.

*Spectra IR ($CHCl_3$):*

diénone 1 656$^{cm^{-1}}$ —1 608$^{cm^{-1}}$
aromatique 1 506$^{cm^{-1}}$

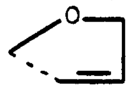 1 806$^{cm^{-1}}$ —1 040$^{cm^{-1}}$

*Analyse:* $C_{30}H_{36}O_3$ 444,619

| | C% | H% |
|---|---|---|
| Calculé: | 81,04 | 8,16 |
| Trouvé: | 81,2 | 8,0 |

Exemple 16

17(R) 11β-[4-(1-pyrrolidinyl] spiro (estra-4,9-dièn-17,2'-(5H) furan) 3-one

*Stade A:* (Z) (1,2-éthane diyl) acétal cyclique de 5α, 17β-dihydroxy 11β-[4-(1-pyrrolidinyl) phényl] 17α-[3-(tétrahydro 2H-2-pyrranyloxy) 1-propényl) estr-9-èn 3-one.

On opère comme au stade B de l'exemple 8 en utilisant 1,17 g de produit obtenu au stade A de l'exemple 8, 108 mg de chlorure cuivreux et 20 $cm^3$ de bromure de 4-(1-pyyrolidinyl) phényl magnésium titrant 1 M/l.

On obtient après extraction à l'éther, lavage à l'eau et concentration sous pression réduite 4,39 g de produit brut que l'on chromatographie sur silice (éluant cyclohexane-acétate d'éthyle 7/3) et récupère 2,3 g de produit attendu que l'on utilise tel quel pour le stade suivant.

*Stade B:* (Z) 17β-hydroxy 17α-(3-hydroxy 1-propényl) 11β-[4-(1-pyrrolidinyl) phényl] estra-4,9-dièn 3-one.

On agite pendant 2 heures et demie à température ambiante, puis 30 minutes à 50°C 2,3 g du produit préparé au stade A dans 25 $cm^3$ de méthanol avec 10 $cm^3$ d'acide chlorhydrique 2N. On refroidit, dilue à l'eau, alcalinise par 20 $cm^3$ de solution aqueuse d'hydroxyde de sodium N, puis avec une solution de carbonate acide de sodium. On extrait à l'acétate d'éthyle, lave à l'eau, sèche et élimine les solvants sous pression réduite. On obtient 1,825 g de produit attendu.

*Spectre IR ($CHCl_3$):*

OH 3 612$^{cm^{-1}}$ + associé
diénone 1 504$^{cm^{-1}}$
aromatique + —C=C } 1 614$^{cm^{-1}}$ 1 559$^{cm^{-1}}$ 1 518$^{cm^{-1}}$

*Stade C:* 17(R) 11β-[4-(1-pyrrolidinyl] spiroi (estra-4,9-dièn-17,2'-(5H) furan) 3-one

On opère comme à l'exemple 2 à partir de 1,4 g de produit obtenu au stade B précédent, 30 $cm^3$ de pyridine, 3 g de chlorure de tosyle en effectuant l'extraction à l'éther. On recueille 1,25 g de produit brut que l'on chromatographie sur silice (éluant cyclohexane-acétate d'éthyle 7/3, puis benzène-acétate d'éthyle 85—15). On obtient 0,9 g de produit attendu.

*Spectre IR ($CHCl_3$):*

diènone 1 654$^{cm^{-1}}$
C=C 1 600$^{cm^{-1}}$
bandes aromatiques 1 614$^{cm^{-1}}$ 1 559$^{cm^{-1}}$ 1 519$^{cm^{-1}}$
C—O—C cyclique 1 081$^{cm^{-1}}$ 1 040$^{cm^{-1}}$

*Analyse:* $C_{31}H_{37}NO_2$ 455,64

| | C% | H% | S% |
|---|---|---|---|
| Calculé: | 81,72 | 8,18 | 3,07 |
| Trouvé: | 81,6 | 8,2 | 3,0 |

Exemple 17

(17R) 11β-(2-thiényl) spiro (estra-4,9-dièn-17,2'-(5H) furan) 3-one

*Stade A:* (Z) (1,2-éthane diyl) acétal cyclique de 5α,17β-dihydroxy 17α-[3-(tétrahydro-2H-2-pyrannyloxy) 1-propényl] 11β-(2-thiényl) estr-9-èn-3-one.

On opère comme au stade B de l'exemple 8 à partir de 2,5 g de produit préparé comme au stade A de l'exemple 8, 110 mg de chlorure cuivreux et 22,2 $cm^3$ de magnésien préparé comme à l'exemple 3 à partir du 2-bromo thiophène, titrant 1,05 M/l.

On obtient 3,6 g de produit brut que l'on chromatographie sur silice (éluant cyclohexane-acétate d'éthyl 5/5) et récupère 2,196 g de produit attendu.

*Spectre IR ($CHCl_3$):*

| | |
|---|---|
| OH | 3 600 $^{cm^{-1}}$ + associé 3 500 $^{cm^{-1}}$ |
| thiophène | 1 520 $^{cm^{-1}}$ 852 $^{cm^{-1}}$ |

*Stade B:* (Z) 17β-hydroxy 17α-(3-hydroxy 1-propényl) 11β-(2-thiényl) estra-4,9-dièn-3-one.

On opère comme au stade B de l'exemple 13 à paire de 2,1 g de produit obtenu précédemment, 20 $cm^3$ d'éthanol, et 2 $cm^3$ d'acide chlorhydrique 2N. On obtient 1,1 g de produit pur attendu.

*Spectre IR ($CHCl_3$):*

| | |
|---|---|
| OH | 3 600 $^{cm^{-1}}$ + associé |
| diènone | 1 658 $^{cm^{-1}}$ 1 604 $^{cm^{-1}}$ |
| thiophène | 1 520 $^{cm^{-1}}$ |

*Stade C:* (17R) 11β-(2-thiényl) spiro (estra-4,9-dièn-17,2'-(5H) furan) 3-one.

On opère comme au stade D de l'exemple 12 au départ de 1,03 g de produit obtenu au stade précédent, 20 $cm^3$ de pyridine, 2,1 g de chlorure de tosyle et 25 $cm^3$ d'acide chlorhydrique concentré. On obtient 984 mg de produit pur que l'on cristallise dans l'éthanol F = 182°C.

*Spectra IR ($CHCl_3$):*

| | |
|---|---|
| diènone | 1 658 $^{cm^{-1}}$(F) −1 604 $^{cm^{-1}}$ |
| 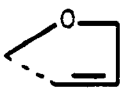 | 1 081 $^{cm^{-1}}$ 1 041 $^{cm^{-1}}$ |
| thiophène | 1 520 $^{cm^{-1}}$ |

*Analyse:* $C_{25}H_{28}SO_2$ 392,564

| | C% | H% | S% |
|---|---|---|---|
| Calculé: | 76,49 | 7,19 | 8,16 |
| Trouvé: | 76,4 | 7,3 | 8,1 |

Exemple 18

(17R) 11β-[4-(éthylthio) phényl] spiro (estra-4,9-dièn-17,2'-(5H) furan) 3-one

*Stade A:* (Z) (1,2-éthane diyl) acétal cyclique de 5α,17β-dihydroxy 11β-[4-(éthylthio) phényl] 17α-[3-(tétrahydro-2H-2-pyrannyloxy) 1-propényl] estr-9-èn-3-one.

Préparation du magnésien:

On opère comme à l'exemple 3 à partir de 1,125 g de magnésium et 9,3 g de 1-bromo 4-éthylthio benzène et obtient une solution de magnésien titrant 0,87 M/l.

Condensation

On opère comme au stade B de l'exemple 8 en utilisant 3 g du produit obtenu au stade A de l'exemple 8, 110 mg de chlorure cuivreux et 30 $cm^3$ du magnésien préparé ci-dessus. Après extraction au chlorure de méthlène, lavage, séchage et élimination des solvants sous pression réduite, on obtient 10 g de produit

brut que l'on chromatographie sur silice (éluant cyclohexane-acétate d'éthyle 5/5) et récupère 3,519 g de produit attendu.

*Spectre IR ($CHCl_3$):*

| | |
|---|---|
| OH | 3 600 $^{cm^{-1}}$ + associé 3 452$^{-1}$ |
| aromatique | 1 592 $^{cm^{-1}}$(f) 1 575 $^{cm^{-1}}$(f) 1 492 $^{cm^{-1}}$(F) |

*Stade B:* (Z) 11β-[4-(éthylthio) phényl] 17β-hydroxy 17α-(3-hydroxy 1-propényl) estra-4,9-dièn-3-one.

On agite pendant 30 minutes 3,474 g du produit obtenu au stade précédent dans 60 $cm^3$ de méthanol avec 2 $cm^3$ de chlorure de méthylène et 5 $cm^3$ d'acide chlorhydrique 2N. On concentre à faible volume, ajoute 10 $cm^3$ de solution aqueuse d'hydroxyde de sodium N et extrait au chlorure de méthylène. On lave à l'eaux, sèche, élimine les solvants sous pression réduite et obtient 2,574 g de produit brut que l'on chormatographie sur silice (éluant cyclohexane acétate d'éthyle 3—7) et cristallise le résidu obtenu dans l'éthanol. On receuille 1,052 g de produit pur attendu F ≃ 100°C.

*Spectre IR ($CHCl_3$):*

| | |
|---|---|
| OH | 3 615 $^{cm^{-1}}$ + associé |
| \| C=O | 1 654 $^{cm^{-1}}$ |
| \| —C=C— | 1 601 $^{cm^{-1}}$ 1 556 $^{cm^{-1}}$ 1 492 $^{cm^{-1}}$ |

*Stade C:* (17R) 11β-[4-(éthylthio) phényl] spiro (estra-4,9-dièn-17,2'-(5H) furan) 3-one.

On opère comme à l'exemple 12 stade D à partir de 600 mg du produit obtenu au stade precédent, 15 $cm^3$ de pyridine, 1,5 g de chlorure de tosyle.

On obtient 872 mg de produit brut que l'on chromatographie sur silice (éluant cyclohexane-acétate d'éthyle 5/5) et cristallise dans l'éthanol F = 100°C.

*Spectre IR ($CHCl_3$):*

| | |
|---|---|
| cétone conjugué | 1 654 $^{cm^{-1}}$ —1 601 $^{cm^{-1}}$ |
| aromatique | 1 558 $^{cm^{-1}}$ 1 492 $^{cm^{-1}}$ |
| 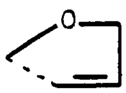 | 1 081 $^{cm^{-1}}$ |

*Analyse:* $C_{30}H_{34}O_2S$ 446,657

| | C% | H% | S% |
|---|---|---|---|
| Calculé: | 77,98 | 7,67 | 7,19 |
| Trouvé: | 77,9 | 7,7 | 7,1 |

## Exemple 19

(17R) 11β-(4-(4,5-dihydro 4,4-diméthyl 2-oxazolyl) phényl] spiro (estra-4,9-dièn-17,2'-(5H) furan) 3-one.

*Stade A:* (Z) (1,2-éthane diyl) acétal cyclique de 11β-[4-(4,5-dihydro 4,4-diméthyl 2-oxazolyl) phényl] 5α,17β-dihydroxy 17α-[3-tétrahydro-2H-2-pyrannyloxy) 1-propényl] estr-9-ène-3-one.

Préparation du magnésien:

On opère comme à l'exemple 3 à partir de 3,5 g de magnésium et 25 g de 2-(4-bromophényl) 4,5-dihydro 4,4-diméthyl oxazole et obtient une solution titrant 0,74 M/l.

Condensation:

On opère à l'exemple 3 à partir de 80 $cm^3$ de la solution de magnésium ci-dessus, 800 mg de chlorure cuivreux et 10 g du produit préparé comme au stade A de l'exemple 8. Après chromatographie sur silice (éluant cyclohexane-acétate d'éthyle 3/7), on obtient 14,3 g de produit attendu utilisé tel quel pour le stade suivant.

*Spectre IR ($CHCl_3$):*

| | |
|---|---|
| 17-OH | 3 605 $^{cm^{-1}}$ + associé 3 420 $^{cm^{-1}}$ |
| 5-OH | 3 505 $^{cm^{-1}}$ |
| \\ C=N / | 1 646 $^{cm^{-1}}$ |
| aromatique | 1609 $^{cm^{-1}}$ 1 563 $^{cm^{-1}}$ 1 509 $^{cm^{-1}}$ |

*Stade B:* (Z) 11β-[4-(4,5-dihydro 4,4-diméthyl 2-oxazolyl) phényl] 17β-hydroxy 17α-(3-hydroxy 1-propényl) estra-4,9-dièn-3-one.

On dissout sous atmosphère inerte 13,1 g du produit précédent dans 130 $cm^3$ de dioxanne et 130 $cm^3$ d'acide chlorhydrique 2N, agite 1 heure, verse la solution dans une solution aqueuse de bicarbonate de sodium, extrait à l'acétate d'éthyle, lave à l'eau, séche et élimine sous pression réduite. On obtient 10,3 g de produit brut que l'on recristallise dans l'éther. F = 269°C.

*Spectre IR ($CHCl_3$):*
C=O + C=N 1 648$^{cm-1}$
C=C diénone } ≃ 1603$^{cm-1}$
aromatique }
bandes aromatiques 1 563$^{cm-1}$ —1 510$^{cm-1}$

*Stade C:* (17R) 11β-[4-(4,5-dihydro 4,4-diméthyl 2-oxazolyl) phényl] spiro (estra-4,9-dièn-17,2'-(5H) furan) 3-one.

On met en suspension 0,49 g de produit obtenu précédemment dans 10 $cm^3$ de pyridine, ajoute 1 g de chlorure de tosyle, agite 2 heures et demie, dilue avec de l'eau glacée, essore le précipité et le dissout dans du chlorure de méthylène, sépare la phase organique, la sèche et élimine les solvants sous pression réduite. On chromatographie le résidu sur silice (éluant: chlorure de méthylène-acétate d'éthyl 1—1) et obtient 0,41 g de produit attendu. F = 250°C.

*Spectre IR ($CHCl_3$):*
C=O + C=N 1 648$^{cm-1}$
C=C diénone } 1603$^{cm-1}$ 1 503$^{cm-1}$ 1509$^{cm-1}$
aromatique }
C—O—C 1 080$^{cm-1}$

*Analyse:* $C_{32}H_{37}NO_3$ 483,65

| | C% | H% | N% |
|---|---|---|---|
| Calculé: | 79,47 | 7,71 | 2,89 |
| Trouvé: | 79,5 | 7,8 | 2,8 |

Exemple 20

(17 R) 11β-[4-[2-diméthylamino) éthoxy] phényl] spiro (estra-4,9-dièn-17,2'-(5H) furan) 3-one.

*Stade A:* (Z) (1,2-éthanediyl) acétal cyclique de 5α,17β-dihydroxy 11β-[4-(2-diméthylamino) éthoxy] phényl] 17α-[3-(tétrahydro-2H-2-pyrannyloxy) 1-propényl] estr -9-èn-3-one.

On opère comme au stade B de l'exemple 8 en utilisant du départ 75,5 $cm^3$ d'une solution de magnésien prépare à partir deu 4-(N,N-diméthyl amino éthoxy) bromobenzène titrant 0,7 M/l, 0,25 g de chlorure cuivreux et 5 g de produit préparé comme au stade A de l'exemple 8. On obtient après chromatographie sur silice (éluant: acétate d'éthyle-triéthylamine 93—2) 2,620 g de produit attendu.

*Spectre IR ($CHCl_3$):*
C
/
—N 2780$^{cm-1}$
\
C
aromatique 1 609$^{cm-1}$ 1 581$^{cm-1}$ 1 508$^{cm-1}$

*Stade B:* (Z) 11β-[4-[2-(diméthylamino) éthoxy] phényl] 17β-hydroxy 17α-(3-hydroxy 1-propényl) estra-4,9-dièn-3-one.

On refroidit à 0°C une solution comprenant 2,62 g de produit obtenu ci-dessus dans 13 $cm^3$ d'éthanol, ajoute 7,9 $cm^3$ d'acide chlorhydrique 2N, agite 30 minutes, ajoute 6 $cm^3$ d'hydroxyde d'ammonium, ajoute 25 $cm^3$ d'eau et agite de nouveau pendant 30 minutes. On essore le précipité forme, le dissout dans du chlorure de méthylène, séche et concentre à sec sous pression réduite. On obtient 1,948 g de produit brut que l'on chromatographie sur silice (éluant: acétate d'éthyle-triéthylamine 8—2) puis recristallise le résidu dans l'isopropanol. F = 136°C.

*Spectre IR ($CHCl_3$):*

| | |
|---|---|
| OH | 3 613$^{cm-1}$ |
| diénone | 1 656$^{cm-1}$ –1 608$^{cm-1}$ |
| aromatique | 1 582$^{cm-1}$ épaulement, 1 508$^{cm-1}$ (F), 832$^{cm-1}$ |

*Stade C:* (17R) 11β-[4-[2-(diméthylamino) éthoxy] phényl] spiro (estra-4,9-dièn-17,2'-(5H) furan) 3-one.

On opère comme au stade D de l'exemple 12 au départ de 2,268 g de produit préparé au stade B précédent, 45 cm$^3$ de pyridine, 4,5 g de chlorure de tosyle et 45 cm$^3$ d'acide chlorhydrique 6N. On obtient après chromatographie (éluant: acétate d'éthyle-triéthylamine 95—5) et cristallisation dans l'isopropanol 0,815 g de produit pur attendu. F = 136°C.

$[\alpha]_D = +156,5° \pm 3°$ (c = 0,6% $CHCl_3$)

*Spectre IR:*

| | |
|---|---|
| C=O | 1 656$^{cm-1}$ |
| C=C | 1 580$^{cm-1}$ |
| aromatique | 1 608$^{cm-1}$ 1 508$^{cm-1}$ |

## Exemple 21

(17R) 11β-[4-[2-(méthylthio) éthoxy] phényl] spiro (estra-4,9-dièn-17,2'-(5H) furan) 3-one.

*Stade A:* (1,2-éthaneidyl) acétal cyclique de 5α, 17β-dihydroxy 11β-[4-[2-(méthylthio) éthoxy] phényl] 17α-[3-(tétrahydro-2H-2-pyrannyloxy) 1-propényl] estr-9-èn-3-one.

Préparation du magnésien.

On opère comme à l'exemple 3 en utilisant 0,8 g de magnésien et 6,1 g de 4-bromo 2-[(méthylthio) éthoxy] benzène. On obtient une solution titrant 0,59 M/l.

Condensation.

On opère comme au stade B de l'exemple 8 en partant de 0,472 g de produit préparé comme au stade A de l'exemple 8,22 mg de chlorure cuivreux et 8,3 cm$^3$ de la solution de magnésien préparée ci-dessus. On obtient 0,46 g de produit attendu.

*Spectre IR ($CHCl_3$):*

| | |
|---|---|
| 17-OH | 3 600$^{cm-1}$ + associé |
| 5-OH | 3 505$^{cm-1}$ |
| aromatique | 1 608$^{cm-1}$ 1 580$^{cm-1}$ 1 507$^{cm-1}$ |

Le 4-bromo 2-[(méthylthio) éthoxy] benzène utilisé au départ du stade A a été préparé comme suit.

On dissout sous atmosphère inerte 73,9 g de parabromophénol dans 430 cm$^3$ d'une solution aqueuse normale d'hydroxyde de sodium, ajoute en 5 minutes 47,3 g de 2-chloroéthylméthyl sulfure et chauffe au reflux 18 heures. On refroidit, essore le chlorure de sodium formé, lave à l'éthanol, sèche et élimine les solvants sous pression réduite. On reprend le résidu à l'eau, extrait à l'éther, lave la phase organique avec une solution aqueuse normale d'hydroxyde de sodium, puis à l'eau, sèche et évapore à sec. On chromatographie le résidu sur silice (éluant: hexaneacétate d'éthyle 95—5) et recueille 87 g de produit attendu, utilisé tel quel pour la préparation du magnésien.

*Stade B:* 17β-hydroxy 17α-(3-hydroxy 1-propényl) 11β-[4-[2-(méthylthio) éthoxy] phényl] estra-4,9-dièn-3-one.

On opère comme au stade B de l'exemple 18 en utilisant 2,75 g de produit préparé au stade A precédént, 60 cm$^3$ de méthanol, 10 cm$^3$ de chlorure de méthylène et 6 cm$^3$ d'acide chlorhydrique 2N. Après chromatographie sur silice (éluant: chlorure de méthylène-acétone 85—15), on obtient 3 g de produit que l'on utilise tel quel pour le stade suivant.

*Spectre IR ($CHCl_3$):*

| | |
|---|---|
| C=O | 1 710$^{cm-1}$ |
| aromatique | 1 607$^{cm-1}$ 1 507$^{cm-1}$ 1 572$^{cm-1}$ |

*Stade C:* (17R) 11β-[4-[2-(méthylthio) éthoxy] phényl] spiro (estra-4,9-dièn-17,2'-(5H) furan) 3-one.

On opère comme au stade D de l'exemple 12 à partir de 1,4 g de produit préparé ci-dessus, 28 cm$^3$ de pyridine, 2,26 g de chlorure de tosyle et 28 cm$^3$ d'acide chlorhydrique 6N. On obtient 1,82 g de produit cristallisé attendu.

$[\alpha]_D = +184,5° \pm 2°$ (c = 1% $CHCl_3$)

*Spectre IR ($CHCl_3$):*

| | |
|---|---|
| C=O | 1,656$^{cm-1}$ |
| C=C | 1 608$^{cm-1}$ 866$^{cm-1}$ (def) |
| aromatique | 1 508$^{cm-1}$ |
| C—O—C | 1 082$^{cm-1}$ –1 402$^{cm-1}$ |

*Analyse: $C_{30}H_{36}O_3S$: 476,68*
(avec solvatation EtOH 5%)

| | C% | H% | S% |
|---|---|---|---|
| Calculé: | 74,42 | 7,89 | 6,39 |
| Trouvé: | 74,5 | 7,9 | 6,4 |

Exemple 22

(17R) 11β-(3-méthoxyphényl)spiro (estra 4,9-dièn-17,2'-(5H) furan) 3-one

*Stade A:* (Z) diméthyl cétal de 5α,17β-dihydroxy 11β-(3-méthoxy phényl) 17-[3-(tétrahydro-2H-2-pyrannyloxy) 1-propényl] estr-9-èn-3-one.

On opère comme à l'exemple 5 C au départ de 3 g de produit préparé comme à l'exemple 5 B, 190 mg de chlorure cuivreux et 19 $cm^3$ de solution de magnésien préparé à partir du 3-bromo anisole et titrant 1 M/l. On obtient, après chromatographie sur silice (éluant cyclohexaneacétate d'éthyle 7—3 à 1% de triéthylamine) 1,522 g de produit attendu.

*Stade B:* (Z) 17β-hydroxy 17α-(3-hydroxy 1-propényl) 11β-(3-méthoxyphényl) estra-4,9-dièn-3-one.

On opère comme à l'exemple 5D, en partant de 1,5 g de produit obtenu précédemment et 1,5 g d'hydrogénosulfate de potassium. Après chromatographie sur silice éluant n-hexane-acétate d'éthyle 7—3), on obtient 0,8 g de produit attendu F = 167—168°C.

*Spectre IR ($CHCl_3$):*
OH 3 613$^{cm^{-1}}$ + associé
diénone 1 656$^{cm^{-1}}$ 1 603$^{cm^{-1}}$
aromatique 1 598$^{cm^{-1}}$ 1 583$^{cm^{-1}}$ 1 487$^{cm^{-1}}$

*Stade C:* (17R) 11β-(3-méthoxyphényl) spiro (estra-4,9-dièn-17,2'-(5H) furan) 3-one.

On opère comme au stade E de l'exemple 5 en utilisant 0,77 g de produit préparé au stade B précédent, 20 $cm^3$ de pyridine, 2,7 g de chlorure de tosyle et 25 $cm^3$ d'acide chlorhydrique concentré. Après chromatographie sur silice (éluant cyclohexane-acétate d'éthyle 5—5), on obtient 0,624 g de produit attendu que l'on cristallise dans l'éthanol F = 170°C.

*Spectre IR ($CHCl_3$):*
C=O 1 656$^{cm^{-1}}$ (F)
C=C }
aromatique } 1 604$^{cm^{-1}}$ 1 598$^{cm^{-1}}$ 1 583$^{cm^{-1}}$
C-O-C 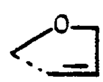 1 080$^{cm^{-1}}$ −1 040$^{cm^{-1}}$

*Analyse:* $C_{28}H_{32}O_3$ 416,565

| | C% | H% |
|---|---|---|
| Calculé: | 80,73 | 7,74 |
| Trouvé: | 80,4 | 7,9 |

Exemple 23

(17R) 11β-(4-méthoxyphényl) spiro (estra-4,9-dièn-17,2'-(5H)-furan) 3-one.

*Stade A:* (Z) diméthyl cétal de 5α,17β-dihydroxy 11β-(4-méthoxyphényl) 17α-[3-(tétrahydro-2H-2-pyrannyloxy) 1-propényl] estr-9-èn-3-one.

On opère comme au stade C de l'exemple 5 en utilisant au départ 3 g du produit préparé au stade B de l'exemple 5, 190 mg de chlorure cuivreux et 19 $cm^3$ de solution de magnésien préparé à partir du 4-bromo anisole et titrant 1 M/l. Après chromatographie sur silice (éluant éther de pétrole (Eb 40°—70°C)-acétate d'éthyle 7—3 à 1% de triéthylamine), on obtient 1,078 g de produit attendu et 0,625 g de l'isomère 5β-hydroxy 11α-(4-méthoxyphényl) correspondant.

*Stade B:* (Z) 17β-hydroxy 17α-(3-hydroxy 1-propényl) 11β-(4-méthoxyphényl) estra-4,9-dièn-3-one.

On opère comme à l'exemple 5, stade D, en partant de 1 g deproduit préparé au stade A précédent et 1 g d'hydrogèné sulfate de potassium. On obtient 736 mg de produit brut que l'on utilise tel quel dans le stade suivant.

*Stade C:* (17R) 11β-(4-méthoxyphényl) spiro (estra 4,9-dièn 17,2'-(5H) furan) 3-one.

On opère comme au stade E de l'exemple 5 au départ de 736 mg de produit obtenu précédemment, 20 $cm^3$ de pyridine, 2,1 g de chlorure de tosyle et 25 $cm^3$ d'acide chlorhydrique concentré. Après chromatographie sur silice (éluant cylohexane-acétate d'éthyle 7—3), on obtient 383 mg de produit attendu que l'on cristallise dans l'éthanol F = 185°C.

*Spectre IR ($CHCl_3$):*

diénone 1 656$^{cm^{-1}}$ —1 607$^{cm^{-1}}$ 864$^{cm^{-1}}$

aromatique 1 582$^{cm^{-1}}$ 1 509$^{cm^{-1}}$

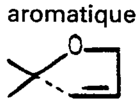 975$^{cm^{-1}}$ —1 080$^{cm^{-1}}$

Exemple 24

(17R) 4',5'-dihydro 11β-[2,3-dihydro 1-méthyl(1H) indol 5-yl] spiro (estra 4,9-dièn 17,2'-(3H) furan) 3-one

*Stade A:* (1,2-éthanediyl) acétal cylcique de 11β-[2,3-dihydro 1-méthyl(1H)-indol 5-yl] 5α, 17β-dihydroxy 17α-[3-tétrahydro-2H-2-pyrannyloxy) propyl] estr-9-èn-3-one.

On opère comme au stade B de l'exemple 12 en utilisant du départ 2.45 g de produit préparé comme au stade A de l'exemple 12, 110 mg de chlorure cuivreux et 35 $cm^3$ de solution de magnésien préparé comme à l'exemple 3 à partir de 5-bromo 2-méthyl indoline et titrant 0,6 M/l. On obtient 1,218 g de produit attendu.

*Spectre IR ($CHCl_3$):*

5-OH 3 500$^{cm^{-1}}$

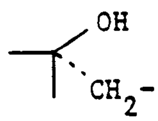 doublet 3 615$^{cm^{-1}}$ 3 593$^{cm^{-1}}$

aromatique 1 612$^{cm^{-1}}$ —1 496$^{cm^{-1}}$

*Stade B:* 11β-[2,3-dihydro 1-méthyl (1H)-indol-5-yl] 17β-hydroxy 17α-(3-hydroxy propyl) estra-4,9-dièn-3-one.

On opère comme au stade C de l'exemple 12 au départ de 3,025 g de produit préparé comme indiqué au stade précédent, 30 $cm^3$ d'éthanol et 5 $cm^3$ d'acide chlorhydrique 2N. On récupère 1,658 g de produit attendu.

*Spectre IR ($CHCl_3$):*

OH libre 3 619$^{cm^{-1}}$ + associé

cétone conjuguée 1 655$^{cm^{-1}}$

aromatique 1 611$^{cm^{-1}}$ —1 497$^{cm^{-1}}$

*Stade C:* (17R) 4',5'-dihydro 11β-[2,3-dihydro 1-méthyl(1H) indol 5-yl] spiro (estra-4,9-dièn-17,2'-(3H) furan) 3-one.

On opère comme au stade D de l'exemple 12 au départ de 1,658 g de produit préparé au stade B, 20 $cm^3$ de pyridine, 2,1 g de chlorure de tosyle et 20 $cm^3$ d'acide chlorhydrique concentré. Après chromatographie sur silice (éluant cyclohexane-acétate d'éthyle 7—3), on obtient 0,729 g de produit pur attendu.

*Spectre IR ($CHCl_3$):*

cétone conjuguée 1 633$^{cm^{-1}}$

aromatique + C=C } 1 602$^{cm^{-1}}$ 1 497$^{cm^{-1}}$

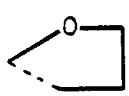 probable

*Analyse:* $C_{30}H_{37}NO_2$ 443,634

| | C% | H% | N% |
|---|---|---|---|
| Caculé: | 81,22 | 8,41 | 3,16 |
| Trouvé: | 81,0 | 8,6 | 3,1 |

Exemple 25

(17R) 4',5'-dihydro 11β-[4-[(3-méthylbutyl) thio] phényl] spiro (estra-4,9-dièn-17,2'-(3H) furan) 3-one

*Stade A:* (1,2-éthane diyl) acétal cyclique de 5α,17β-dihydroxy 11β-[4-[(3-méthylbutyl)thiol phenyl] 17α-[3-(tétrahydro 2H-2-pyrannyloxy) propyl] estr-9-èn-3-one.

On opère comme au stade B de l'exemple 12 au départ de 3,5 g du produit préparé au stade A de l'exemple 12, 1,24 g de chlorure cuivreux et 22 $cm^3$ d'une solution de magnésien préparé comme à l'exemple 3 à partir de 4-[(3-méthyl) butyl thio] bromo benzène et titrant 1,05 M/l.

Après chromatographie sur silie (éluant chlorure de méthylèneacétone 95—5 à 1% de triéthylamine), on obtient 4,08 g de produit attendu.

*Spectre IR ($CHCl_3$):*

| | |
|---|---|
| OH | 3 620$^{cm^{-1}}$ —3 597$^{cm^{-1}}$ + associé |
| aromatique | 1 594$^{cm^{-1}}$(f) 1 552$^{cm^{-1}}$(f) 1 492$^{cm^{-1}}$ (f) |

*Stade B:* 17β-hydroxy 17α-(3-hydroxy propyl) 11β-[4-[(3-méthyl butyl) thio] phényl] estra-4,9-dièn-3-one.

On opère comm au stade B de l'exemple 16 au départ de 4,05 g de produit préparé ci-dessus, 40 $cm^3$ de méthanol, 12 $cm^3$ d'acide chlorhydrique 2N. On obtient 3,34 g de produit brut que l'on chromatographie sur silice (éluant chlorure de méthylène-acétate d'éthyle 3—7) et récupère 2,3 g de produit pur attendu.

*Spectre IR ($CHCl_3$):*

| | |
|---|---|
| OH libre | 3 620$^{cm^{-1}}$ + associé |
| diénone | 1 654$^{cm^{-1}}$ 1 601$^{cm^{-1}}$ |
| aromatique | 1 554$^{cm^{-1}}$ —1 492$^{cm^{-1}}$ |

*Stade C:* (17R) 4',5'-dihydro 11β-[4-[(3-méthylbutyl) thio] phényl] spiro (estra-4,9-dièn-17,2'-(3H) furan) 3-one.

On opère comme à l'exemple 2 enpartant de 2,3 g de produit obtenu ci-dessus, 45 $cm^3$ de pyridine, 4,3 g de chlorure de tosyle.

On obtient 2,14 g de produit brut que l'on purifie par recristallisation dans l'éthanol F = 200°C.

*Spectre IR ($CHCl_3$):*

| | |
|---|---|
| C=O | 1 653$^{cm^{-1}}$ |
| —C=C } aromatique | 1 602$^{cm^{-1}}$ 1 555$^{cm^{-1}}$ 1 492$^{cm^{-1}}$ |
| C—O—C | 1 078$^{cm^{-1}}$ —1 055$^{cm^{-1}}$ |

Exemple 26

(17R) 4',5'-dihydro 11β-[4-(1-pyrrolidinyl) phényl] spiro (estra-4,9-dièn-17,2'-(3H) furan) 3-one.

*Stade A:* (1,2-éthane diyl) acétal cyclique de 5α,17β-dihydroxy 11β-[4-(1-pyrrolidinyl) phényl] 17α-[3-[(tétrahydro-2H-pyrannyloxy) 1-propynyl] estr-9-èn-3-one.

On opère comme au stade B de l'exemple 3 en partant de 3,5 g de produit préparé comme au stade A de l'exemple 3, 73 mg de chlorure cuivreux et 23 $cm^3$ de solution de magnésien préparé comme indiqué à l'exemple 3 à partir de la N-(4-bromophényl) pyrrolidine et titrant 1,3 M/l.

Après chromatographie sur silice (éluant chlorure de méthylèneacétone 92—8 à 1% de triéthylamine puis acétate d'éthyle — n-hexane 5—5 à 2% de triéthylamine), on obtient 3,52 g de produit pur attendu.

*Spectre IR ($CHCl_3$):*

| | |
|---|---|
| 17-OH | 3 599$^{cm^{-1}}$ |
| 5-OH | 3 508$^{cm^{-1}}$ |
| aromatique | 1 615$^{cm^{-1}}$ 1 599$^{cm^{-1}}$ 1 517$^{cm^{-1}}$ |

*Stade B:* (1,2-éthane diyl) acétal cyclique de 5α,17β-dihydroxy 11β-[4-(1-pyrrolidinyl) phényl] 17α-[3-(tétra-hydro-2H-2-pyrannyloxyl] propyl] estr-9-èn-3-one.

On opère comme à l'exemple 4, stade A, au départ de 2,62 g de produit préparé ci-dessus, dissous dans 172 cm3 d'une solution (1—1) de benzène et d'éthanol et de 1,048 g de réactif de Wilkinson, en hydrogénant pendant 4 heures 45 minutes.

On obtient 2,1 g de produit attendu.

*Spectre IR ($CHCl_3$):*

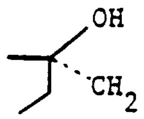 doublet $3\,620^{cm^{-1}}$ $3\,600^{cm^{-1}}$

5 OH $3\,505^{cm^{-1}}$
aromatique $1\,614^{cm^{-1}}$ $-1\,517^{cm^{-1}}$

*Stade C:* 17β-hydroxy 17α-(3-hydroxy propyl) 11β-[4-(1-pyrrolidinyl) phényl] estra-4,9-dièn-3-one.

On opère comme au stade B de l'exemple 4, en partant de 2,09 g de produit obtenu ci-dessus, 32 $cm^3$ de méthanol et 10,45 $cm^3$ d'acide chlorhydrique 2N.

Après chromatographie sur silice (éluant chlorure de méthylèneméthanol 95—5 à 2% de triéthylamine), on obtien 1,31 g de produit attendu.

*Spectre IR ($CHCl_3$):*
diénone $1\,654^{cm^{-1}}$ $-1\,590^{cm^{-1}}$
aromatique $1\,614^{cm^{-1}}$ $1\,559^{cm^{-1}}$ $1\,518^{cm^{-1}}$
OH $3\,620^{cm^{-1}}$ + associé

*Stade D:* (17R) 4',5'-dihydro 11β-[4-(1-pyrrolidinyl) phényl] spiro (estra-4,9-dièn-17,2'-(3H) furan) 3-one.

On opère comme au stade C de l'exemple 4 au départ de 1,28 g de produit obtenu au stade précédent, 20 $cm^3$ de pyridine et 2,6 g de chlorure de tosyle.

Après chromatographie sur silice (éluant cyclohexane-acétate d'éthyle 75—25 à 2% de triéthylamine, on obtient 0,73 g de produit attendu.

*Spectre IR ($CHCl_3$):*
diénone $1\,653^{cm^{-1}}$ (F)
C=C $1\,600^{cm^{-1}}$ (épaulement)
aromatique $1\,614^{cm^{-1}}$ (F) $1\,559^{cm^{-1}}$ $1\,518^{cm^{-1}}$ (F)

*Analyse:* $C_{31}H_{39}NO_2$ 457,66

| | C% | H% | N% |
|---|---|---|---|
| Calculé: (solvatation 12% cyclohexane) | 81,86 | 9,27 | 2,69 |
| Trouvé: | 81,6 | 9,4 | 2,6 |

## Exemple 27

(17R) 11β-[4-(méthylthio) phényl] spiro (estra-1,3,5,(10) trièn-17,2'-(5H) furan) 3-ol

1). *Formation de l'acetate phénolique:*

On refroidit à +3°C 0,5 g de produit préparé à l'exemple 3E en solution dans 10 $cm^3$ de chlorure de méthylène, puis ajoute goutte à goutte 0,5 $cm^3$ d'anhydride acétique, puis 0,25 $cm^3$ de bromure d'acétyle et agite 50 minutes. On dilue le milieu réactionnel par 20 $cm^3$ d'eau glacée, ajoute 5 $cm^3$ d'une solution aqueuse d'hydroxyde de sodium N, agite 30 minutes, extrait au chlorure de méthylène, lave la phase organique, la sèche et élimine les solvants sous pression réduite. On obtient 0,584 g de produit brut que l'on chromatographie sur silice (éluant n hexane-acétate d'éthyle 7—3) et récupère 0,241 g de l'acétate attendu.

2). *Saponification:*

On reprend le produit obtenu ci-dessus dans 3 $cm^3$ d'éthanol, ajoute 4 gouttes d'une solution aqueuse d'hydroxyde de sodium N, agite, dilue avec 10 $cm^3$ d'eau, filtre le précipité, le lave, le sèche sous pression réduite et le purifie par chromatographie sur silice (éluant n-hexane-acétate d'éthyle 8—2). On obtient 0,205 g de produit attendu.

*Spectre IR ($CHCl_3$):*
OH $3\,600^{cm^{-1}}$
aromatique $1\,602^{cm^{-1}}$ $1\,582^{cm^{-1}}$ $1\,434^{cm^{-1}}$

Exemple 28

(17R) 11β-[4-[2-(méthylthio) éthoxy] phényl] spiro (estra-1,3,5,(10) trièn-17,2'-(5H) furan) 3-ol et son acétate et le (17R) 11β-[4-[2-méthylthio) éthoxy] phényl] spiro (estra-5(10),9(11)-dièn-17,2'-(5H) furan) 3-one.

1). *Formation de l'acetate phénolique:*

On refroidit à environ +5°C une solution comprenant 1 g de produit obtenu à l'exemple 21 dans 20 $cm^3$ de chlorure de méthylène, ajoute 1 $cm^3$ d'anhydride acétique et 0,5 $cm^3$ de bromure d'acétyle, puis laisse revenir à température ambiante. On agite 50 minutes, verse dans une solution glacée de bicarbonate de sodium, agite 10 minutes, extrait au chlorure de méthylène, sépare les phases organiques, les lave à l'eau, les sèche et élimine les solvants sous pression réduite. On chromatographie le résidu sur silice (éluant hexane-acétate d'éthyle 80—20, puis 85—15) 0,509 g de l'acétate de (17R) 11β-[4-[2-(méthylthio) éthoxy] phényl] spiro (estra-1,3,5(10)-trièn-17,2'-(5H) furan) 3-ol.

*Spectre IR ($CHCl_3$):*

C=O 1 746$^{cm^{-1}}$

presence de 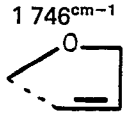

bande moyenne 1 664$^{cm^{-1}}$

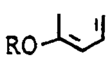

d'autre part 90 mg de (17R) 11β-[4-[2-(méthylthio) éthoxy] phényl] spiro [estra 5(10) 9(11)-dièn-17,2'-(5H) furan] 3-one

*Spectre IR ($CHCl_3$):*

C=O 1 711$^{cm^{-1}}$

C=C } artomatique 1 607$^{cm^{-1}}$ 1 507$^{cm^{-1}}$ 1 507$^{cm^{-1}}$

2). *Saponification:*

On reprend 0,5 g de l'acétate phénolique ci-dessus dans 7,5 $cm^3$ de méthanol et 1 $cm^3$ de chlorure de méthylène, puis ajoute goutte à goutte 0,5 $cm^3$ de lessive de soude et agite 20 minutes à température ambiante. On dilue à l'eau, acidifie à l'aide d'acide chlorhydrique 2N, extrait au chlorure de méthylène, lave à l'eau, sèche et élimine les solvants sous pression réduite, chromatographie le résidu sur silice (éluant hexane acétate d'éthyle 8—2), puis chlorure de méthylène et obtient 354 mg de (17R) 11β-[4-[2-(méthylthio) éthoxy] phényl] spiro (estra-1,3,5(10)-trièn-17,2'-(5H) furan) 3-ol attendu que l'on recristallise dans l'isopropanol F = 198°C

*Spectre IR ($CHCl_3$):*

OH phénolique 3 599$^{cm^{-1}}$

aromatique 1 610$^{cm^{-1}}$ 1 582$^{cm^{-1}}$ 1 511$^{cm^{-1}}$

C-O-C 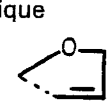 1 086$^{cm^{-1}}$ –1 037$^{cm^{-1}}$

$[\alpha]_D$: –84,5° ± 1,5° (c = 0,8% $CHCl_3$)

Exemple 29

(17R) 2β-méthyl 11β-[4-(méthylthio) phényl] spiro (estra-4,9-diène-17,2'-(5H)-furan 3-one et l'isomère 2α-méthyl correspondant et (17R) 2,2-diméthyl 11β-[4-(méthylthio) phényl] spiro (estra-4,9-dièn-17,2'-(5H) furan) 3-one.

On refroidit à –65°C/–70°C sous atmosphère inerte 2,45 $cm^3$ de n-butyl lithium en solution dans l'hexane (1,6 M) et 5 $cm^3$ de tétrahydrofuranne et ajoute en 20 minutes 0,66 $cm^3$ de cyclohexyl isopropylamine dans 5 $cm^3$ de tétrahydrofuranne; on agite 15 minutes, ajoute en 30 minutes 1,4 g de (17R) 11β-[4-(méthylthio) phényl] spiro (estra-4,9-dièn-17,2'-(5H)-furan) 3-one obtenu à l'exemple 3 en suspension dans 15 $cm^3$ de tétrahydrofuranne, agite 15 minutes, ajoute 0,4 $cm^3$ d'iodure de méthyle. On laisse revenir à température ambiante, agite 1 heure, ajoute 20 $cm^3$ d'une solution aqueuse de chlorure d'ammonium, décante, lave la phase organique à l'eau salée, réextrait à l'acétate d'éthyle, sèche et concentre à sec. On obtient 1,5 g de produit brut que l'on chromatographie sur silice en éluant par un mélange d'éther de pétrole ((Eb 40°/70°C) acétate d'éthyl 9—1).

On recueille 290 mg d'isomère (17R) 2β-méthyl 11β-[4-(méthylthio) phényl] spiro (estra-4,9-diène-17,2'-(5H) furan) 3-one que l'on recristallise dans l'éther isopropylique F = 176°C, 355 mg d'isomère 2α-

méthyle que l'on recristallise dans l'éther isopropylique F: 164°C et 65 mg de (17R) 2,2-diméthyl 11β-[4-(méthylthio) phényl] spiro (estra-4,9-diène-17,2'-(5H) furan) 3-one.

*Spectre IR ($CHCl_3$):*

| | |
|---|---|
| isomère 2βméthyl | C=0 1 656$^{cm^{-1}}$ C=C 1 605$^{cm^{-1}}$ et 865$^{cm^{-1}}$ |
| isomère 2αméthyl | C=O 1 654$^{cm^{-1}}$ C=C complexe max. 1 595$^{cm^{-1}}$ <br> aromatique 1492$^{cm^{-1}}$ épaulement 1 607$^{cm^{-1}}$ |
| produit 2,2-diméthyl | C=0 651$^{cm^{-1}}$ C=C 1 603$^{cm^{-1}}$ <br> aromatique 1 492$^{cm^{-1}}$ |

## Exemple 30

(E) (17R) oxime de 11β-[4-(méthythio) phényl] spiro (estra-4,9-diène-17 2'-(5H)-furan) 3-one et l'isomère (Z) correspondant.

On chauffe au reflux pendant 75 minutes sous atmosphère inerte 1,36 g de (17R) 11β-[4-(méthylthio) phényl] spiro (estra-4,9-diène-17,2'-(5H) furan) 3-one obtenu à l'exemple 3 dans 14 $cm^3$ d'éthanol avec 2,6 $cm^3$ de pyridine et 0,44 g de chlorhydrate d'hydroxylamine. On laisse revenir à température ambiante, verse dans 100 $cm^3$ d'eau, extrait à l'acétate d'éthyle, lave la phase organique, le sèche et concentre à sec sous pression réduite. On obtient 1,47 g de produit brut que l'on chromatographie sur silice en éluant par le mélange éther de pétrole (Eb 40°—70°C) acétate d'éthyle 8—2).

On récupère 805 mg d'isomère (E) qui est dissous à chaud dans le chlorure de méthylène et recristallisé dans l'éther isopropylique F = 250°C et 390 mg d'isomère (Z) qui est recristallisé de la même mainère F = 266°C.

Isomère (E):

*Spectre IR ($CHCl_3$):*

| | |
|---|---|
| OH (oxime) | 3 585$^{cm^{-1}}$ |
| système conjugué + aromatique | 1 613$^{cm^{-1}}$ (max) 1 592$^{cm^{-1}}$ (épaulement) <br> 1 544$^{cm^{-1}}$ et 1 491$^{cm^{-1}}$ |
| C—O—C | 1 081$^{cm^{-1}}$ et 1 040$^{cm^{-1}}$ |

*Analyse:* $C_{28}H_{33}NO_2S$ 447,64

| | C% | H% | N% | S% |
|---|---|---|---|---|
| Calculé: | 75,13 | 7,43 | 3,13 | 7,16 |
| Trouvé: | 75,0 | 7,5 | 3,1 | 7,1 |

Isomère (Z):

*Spectre IR ($CHCl_3$):*

| | |
|---|---|
| OH (oxime) | 3 589$^{cm^{-1}}$ (+ associés) |
| système conjugué + aromatique | 1 612$^{cm^{-1}}$ (max) 1 598$^{cm^{-1}}$ <br> 1 555$^{cm^{-1}}$ (f) et 1 492$^{cm^{-1}}$ (F) |
| C—O—C | 1 081$^{cm^{-1}}$ et 1 040$^{cm^{-1}}$ |

| | C% | H% | N% | S% |
|---|---|---|---|---|
| Trouvé: | 74,9 | 7,3 | 2,9 | 7,0 |

## Exemple 31

(17R) 4',5'-dihydro 9α,10α-époxy 11β-[4-[(3-méthylbutyl) sulfonyl] phényl] spiro (estr-4-ène-17,2'-(3H) furan) 3-one.

On dissout 0,8 g de produit obtenu à l'exemple 25 dans 20 $cm^3$ de chlorure de méthylène, refroidit à 0°C, ajoute par petites fractions 1,64 g d'acide métachloroperbenzoïque et agite 1 heure. On ajoute une solution de thiosulfate de sodium 0,2 N, agite 5 minutes, verse dans une solution aqueuse saturée en bicarbonate de sodium, extrait au chlorure de méthylène, lave à l'eau, sèche et élimine les solvants sous pression réduite. On obtient 0,980 g de produit attendu que l'on cristallise dans le mélange chlorure de méthylène-éther isopropylique F=203°C.

*Spectre IR (CHCl₃):*

| | |
|---|---|
| \ C=O / | 1 669$^{cm-1}$ (F) |
| —C=C, aromatique | 1 620$^{cm-1}$ 1 597$^{cm-1}$ 1 492$^{cm-1}$ |
| $SO_2$ | 1 317$^{cm-1}$ —1 144$^{cm-1}$ |

*Analyse:* $C_{32}H_{42}O_5S$ 538,75

| | C% | H% | S% |
|---|---|---|---|
| Calculé: | 71,34 | 7,85 | 5,95 |
| Trouvé: | 71,2 | 7,9 | 5,7 |

Exemple 32

(17R) 4-[3-oxo spiro (estra-4,9-diène-17,2'-(5H) furan) 11β-yl] benzoate de 2-amino 2-méthyl propyle.

On dissout 4,32 g de (17R) 11 -[4-(4,5-dihydro 4,4-diméthyl 2-oxazolyl) phényl] spiro (estra-4,9-dièn-17,2'-(5H) furan 3-one préparé comme à l'exemple 19 dans 40 cm³ de dioxanne et 40 cm³ d'acide chlorhydrique 2N, puis chauffe 3 heures et demise à 60°C. On refroidit, verse dans une solution aqueuse glacée de bicarbonate de sodium, extrait au chlorure de méthylène, lave à l'eau, sèche et élimine les solvants sous pression réduite.

On recueille 4,6 g de produit brut que l'on chromatographie sur silice (éluant acétate d'éthyle, puis acétate d'éthyle-triéthylamine 9—1). Après cristallisation dans l'éther, puis dans l'acétate d'éthyle, on obtient 2,54 g de produit attendu F=178°C.

*Spectre IR (CHCl₃):*

| | |
|---|---|
| ester conjugué | 1 715$^{cm-1}$ |
| cétone en 3 | 1 658$^{cm-1}$ |
| C=C, aromatique, $NH_2$ (déf.) | 1 608$^{cm-1}$ 1 569$^{cm-1}$ (épaulement) 1 504$^{cm-1}$ |

*Analyse:* $C_{32}H_{39}SO_4$ 501,67

| | C% | H% | S% |
|---|---|---|---|
| Calculé: | 76,61 | 7,83 | 2,79 |
| Trouvé: | 76,3 | 7,7 | 2,6 |

Exemple 33

(17R) 4-[3-oxo spiro (estra-4,9-dièn-17,2'-(5H) furan) 11β-yl] benzoate d'éthyle et (17R) 4-[3-oxo spiro (estra-4,9-dièn-17,2'-(5H)-furan) 11β-yl] N-2-hydroxy 1,1-diméthyléthyl) benzamide.

On agite 2 heures aink ribbon à température ambiante sous atmosphère inerte, 2,06 g de produit préparé à l'exemple 32 en suspension dans 50 cm³ d'éthanol et 8 cm³ d'une solution éthanolique d'éthylate de sodium (0,7M). On verse la suspension dans une solution aqueuse glacée d'acide chlorhydrique et extrait au chlorure de méthylène. On lave la phase organique, la sèche, élimine les solants sous pression réduite, chromatographie le résidu sur silice (éluant cyclohexane-acétate d'éthyle 7—3, puis acétate d'éthyle triéthylamine 9—1 et 6—4). On obtient d'une part 1,375 g du produit attendu sous forme de benzoate d'éthyle (produit A) que l'on recristallise dans l'éther puis dans l'éthanol, F = 140°C puis 182°C, d'autre part 0,385 g de produit attendu N-2-hydroxy (1,1-diméthyléthyl) benzamide (produit B) que l'on recristallise dans l'éther puis dans l'isopropanol F = 147°—157°C.

*Spectre IR du produit A (CHCl₃):*

| | |
|---|---|
| ester conjugué | 1 711$^{cm-1}$ |
| diénone | 1 658$^{cm-1}$ |
| C=C, aromatique | 1 608$^{cm-1}$ 1 570$^{cm-1}$ 1 503$^{cm-1}$ |

*Spectre IR du produit B ($CHCl_3$):*

| | |
|---|---|
| amide secondaire NH | 3 430$^{cm-1}$ |
| amide II | 1 526$^{cm-1}$ |
| amide+C=O diènone | 1 655$^{cm-1}$ |
| C=C diènone | 1 607$^{cm-1}$ |
| aromatique | 1 566$^{cm-1}$ −1 498$^{cm-1}$ |
| OH | ≃ 3 618$^{cm-1}$ |

Exemple 34

Acide (17R) 4-[spiro-(3-oxo estra-4,9-dièn-17,2'-(5H) furan) 11β-yl] benzoïque

On effectue un balayage d'azote pendat 20 minutes dans une suspension comprenant 0,3 g de produit A obtenu à l'exemple 33 dans 3 cm³ d'éthanol et ajoute 1 cm³ d'une solution aqueuse d'hydroxyde de sodium (N). On chauffe une heure et demie à 60°C, refroidit, et verse dans une solution diluée d'acide chlorhydrique. On extrait à l'acétate d'éthyle, lave à l'eau, sèche et élimine les solvants sous pression réduite et obtient 0,250 g de produit brut que l'on purifie par chromatographie sur silice (éluant acétate d'éthyle) F ≃ 170°C.

Exemple 35

(17R) 13β-éthyl 11β-[4-(méthylthio) phényl] spiro (gona 4,9-dièn 17,2'(5H) furan 3-one

*Stade A:* (1,2-éthane diyl) acétal cyclique de 5α,10α-époxy 13β-èthyl gona 9(11)-èn 3,17-dione.

On ajoute à température ambiante 21,3 cm³ d'hexafluoroacétone sesquihydrate dans une solution de 21,36 g de (1,2-éthanediyl) acétal cyclique de 13β-éthyl gon-5(10),9(11)-èn-17-one dans 213 cm³ de chlorure de méthylène, refroidit à 0°/+5°C et ajoute en 5 minutes 42,7 cm³ d'eau oxygénée et agite 2 heures 15 minutes sous atmosphère inerte. On ajoute du thiosulfate de sodium, extrait au chlorure de méthylène, lave, sèche et élimine les solvants sous pression réduite. On obtient 26,41 g de produit attendu.

*Spectre IR ($CHCl_3$):*

| | |
|---|---|
| C=O | 1 730$^{cm-1}$ |
| $CH_2$ en 16 | 1 406$^{cm-1}$ |
| C=C | 1 640$^{cm-1}$ |

*Stade B:* (Z) 3,3-(1,2-éthane diyl) acétal cyclique de 5α,10α-époxy 13β-éthyl 17β-hydroxy 17α-[3-(1-tétrahydro 2H-2-pyrannyloxy) 1-propynyl] gon-9(11)-èn-3,17-dione.

On opère comme au stade A de l'exemple 5 à partir de 19,9 g de produit obtenu précédemment et 14,6 cm³ du réactif HC = C—$CH_2$OTHP en laissant réagir pendant 15 heures à température ambiante. On obtient 33,533 g de produit brut que l'on chromatographie sur silice (éluant chlorure de méthylène-acétate d'éthyle 9—1) et obtient 15,498 g de produit attendu.

*Spectre IR ($CHCl_3$):*

| | |
|---|---|
| OH | 3 599$^{cm-1}$ |
| —C=C— | 1 640$^{cm-1}$ |

*Stade C:* (Z) (1,2-éthane diyl) acétal cyclique de 5α,10α-époxy 13β-éthyl 17β-hydroxy 17α-[3-(1-tétrahydro 2H-2-pyrannyloxy) 1-propényl] gon-9(11)-èn-3-one.

On hydrogène pendant 4 heures sous un pression de 1,2 bar 15,45 g de produit obtenu au stade C dans 320 cm³ d'acétate d'éthyle et 3,2 cm³ de pyridine en présence de 154 mg de sulfate de baryum à 10% de palladium. On filtre le catalyseur, lave à l'acétate d'éthyle et concentre à sec. On obtient 14,705 g de produit brut que l'on chromatographie sur silice (éluant chlorure de méthylène-acétate d'éthyle 9—1). On recueille 9,819 g de produit attendu.

*Spectre IR ($CHCl_3$):*

| | |
|---|---|
| OH | 3 600$^{cm-1}$(f) + associé 3 420$^{cm-1}$ |
| —C=C | 1 640$^{cm-1}$(f) |

*Stade D:* (Z) (1,2-éthane diyl) acétal cyclique de 5α,17β-dihydroxy 13β-éthyl 11β-[4-(méthylthio) phényl] 17α-[3-(tétrahydro 2H-2-pyrannyloxy) 1-propényl] gon-9(11)-èn-3-one.

On opère comme au stade B de l'exemple 3 à partir de 3,5 g de produit obtenu au stade précédent, 178 mg de chlorure cuivreux et 16,3 cm³ de solution de magnésien de parabromothioanisole titrant 1,1 M/l. Après chromatographie sur silice (éluant: cyclohexane-acétate d'éthyle 7—3), on obtient 3,46 g de produit attendu.

*Spectre IR ($CHCl_3$):*

| | |
|---|---|
| OH | ≃ 3 600$^{cm-1}$(f) + associé 3 500$^{cm-1}$(F) |
| aromatique | 1 592$^{cm-1}$(f)<br>1 556$^{cm-1}$<br>1 492$^{cm-1}$ |

*Stade E:* (Z) 13β-éthyl 17β-hydroxy 17α-(3-hydroxy 1-propényl) 11β-[4-(méthylthio) phéntl] gona-4,9-dièn-3-one.

On ajoute sous atmosphère inerte 17 $cm^3$ d'acide chlorhydrique 2N dans une suspension de 3,4 g de produit obtenu précédemment dans 68 $cm^3$ d'éthanol et agite 1 heure et demie à la température ambiante. On verse sur de la glace, ajoute 5 $cm^3$ d'ammoniaque concentrée, lave à l'eau, sèche et concentre à sec sous pression réduite. On obtient 2,961 g de produit brut que l'on chromatographie sur silice (éluant cyclohexane-acétate d'éthyle 5—5). Après cristallisation dans l'éther isopropylique, on obtient 1,816 g de produit attendu F = 186°C.

*Spectre IR ($CHCl_3$):*

| | |
|---|---|
| OH | 3 616$^{cm^{-1}}$ |
| C=O conjuguée | 1 652$^{cm^{-1}}$ (F) |
| —C=C— / aromatique | 1 597$^{cm^{-1}}$ 1 555$^{cm^{-1}}$ 1 492$^{cm^{-1}}$ (F) |

*Stade F:* (17R) 13β-éthyl 11β-[4-méthylthio) phényl] spiro (gona-4,9-dièn-17,2'-(5H) furan) 3-one.

On opère comme au stade E de l'exemple 3 à partir de 1,5 g du produit précédent, 30 $cm^3$ de pyridine, 3 g de chlorure de tosyle et 180 $cm^3$ d'acide chlorhydrique 2N. On obtient 1,981 g de produit brut que l'on chromatographie sur silice (éluant cyclohexane-acétate d'éthyle 7—3, puis 8—2).

*Spectre IR ($CHCl_3$):*

| | |
|---|---|
| C=O conjuguée | 1 653$^{cm^{-1}}$ |
| —C=C— / aromatique | 1 598$^{cm^{-1}}$ 1 556$^{cm^{-1}}$ 1 491$^{cm^{-1}}$ |

Exemple 36

(17R) 11β-[4-[2-diméthylamino) éthoxy] phényl] spiro (estra-1,3,5 (10)-trièn-17,2'-(5H) furan) 3-ol et son acétate

1). *Formation de l'acétate phénolique:*

On opère comme à l'exemple 28 en utilisant au départ 0,205 g du produit obtenu à l'exemple 20, 0,25 $cm^3$ d'anhydride acétique et 0,2 $cm^3$ de bromure d'acétyle.

Après purification par chromatographie, on obtient 0,150 g de l'acétate recherché.

2). *Saponification:*

On opère comme à l'exemple 28 en utilisant au départ 70 mg de l'acétate obtenu ci-dessus dans 1,5 $cm^3$ de méthanol avec 0,1 $cm^3$ de lessive de soude et obtient 60 mg de produit attendu.

*Spectre IR ($CHCl_3$):*

| | |
|---|---|
| OH | 3 600$^{cm^{-1}}$ |
| aromatique | 1 610$^{cm^{-1}}$ 1 581$^{cm^{-1}}$ 1 512$^{cm^{-1}}$ |
| 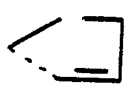 | 1 086$^{cm^{-1}}$ |

En utilisant un mode opératoire indentique à ceux décrits ci-dessus, on a préparé également le:

(17R) 4',5'-dihydro 11β[4-(méthyl(dimethylamino éthyl) amino] phényl] spiro (estra 4,9-dièn-17,2'-(3H)-furan) 3-one,

(17R) 4',5'-dihydro 11β[4-(méthyl(diméthylamino éthyl) amino] phényl] spiro (estra-1,3,5(10)-trièn-17,2'-(3H) furan) 3-ol,

(17S) 4',5'-dihydro 11β[4-(diméthylamino) phényl) 15α-méthyl spiro (gona-4,9-dìen-17,2'-(3H) furan) 3-one,

(17R) 4',5'-dihydro 11β[4-(diméthylamino) phényl] 13α-méthyl spiro (gona-4,9-dièn-17,2'-(3H) furan) 3-one.

*Composition pharmaceutique*:

On a préparé des comprimés répondant à la formule suivante:

| | |
|---|---|
| Produit de l'exemple 4 | 50 mg |
| Excipient (talc, amidon, stéarate de magnésium) q.s. pour un comprimé terminé a | 120 mg |

Etude Pharmacologique des Produits de l'Invention

I). Etude de l'activité des produits de l'invention sur les récepteurs hormonaux:

*Récepteur Progestogène de l'utérus de lapine:*

Des lapines impubères d'environ 1 kg reçoivent une application cutanée de 25 g d'estradiol. 5 jours après ce traitement, les animaux sont sacrifiés, les utérus sont prélevés, pesées et homogénéisés à 0°C, à l'aide d'un Potter teflon-verre dans une solution tamponée TS (Tris 10 mM, saccharose 0,25 M HCl pH 7,4) (1 g de tissu pour 50 ml de TS). L'homogénat est ensuite ultracentrifugé (105 000 g × 90 mn) à 0°C. Des aliquotes du surnageant ainsi obtenu, sont incubées a 0°C. Des aliquotes du surnageant ainsi obtenu, sont incubées à 0°C pendant un temps t, avec une concentration constante (T) de Produit R tritié (17,21-diméthyl 19-nor, 4,9-pregnadiène 3,20-dione) en présence de concentrations croissants (0—2 500. $10^{-9}$M) soit de R froid, soit de progestérone froide, soit du produit froid à tester. La concentration de R tritié lié (B) est ensuite mesurée dans chaque incubat par la technique d'adsorption au charbon dextran.

*Récepteur glucocorticoïde du thymus de rat:*

Des rats mâles Sprague-Dawley EOPS, pesant 160 à 200 g sont surrénalectomisés. 4 à 8 jours après cette ablation, les animaux sont sacrifiés, et les thymus sont prélevés et homogéisés à 0°C dans un tampon Tris 10 mM, saccharose 0,25M, dithiothreitol 2 mM, HCl pH 7, 4, à l'aide d'un Potter polytétrafluoroéthylène-verre (1 g de tissu pour 10 ml de TS). L'homogénat est ensuite ultracentrifugé (105 000 g × 90 mn) à 0°C. Des aliquotes du surnageant ainsi obtenu, sont incubées à 0°C. Des aliquotes du surnageant ainsi obtenu, sont incubées à 0°C pendant un temps (t) avec un concentration constante (T) de dexaméthasone tritiée en présence de concentrations croissantes (0—2 500.$10^{-9}$M) soit de dexaméthasone froide, soit du produit froid à tester. La concentration de la dexaméthasone tritiée liée (B) est ensuite mesurée dans chaque incubat par la technique d'adsorption au charbon-dextran.

*Calcul de l'affinité relative de liaison:*

Le calcul de l'affinité relative de liaison (ARL) est identique pour tous les récepteurs.

On trace les 2 courbes suivantes: le pourcentage de l'hormone tritiée liée

$$\frac{B}{T}$$

en fonction du logarithme de la concentration de l'hormone de référence froide et

$$\frac{B}{T}$$

en fonction du logarithme de la concentration du produit froid testé.

On détermine la droite d'équation

$$I^{50} = \left(\frac{B}{T}\max + \frac{B}{T}\min\right)/2$$

$$\frac{B}{T}\max =$$

Pourcentage de l'hormone tritiée liée pour une incubation de cette hormone tritiée à la concentration (T).

$$\frac{B}{T}\min =$$

Pourcentage de l'hormone tritée liée pour une incubation de cette hormone à la concentration (T) en présence d'un grand excès d'hormone froide (2500.$10^{-9}$M).

Les intersections de la droite $I_{50}$ et des courbes permettent d'évaluer les concentrations de l'hormone de référence froide (CH) et du produit froid testé (CX) qui inhibent de 50% la liaison de l'hormone tritiée sur le récepteur.

L'affinité relative de liasion (ARL) du produit testé est déterminée par l'équation:

$$ARL = 100\,\frac{(CH)}{(CX)}$$

Les résultats obtenus sont les suivants:

| Produits des exemples / Temps d'incubation à °C | Progestogène 2H | Progestogène 24H | Glucocorticoïde 4H | Glucocorticoïde 24H |
|---|---|---|---|---|
| 1 | 62 | 307 | 118 | 48 |
| 2 | 28 | 222 | 142 | 96 |
| 3 | 35 | 242 | 56 | 41 |
| 4 | 78 | 388 | 69 | 39 |

*Conclusion:*

Les produits étudiès, particulèrement le produit de l'exemple 4, présentent une affinité très marquée pur les récepteurs glucocorticoïde et progestogène.

Des résultats obtenus, on peut conclure que les produits peuvent présenter des activités agonistes ou antagonistes de glucocorticoïdes et des progestogènes.

II). Activités antiglucocorticoïde:

La technique utilisée découle de la méthode décrite par Dausse et Coll dans Molecular Pharmacology 13, 948—955 (1977) ("the relationship between glucocorticoïd structure and effects upon Thymoxytes"), pour des thymocytes de souris.

Des thymocytes de rats surrénalectomisés sont incubés à 37°C pendant 3 heures, dans un milieu nutritif renfermant $5.10^{-8}$M de dexaméthasone en présence ou non d'un produit à étudier à différentes concentrations. On ajoute l'uridine tritée, et poursuit l'incubation pendant une heure. On refroidit les incubats, les traite avec une solution d'acide trichloroacétate à 5%, les filtre sur papier Whatman GF/A, les lave trois fois à l'aide d'un solution d'acide trichloroacétique à 5%. On détermine la radioactivité retenue par le filtre.

Les glucocoticoïdes et en particulier la dexaméthasone provoquent une dimiminution de l'incorporation d'uridine tritiée. Les produits des exemples 1 à 4 s'opposent à cet effet.

| Produit de l'exemple | $5.10^{-8}$ Dexaméthasone + produit à tester à la concentration de: | % d'inhibition de l'effet de la Dexaméthasone |
|---|---|---|
| 1 | $10^{-8}$M | 11 |
| | $10^{-7}$M | 51 |
| | $10^{-6}$M | 125 |
| 2 | $10^{-8}$M | 0 |
| | $10^{-7}$M | 18 |
| | $10^{-6}$M | 82 |
| 3 | $10^{-8}$M | 0 |
| | $10^{-7}$M | 12 |
| | $10^{-6}$M | 84 |
| 4 | $10^{-8}$M | 0 |
| | $10^{-7}$M | 21 |
| | $10^{-6}$M | 80 |

Il a par ailleurs été constaté qu'utilisés seuls les produits testés ne provoquent aucun effet du type glucocorticoïde.

*Conclusion:*

Les produits étudiés présentent une activité anti-glucocorticoïde très marquée tout en étant dépourvous d'activité glucocorticoïde.

III). Activité abortive chez la rate

On détermine le jour $J_1$ de la gestation par la présence de spermatozoïdes dans le frottis vaginal. Au jour J9 de la gestation, on administre le produit en suspension dans la carboxyméthyl cellulose contenant 0,5% de tween.

Les animaux sont sacrifiés 72 h après le traitement et l'utérus est examiné pour déterminer l'état de la gestation.

On constate un avortement complet sur trous les animaux du groupe avec les produits des exemples 1 à 4 administrés à la dose de 3 mg/kg.

**Revendications**

1. Les produits de formule générale (I):

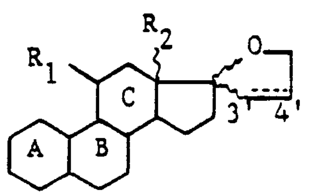

(I)

dans laquelle $R_1$ représente soit un radical phényle, benzyle ou un radical aryle hétérocyclique choisi parmi les radicaux thiényle, furyle, isothiényle, isofuryle, thiazolyle, isothiazolyle, oxazolyle, isoxazolyle, thiadiazolyle, pyridinyle ou pipéridinyle, chacun de ces radicaux étant éventuellement substitué par un ou plusieurs radicaux choisis parmi:

les radicaux alkyle ayant de 1 à 8 atomes de carbone;

les radicaux alkoxy ayant de 1 à 8 atomes de carbone;

les radicaux alkényloxy choisis parmi les radicaux vinyloxy ou allyloxy;

les atomes d'halogène;

les radicaux hydroxyle, trifluorométhyle, acyle ayant de 1 à 6 atomes de carbone, carboxy éventuellement estérifié;

les radicaux alkylthio ayant de 1 à 8 atomes de carbone éventuellement oxydé sous forme de sulfoxyde ou de sulfone;

les radicaux amino, amino mono ou disubstitués par des radicaux alkyles ayant de 1 à 8 atomes de carbone et éventuellement oxydés en N-oxyde et le radical bis (chloroéthyl) amino;

les radicaux diméthylamino méthyle, diméthylamino éthyle, méthyl (diméthylamino éthyl) amino, diméthylamino éthoxy, morpholino ou pipéridinyle;

soit $R_1$ représente les radicaux:

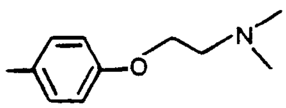
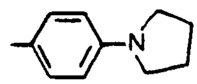
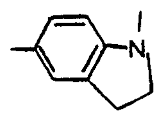
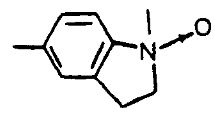

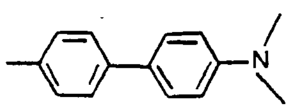
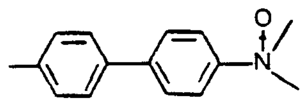
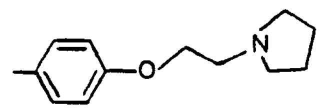

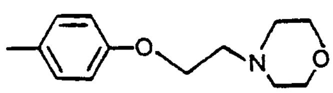
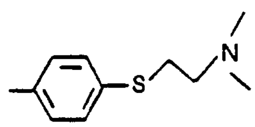
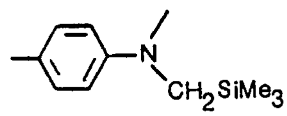

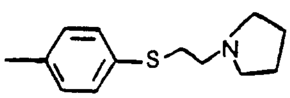
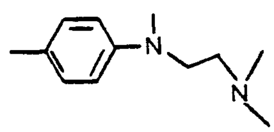
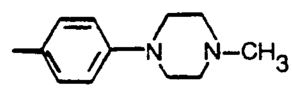

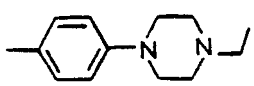
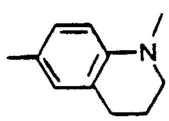
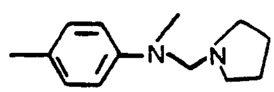
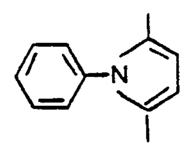

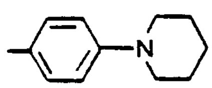
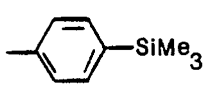
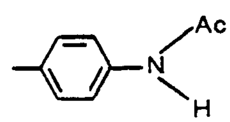

$R_2$ en position alpha ou béta, représente un radical hydrocarboné renfermant de 1 à 18 atomes de carbone le trait ondulé du spiro éther indique que l'atome d'oxygène peut se trouver en position alpha ou béta, le trait pointillé en position 3', 4' indique la présence éventuelle d'une seconde liaison entre les carbones qui le portent, les cycles A et B ayant l'une des structures suivantes:

a) — soit A et B représentent le groupement:

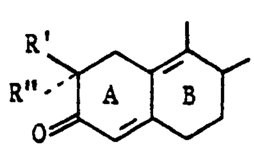

dans lequel R' et R'' identiques ou différents représentent un atome d'hydrogène, ou un radical alkyle ayant de 1 à 4 atomes de carbone;

b) — soit A et B représentent le groupement:

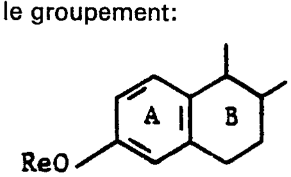

dans lequel Re représente un atome d-hydrogène, un radical alkyle ayant de 1 à 6 atomes de carbone éventuellement substitué ou un radical acyle;

c) — soit A, B et C représentent le groupement:

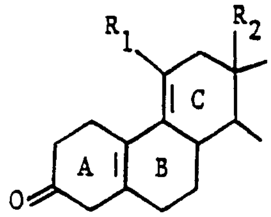

d) — soit A et B représentent le groupement:

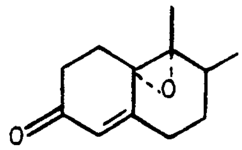

e) — soit A et B représentent le groupement:

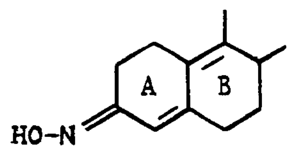

ainsi que leurs sels.

2. Les produits de formule (I) telle que définie à la revendication 1, répondant à la formule (I'):

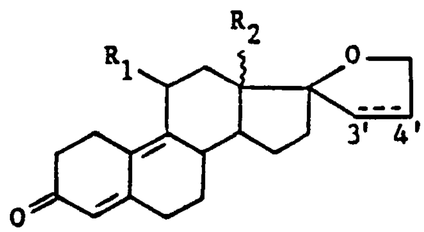

(I')

dans laquelle $R_1$ a la revendication indiquée à la revendication 1, $R_2$ en position alpha ou béta, représente un radical hydrocarboné renfermant de 1 à 18 atomes de carbone, l'atome d'oxygène du spiro éther est en position 17 béta, le trait pointillé en position 3', 4' indique la présence éventuelle d'une seconde liaison entre les carbones qui le portent ainsi que leurs sels.

3. Les produits de formule (I), telle que définie à la revendication 1, dans laquelle l'atome d'oxygène du spiroéther est en position 17 béta et les cycles A et B représentent le groupement:

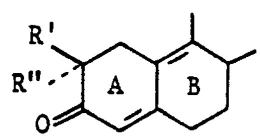

dans lequel R' et R'' représentent un atome d'hydrogène ainsi que leurs sels.

4. Les produits de formule (I), telle que définie à l'une quelconque des revendications 1 à 3, dans laquelle $R_1$ représente soit un radical aryle ou aralkyle portant une fonction amine

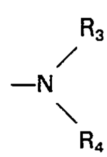

dans laquelle $R_3$ et $R_3$ représentent chacun un radical alkyle primaire, secondaire ou tertiaire renfermant de 1 à 8 atomes de carbone, soit un radical aryle portant une fonction méthylthio ou éthylthio, ainsi que leurs sels.

5. Les produits de formule (I) telle que définie à l'une quelconque des revendications 1 à 4, dans laquelle $R_1$ est un radical phényle, le substituant porté par ce radical phényle est en position para, ainsi que leurs sels.

6. Les produits de formule (I) telle que définie à l'une quelconque des revendications 1 à 5 dans laquelle $R_1$ représente l'un des radicaux suivants:

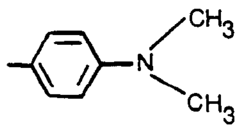 ; 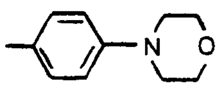 ; 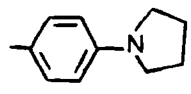 ; 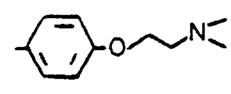

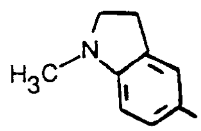 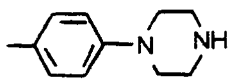 ; 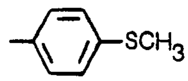 et 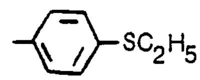

ainsi que leurs sels.

7. Les produits de formule (I) telle que définie à la revendication 6, dans laquelle $R_1$ représente un radical

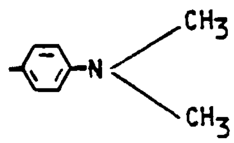

ou un radical

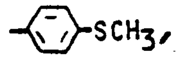

ainsi que leurs sels.

8. Les produits de formule (I) telle que définie à l'une quelconque des revendications 1 à 7 dans laquelle le substituant $R_2$ représente un radical méthyle en position alpha ou béta ou un radical éthyle en position béta, ainsi que leurs sels.

9. Les produits de formule (I) telle que défini à l'une quelconque des revendications 1 à 8, dont les noms suivent:

(17R)4',5'-dihydro 11 béta-[4-diméthylamino)phényl]spiro(estra-4,9-dièn-17,2'-(3H)furan) 3-one,

(17R) 11 béta-[4-(diméthylamino)phényl]spiro(estra-4,9-dièn-17,2'-(3H)furan) 3-one,

(17R) 11 béta-[4-(méthylamino)phényl]spiro(estra-4,9-dièn-17,2'-(3H)furan) 3-one,

(17R) 4',5'-dihydro 11 béta-[4-(méthylthio)phényl]spiro(estra-4,9-dièn-17,2'-(5H)furan) 3-one,

(17R) 11 béta-[4-(1-pyrrolidinyl)phényl]spiro(estra-4,9-dièn-17,2'-(5H)furan) 3-one,

(17R) 4',5'-dihydro 11 béta-[4-(1-pyrrolidinyl)phényl]spiro(estra-4,9-dièn-17,2'-(3H)furan) 3-one,

(17R) 4',5'-dihydro 11 béta-[(2,3-dihydro 1-méthyl) (1H)indol-5-yl spiro (estra-4,9-dièn-17,2'-(3H)furan) 3-one,

(17R) 11 béta-[4-(éthylthio)phényl]spiro(estra-4,9-dièn-17,2'-(5H)furan) 3-one, ainsi que leurs sels.

10. Procédé de préparation des produits de formule générale (I) telle que définie à la revendication 1 caractérisé en ce que:

a) pour préparer les produits de formule ($I_A$):

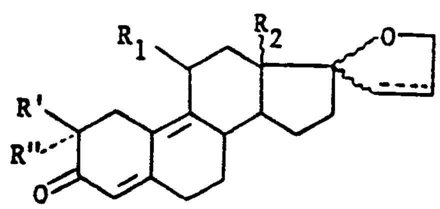

($I_A$)

dans laquelle $R_1$ et $R_2$ conservant la même signification que dans la revendication 1, R' et R'' représentent chacun un atome d'hydrogène ou un radical alkyle ou bien l'un représente un atome d'hydrogène et l'autre représente un radical alkyle, l'on soumet soit un produit de formule (II):

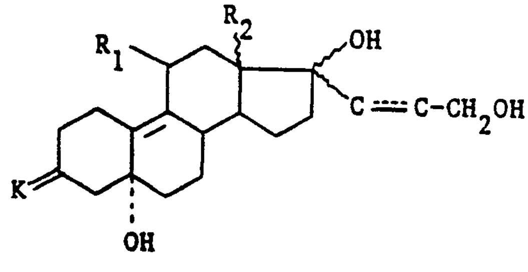

(II)

*soit* un produit de formule (III):

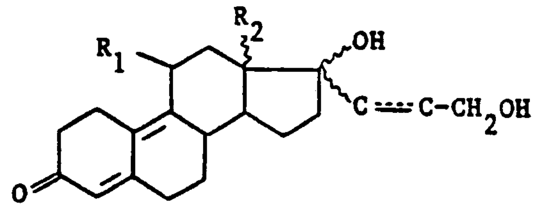

(III)

produits dans lesquels $R_1$ et $R_2$ ont la signification indiquée à la revendication 1 et K représente un groupement protecteur du radical cétone, à l'action d'un réactif de cyclisation pour obtenir respectivement, *soit* les produits de formule (IV):

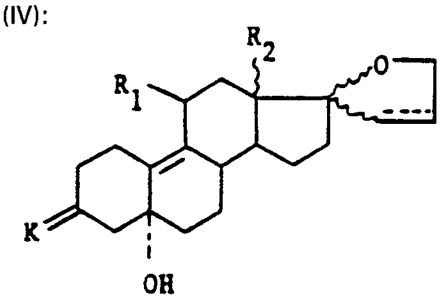

(IV)

produits de formule (IV) que l'on soumet à l'action d'un réactif de déshydratation également susceptible de libérer la fonction cétone pour obtenir les produits de formule ($I_A$), dans laquelle R' et R'' représentent un atome d'hydrogène,

*soit* lesdits produits de formule ($I_A$) et que, si désiré, l'on soumet à une oxydation les produits de formule ($I_A$) dans laquelle $R_1$ comporte un atome de soufre ou d'azote pour obtenir les produits dans lesquels $R_1$ comporte un atome de soufre oxydé en sulfoxyde ou en sulfone ou un atome d'azote oxydé en N-oxyde et que, si désiré l'on soumet les produits de formule ($I_A$) à une salification,

ou si désiré soumet les produits de formule ($I_A$) à l'action d'une base forte puis d'un halogénure d'alkyle pour obtenir un produit de formule ($I_A$) dans laquelle R' et/ou R'' représentent un radical alkyle ayant de 1 à 4 atomes de carbone;

b) pour préparer les produits de formule ($I_B$):

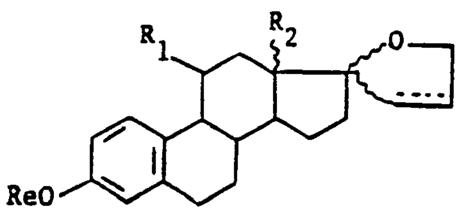

($I_B$)

dans laquelle $R_1$, $R_2$ et Re conservent la même signification qu'à la revendication 1, on soumet un produit de formule ($I'_A$):

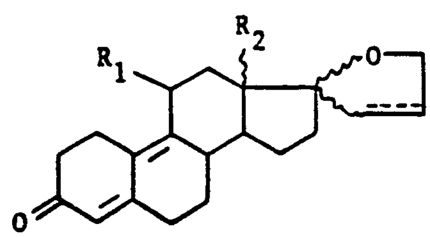

($I'_A$)

dans laquelle $R_1$ et $R_2$ ont la signification déjà indiquée, à l'action d'un agent d'aromatisation puis le cas échéant à l'action d'un agent de saponification et enfin si désiré soumet le produit de formule ($I_B$) dans laquelle Re est un atome d'hydrogène à un réactif d'alkylation;

c) pour préparer les produits de formule ($I_C$):

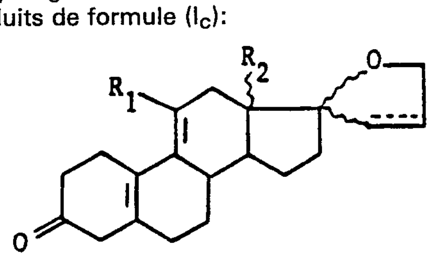

($I_C$)

dans laquelle $R_1$ et $R_2$ conservent la même signification qu'à la revendication 1, on soumet *soit* un produit de formule (V):

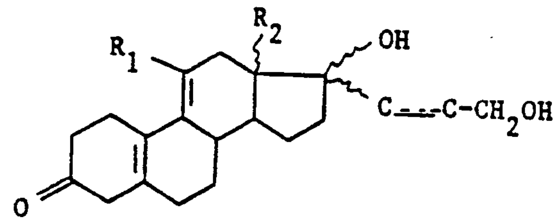

(V)

à l'action d'un agent de cyclisation, *soit* un produit de formule ($I'_A$) à l'action d'un agent d'acylation puis de saponification;

d) pour préparer les produits de formule ($I_D$):

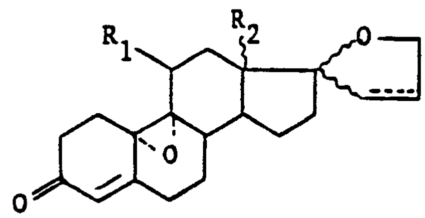

($I_D$)

dans laquelle $R_1$ et $R_2$ conservent la même signification qu'à la revendication 1, on soumet un produit de formule ($I'_A$) à l'action d'un agent d'époxydation;

e) pour préparer les produits de formule ($I_E$):

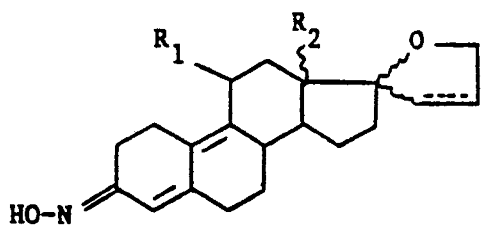

($I_E$)

dans laquelle $R_1$ et $R_2$ conservent la même signification qu'à la revendication 1, on fait agir l'hydroxylamine sur un produit de formule ($I'_A$).

11. Procédé selon la revendication 10 de préparation des produits de formule (I) dans laquelle $R_1$ représente un radical aryle ou aralkyle substitué par un radical carboxy éventuellement estérifié ou salifié, caractérisé en ce que l'on soumet un produit de formule ($I_F$):

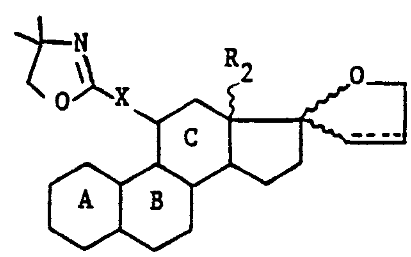

($I_F$)

dans laquelle R, B, C et $R_2$ ont la signification déjà indiquée à la revendication 1 et X représente un radical aryle ou aralkyle carbocyclique ou hétérocyclique à une hydrolyse acide pour obtenir un produit de formule ($I_G$):

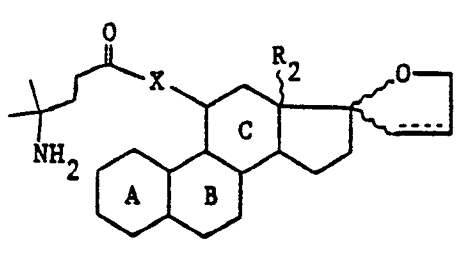

($I_G$)

que le cas échéant, l'on soumet à l'action d'un agent basique pour obtenir un produit de formule ($I_H$):

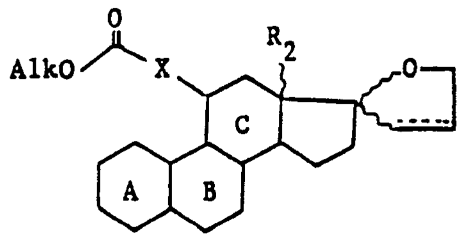

($I_H$)

dans laquelle alk représente un radical alkyle ayant de 1 à 6 atomes de carbone, que le cas échéant, l'on saponifie pour obtenir un produit de formule ($I_J$):

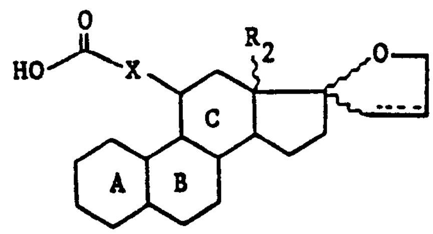

($I_J$)

que le cas échéant, l'on estérifie ou salifie.

12. Procédé selon la revendication 10 de préparation des produits de formule (I) caractérisé en ce que: le réactif de cyclisation que l'on fait agir sur les produits de formule (II), (III) ou (V) est le chlorure de tosyle en présence de pyridine.

13. A titre de médicaments, les produits de formule générale (I) telle que définie à la revendication 1, ainsi que leurs sels pharmaceutiquement acceptables.

14. A titre de médicaments, les produits de formule générale (I) telle que définie à la revendication 2, ainsi que leurs sels pharmaceutiquement acceptables.

15. A titre de médicaments, les produits de formule générale (I) telle que définie à l'une quelconque des revendication 3 à 8, ainsi que leurs sels pharmaceutiquement acceptables.

16. A titre de médicaments, les produits de formule générale (I) telle que définie à la revendication 9, ainsi que leurs sels pharmaceutiquement acceptables.

17. Compositions pharmaceutiques renfermant comme principe actif, un au moins des médicaments selon l'une quelconque des revendications 13 à 16.

18. A titre de produits industriels nouveaux, les produits de formule générale (IV):

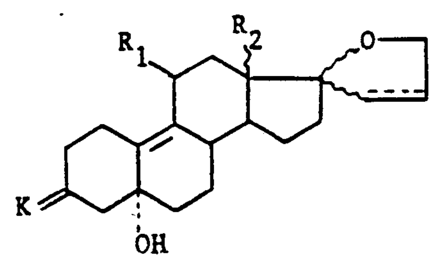

(IV)

dans laquelle $R_1$ et $R_2$ conservant la même signification que dans la revendication 1 et R a la même signification que dans la revendication 10.

**Patentansprüche**

1. Produkte der allgemeinen Formel (I)

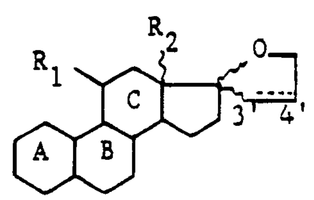

(I)

worin $R_1$ entweder einen Phenylrest, einen Benzylrest oder einen heterocyclischen Arylrest, ausgewählt unter den Thienyl-, Furyl-, Isothienyl-, Isofuryl-, Thiazolyl-, Isothiazolyl-, Oxazolyl-, Isoxazolyl-, Thiadiazolyl-, Pyridinyl- oder Piperidinylresten, bedeutet, wobei ein jeder dieser Reste gegebenenfalls durch einen oder mehrere Reste, ausgewählt unter

den Alkylresten mit 1 bis 8 Kohlenstoffatomen,
den Alkoxyresten mit 1 bis 8 Kohlenstoffatomen,
den Alkenyloxyresten, ausgewählt unter den Vinyloxy- oder Allyloxyresten,
den Halogenatomen,
den Hydroxyl-, Trifluormethyl-, Acyl- mit 1 bis 6 Kohlenstoffatomen, gegebenenfalls veresterten Carboxyresten,
den Alkylthioresten mit 1 bis 8 Kohlenstoffatomen, die gegebenenfalls in Form des Sulfoxids oder Sulfons oxidiert sind,
den Aminoresten, den Aminoresten, die durch Alkylreste mit 1 bis 8 Kohlenstoffatomen mono- oder disubstituiert und gegebenenfalls zum N-Oxid oxidiert sind, und dem Bis-(chloroethyl)-aminorest,
den Dimethylaminomethyl-, Dimethylaminoethyl-, Methyl-(dimethylaminoethyl)-amino-, Dimethylaminoethoxy-, Morpholino- oder Piperidinylresten, substituiert ist; oder $R_1$ bedeutet die Reste

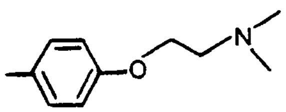 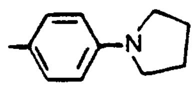 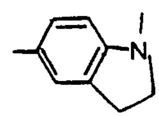 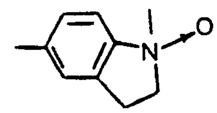

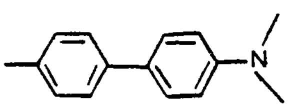 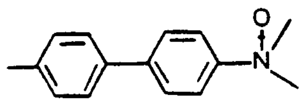 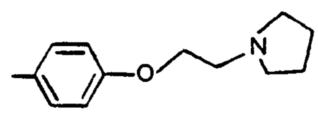

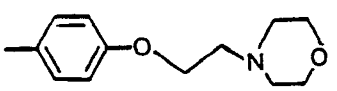 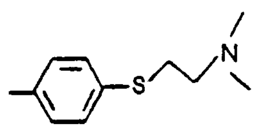 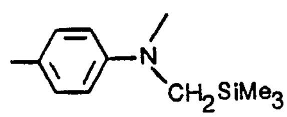

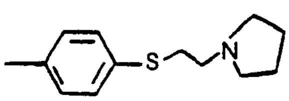 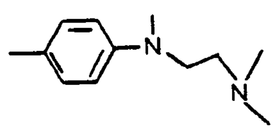 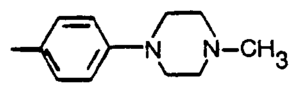

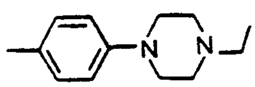 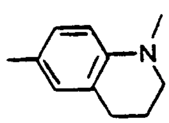 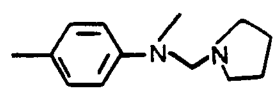 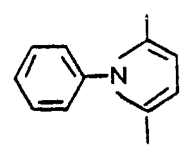

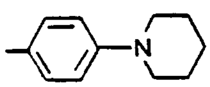 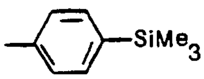 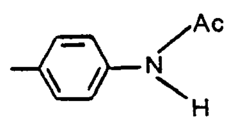

$R_2$ in α- oder β-Stellung für eine Kohlenwasserstoffgruppe mit 1 bis 18 Kohlenstoffatomen steht, die gewellte Linie des Spiroethers anzeigt, daß sich das Sauerstoffatom in der α- oder β-Stellung befinden kann, die gestrichelte Linie in 3',4'-Stellung die etwaige Anwesenheit einer zweiten Bindung zwischen den Kohlenstoffatomen, die sie aufweisen, anzeigt, wobei die Ringe A und B eine der folgenden Strukturen besitzen können:

(a) entweder geben A und B die Gruppe

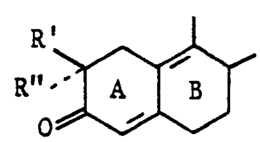

wieder, worin R' und R'', gleich oder voneinander verschieden, für ein Wasserstoffatom oder einen Alkylrest mit 1 bis 4 Kohlenstoffatomen stehen;

(b) oder A und B die Gruppe

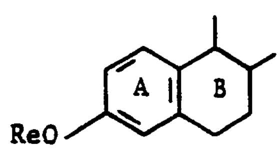

wiedergeben, worin Re für ein Wasserstoffatom, eine gegebenenfalls substituierte Alkylgruppe mit 1 bis 6 Kohlenstoffatomen oder eine Acylgruppe steht;

(c) oder A, B und C die Gruppe

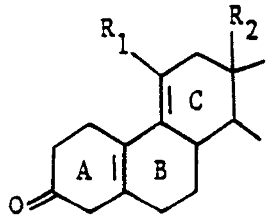

wiedergeben;

(d) oder A und B die Gruppe

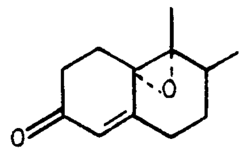

wiedergeben;

(e) oder A und B die Gruppe

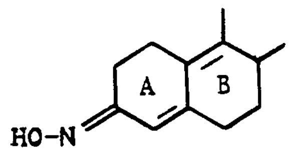

wiedergeben,
sowie deren Salze.

2. Produkte der Formel (I) gemäß Anspruch 1 der Formel (I')

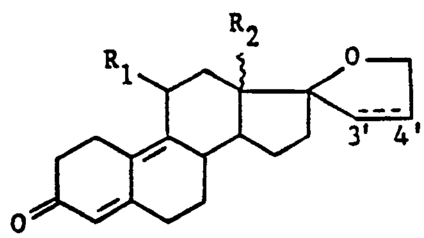

(I')

worin $R_1$ die in Anspruch 1 angegebene Bedeutung besitzt, $R_2$ in α- oder β-Stellung für eine Kohlenwasserstoffgruppe mit 1 bis 18 Kohlenstoffatomen steht, das Sauerstoffatom des Spiroethers sich in der 17β-Stellung befindet, die gestrichelte Linie in 3',4'-Stellung die etwaige Anwesenheit einer zweiten Bindung zwischen den Kohlenstoffatomen, die sie aufweisen, anzeigt, sowie deren Salze.

3. Produkte der Formel (I) gemäß Anspruch 1, worin das Sauerstoffatom des Spiroethers sich in der 17β-Stellung befindet und die Ringe A und B die Gruppe

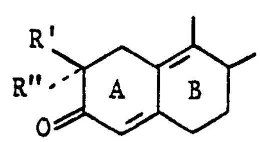

wiedergeben, worin R' und R'' für ein Wasserstoffatom stehen, sowie deren Salze.

4. Produkte der Formel (I) gemäß einem der Ansprüche 1 bis 3, worin $R_1$ entweder einen Aryl- oder Aralkylrest mit einer Aminfunktion

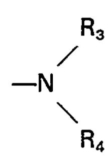

bedeutet, worin $R_3$ und $R_4$ jeweils einen primären, sekundären oder tertiären Alkylrest mit 1 bis 8 Kohlenstoffatomen bedeuten, oder einen Arylrest mit einer Methylthio- oder Ethylthiofunktion wiedergeben, sowie deren Salze.

5. Produkte der Formel (I) gemäß einem der Ansprüche 1 bis 4, worin $R_1$ für eine Phenylgruppe steht und der durch diese Phenylgruppe getragene Substituent sich in para-Stellung befindet, sowie deren Salze.

6. Produkte der Formel (I) gemäß einem der Ansprüche 1 bis 5, worin $R_1$ einen der folgenden Reste

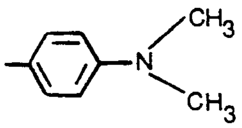 ; 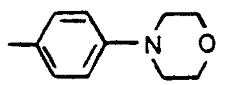 ; 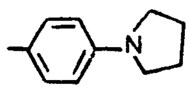 ; 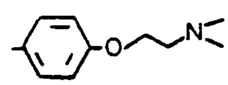

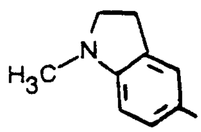 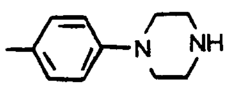 ; 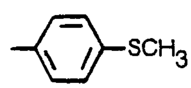 und 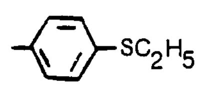

wiedergibt, sowie deren Salze.

7. Produkte der Formel (I) gemäß Anspruch 6, worin $R_1$ einen Rest

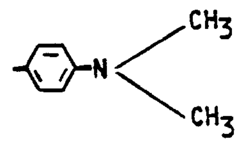

oder einen Rest

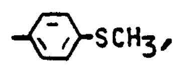 ,

wiedergibt, sowie deren Salze.

8. Produkte der Formel (I) gemäß einem der Ansprüche 1 bis 7, worin der Substituent $R_2$ für eine Methylgruppe in α- oder β-Stellung oder eine Ethylgruppe in β-Stellung steht, sowie deren Salze.

9. Produkte der Formel (I) gemäß einem der Ansprüche 1 bis 8 mit den folgenden Bezeichnungen:
(17R)-4',5'-Dihydro-11β-[4-(dimethylamino)-phenyl]-spiro-(östra-4,9-dien-17,2'-(3H)-furan)-3-on,
(17R)-11β-[4-(Dimethylamino)-phenyl]-spiro-(östra-4,9-dien-17,2'-(5H)-furan)-3-on,
(17R)-11β-[4-(Methylthio)-phenyl]-spiro-(östra-4,9-dien-17,2'-(5H)-furan)-3-on,
(17R)-4',5'-Dihydro-11β-[4-(methylthio)-phenyl]-spiro-(östra-4,9-dien-17,2'-(5H)-furan)-3-on,
(17R)-11β-[4-(1-Pyrrolidinyl)-phenyl]-spiro-(östra-4,9-dien-17,2'-(5H)-furan)-3-on,
(17R)-4',5'-Dihydro-11β-[4-(1-pyrrolidinyl)-phenyl]-spiro-(östra-4,9-dien-17,2'-(3H)-furan)-3-on,
(17R)-4',5'-Dihydro-11β-[(2,3-dihydro-1-methyl)-(1H)-indol-5-yl-spiro-(östra-4,9-dien-17,2'-(3H)-furan)-3-on,
(17R)-11β-[4-Ethylthio)-phenyl]-spiro-(östra-4,9-dien-17,2'-(5H)-furan)-3-on sowie deren Salze.

10. Verfahren zur Herstellung der Produkte der allgemeinen Formel (I) gemäß Anspruch 1, dadurch gekennzeichnet, daß man

(a) zur Herstellung der Produkte der Formel ($I_A$)

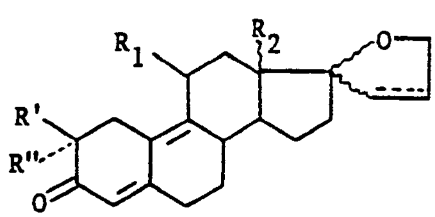

($I_A$)

worin $R_1$ und $R_2$ die in Anspruch 1 angegebene Bedeutung besitzen, R' und R'' jeweils für ein Wasserstoffatom oder eine Alkylgruppe stehen oder einer dieser Reste ein Wasserstoffatom bedeutet und der andere eine Alkylgruppe wiedergibt, entweder ein Produkt der Formel (II)

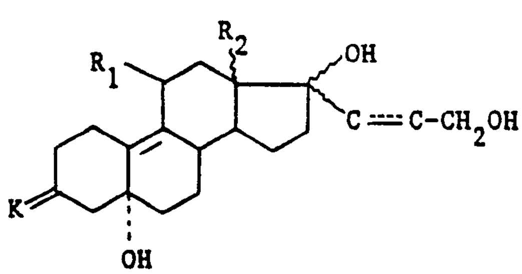

(II)

oder ein Produkt der Formel (III)

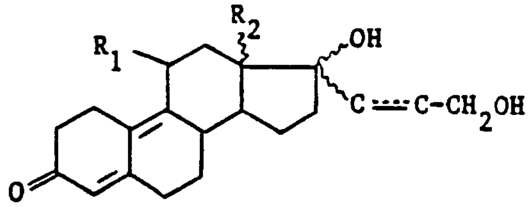

(III)

in welchen Produkten $R_1$ und $R_2$ die in Anspruch 1 angegebene Bedeutung besitzen und K für eine Schutzgruppe des Ketonrestes steht, der Einwirkung eines Cyclisierungsreagens unterzieht, um *entweder* zu den Produkten der Formel (IV)

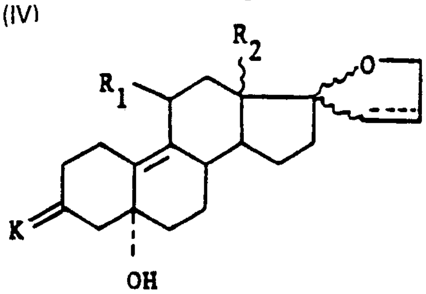

(IV)

zu gelangen, welche Produkte der Formel (IV) man der Einwirkung eines Dehydrationsreagens unterzieht, das auch in der Lage ist, die Ketonfunktion freizusetzen, um zu den Produkten der Formel ($I_A$) zu gelangen, worin R′ und R″ für ein Wasserstoffatom stehen, *bzw.* um zu den Produkten der Formel ($I_A$) zu gelangen, und gewünschtenfalls die Produkte der Formel ($I_A$), hworin R′ ein Schwefel- oder Stickstoffatom enthält, einer Oxidation unterzieht, um zu den Produkten zu gelangen, worin $R_1$ ein zum Sulfoxid oder zum Sulfon oxidiertes Schwefelatom oder ein zum N-Oxid oxidiertes Stickstoffatom enthält, und gewünschtenfalls die Produkte der Formel ($I_A$) einer Salzbildung unterzieht, oder gewünschtenfalls die Produkte der Formel ($I_A$) der Einwirkung einer starken Base, danach derjenigen eines Alkylhalogenids unterzieht, um zu einem Produkt der Formel ($I_A$) zu gelangen, worin R′ und/oder R″ für eine Alkylgruppe mit 1 bis 4 Kohlenstoffatomen stehen;

(b) zur Herstellung der Produkte der Formel ($I_B$)

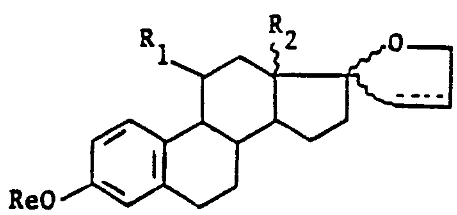

($I_B$)

worin $R_1$, $R_2$ und Re die in Anspruch 1 angegebene Bedeutung besitzen, ein Produkt der Formel ($I'_A$)

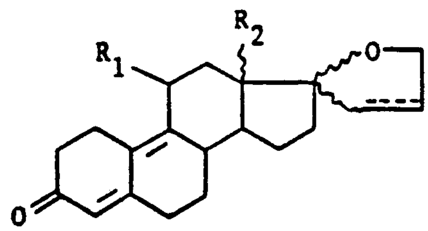

($I'_A$)

worin $R_1$ und $R_2$ die angegebene Bedeutung besitzen, der Einwirkung eines Aromatisierungsmittels, danach gegebenenfalls der Einwirkung eines Verseifungsmittels unterzieht und schließlich gewünschtenfalls das Produkt der Formel ($I_B$), worin Re ein Wasserstoffatom ist, einem Alkylierungsreagens unterzieht;

(c) zur Herstellung der Produkte der Formel ($I_C$)

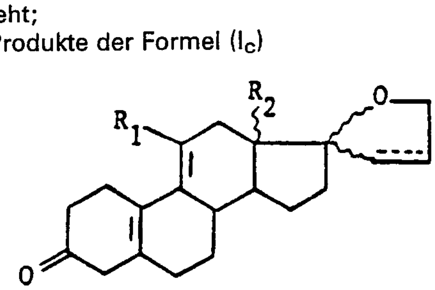

($I_C$)

worin $R_1$ und $R_2$ die in Anspruch 1 angegebene Bedeutung besitzen, *entweder* ein Produkt der Formel (V)

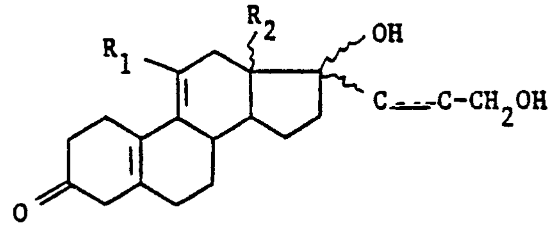

(V)

der Einwirkung eines Cyclisierungsmittels unterzieht, *oder* ein Produkt der Formel ($I'_A$) der Einwirkung eines Acylierungsmittels und danach derjenigen eines Verseifungsmittels unterzieht;

(d) zur Herstellung der Produkte der Formel ($I_D$)

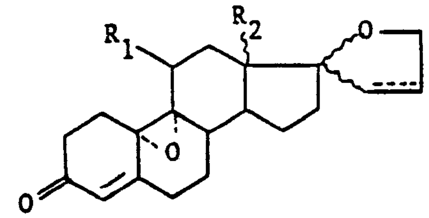

($I_D$)

worin $R_1$ und $R_2$ die in Anspruch 1 angegebene Bedeutung besitzen, ein Produkt der Formel ($I'_A$) der Einwirkung eines Epoxidierungsmittels unterzieht;

(e) zur Herstellung der Produkte der Formel ($I_E$)

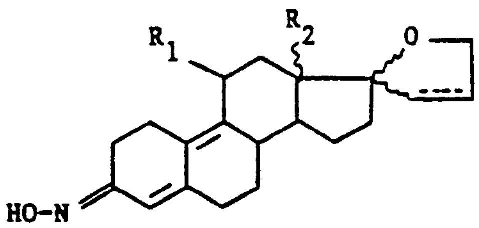

($I_E$)

worin $R_1$ und $R_2$ die in Anspruch 1 angegebene Bedeutung besitzen, Hydroxylamin mit einem Produkt der Formel ($I'_A$) umsetzt.

11. Verfahren gemäß Anspruch 10 zur Herstellung der Produkte der Formel (I), worin $R_1$ für einen Aryl- oder Aralkylrest, der durch eine gegebenenfalls veresterte oder in ein Salz überführte Carboxygruppe substituiert ist, steht, dadurch gekennzeichnet, daß man ein Produkt der Formel ($I_F$)

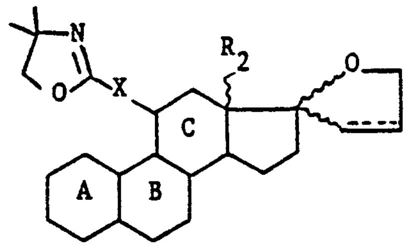

($I_F$)

worin A, B, C und $R_2$ die in Anspruch 1 angegebenen Bedeutung besitzen und X für eine carboxcyclische oder heterocyclische Aryl- oder Aralkylgruppe steht, einer sauren Hydrolyse unterzieht, um zu einem Produkt der Formel ($I_G$)

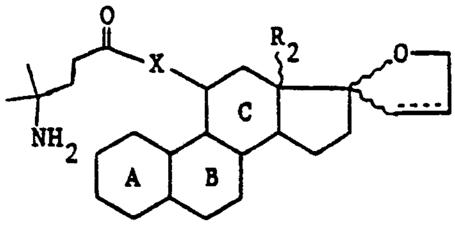

($I_G$)

zu gelangen, welches man gegebenenfalls der Einwirkung eines basischen Mittels unterzieht, um zu einem Produkt der Formel ($I_H$)

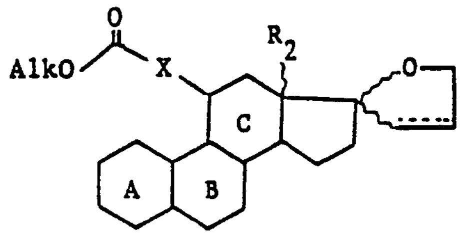

$(I_H)$

zu gelangen, worin alk für einen Alkylrest mit 1 bis 6 Kohlenstoffatomen steht, welches man gegebenenfalls verseift, um zu einem Produkt der Formel $(I_J)$

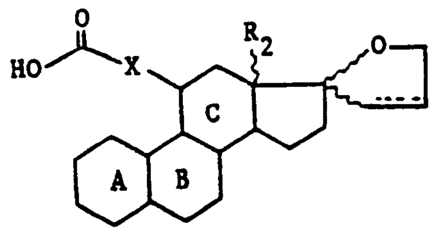

$(I_J)$

zu gelangen, welches man gegebenenfalls verestert oder in ein Salz überführt.

12. Verfahren gemäß Anspruch 10 zur Herstellung von Produkten der Formel (I), dadurch gekennzeichnet, daß das Cyclisierungsreagens, welches man mit den Produkten der Formel (II), (III) oder (V) umsetzt, Tosylchlorid in Gegenwart von Pyridin ist.

13. Als Arzneimittel die Produkte der allgemeinen Formel (I) gemäß Anspruch 1 sowie deren pharmazeutisch verträgliche Salze.

14. Als Arzneimittel die Produkte der allgemeinen Formel (I) gemäß Anspruch 2 sowie deren pharmazeutisch verträgliche Salze.

15. Als Arzneimittel die Produkte der allgemeinen Formel (I) gemäß einem der Ansprüche 3 bis 8 sowie deren pharmazeutisch verträgliche Salze.

16. Als Arzneimittel die Produkte der allgemeinen Formel (I) gemäß Anspruch 9 sowie deren pharmazeutisch verträgliche Salze.

17. Pharmazeutische Zusammensetzungen, enthaltend als Wirkstoff zumindest eines der Arzneimittel gemäß einem der Ansprüche 13 bis 16.

18. Als neue, industrielle Produkte die Produkte der allgemeinen Formel (IV)

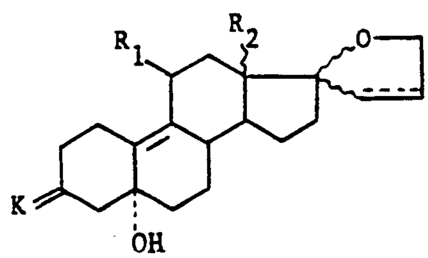

(IV)

worin $R_1$ und $R_2$ die in Anspruch 1 angegebene Bedeutung besitzen und K eine der in Anspruch 10 angegebenen Bedeutungen besitzt.

## Claims

1. The products of general formula (I):

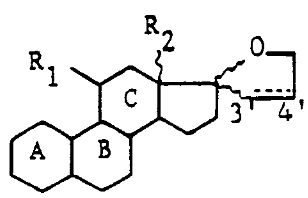

(I)

in which $R_1$ represents *either* a phenyl radical, benzyl radical or a heterocyclic aryl radical chosen from the radicals thienyl, furyl, isothienyl, isofuryl, thiazolyl, isothiazolyl, oxazolyl, isoxazolyl, thiadiazolyl, pyridinyl or piperidinyl, each of these radicals being optionally substituted by one or more radicals chose from:

alkyl radicals having 1 to 8 carbon atoms;

alkoxy radicals having 1 to 8 carbon atoms;

alkenyloxy radicals chosen from the vinyloxy or allyloxy radicals;

halogen atoms;

hydroxyl, trifluoromethyl, acyl radicals having 1 to 6 carbon atoms, optionally esterified carboxy radicals;

alkylthio radicals having 1 to 8 carbon atoms optionally oxidized in the form of the sulphoxide or sulphone;

amino radicals, amino radicals mono or disubstituted by alkyl radicals having 1 to 8 carbon atoms and optionally oxidized to N-oxide and the bis (chloroethyl) amino radical.

dimethylamino methyl, dimethylamino ethyl, methyl (dimethylamino ethyl) amino, dimethylamino ethoxy, morpholino or piperidinyl radicals; *or* $R_1$ represents,

the radicals:

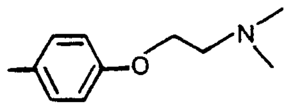
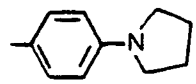
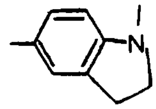
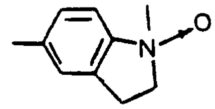

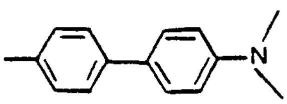
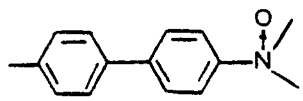
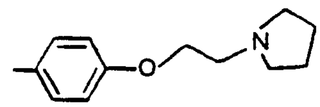

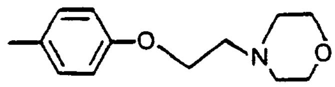
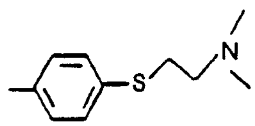
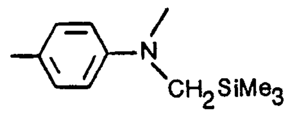

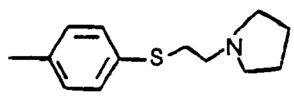
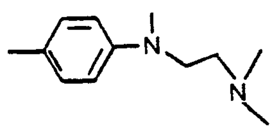
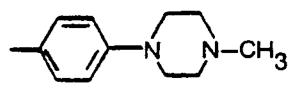

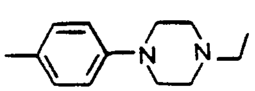
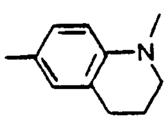
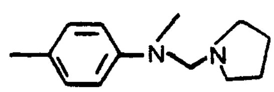
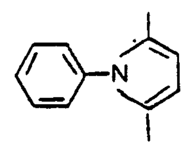

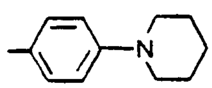
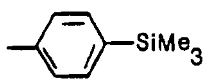
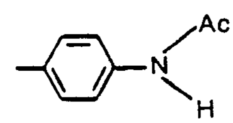

$R_2$ in alpha or beta position, represents a hydrocarbonated radical containing 1 to 18 carbon atoms, the wavy line of the spiro ether indicates that the oxygen atom can be found in alpha or beta position, the dotted line in position 3', 4' indicates the possible presence of a second bond between the carbons carrying it, the A and B rings having one of the following structures:

a) either A and B represent the group:

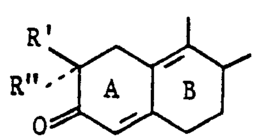

in which R' and R'' identical or different, represent a hydrogen atom, or an alkyl radical having 1 to 4 carbon atoms;

b) or A and B represent the group:

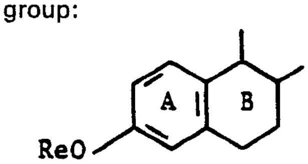

in which Re represents a hydrogen atom, an optionally substituted alkyl radical having 1 to 6 carbon atoms or an acyl radical;

c) or A, B, and C represent the group:

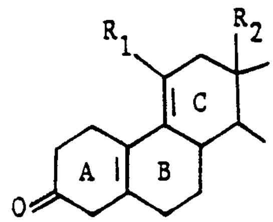

d) or A and B represent the group:

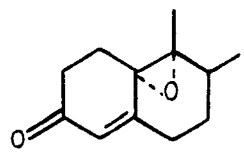

e) or A and B represent the group:

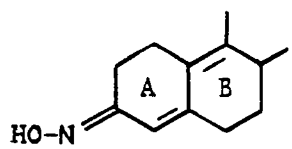

as well as their salts.

2. The products of formula (I) as defined in claim 1, corresponding to the formula (I'):

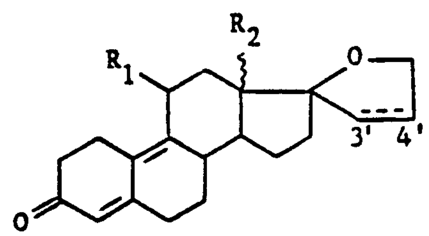

(I')

in which $R_1$ has the meaning indicated in claim 1, $R_2$ in alpha or beta position, represents a hydrocarbonated radical containing 1 to 18 carbon atoms, the oxygen atom of the spiro ether is in position 17-beta, the dotted line in position 3′, 4′ indicates the possible presence of a second bond between the carbons carrying it, as well as their salts.

3. The products of formula (I), as defined in claim 1, in which the oxygen atom of the spiro ether is in position 17-beta and the A and B rings represent the group:

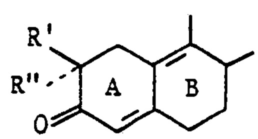

in which R′ and R″ represents a hydrogen atom, as well as their salts.

4. The products of formula (I), as defined in any one of claims 1 to 3, in which $R_1$ represents either an aryl radical or an aralkyl radical carrying a

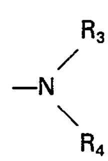

amine function in which $R_3$ and $R_4$ each represent a primary, secondary or tertiary alkyl radical containing 1 to 8 carbon atoms, or an aryl radical carrying a methylthio or ethylthio function, as well as their salts.

5. The products of formula (I) as defined in any one of claims 1 to 4, in which $R_1$ is a phenyl radical, the substituent carried by this phenyl radical is in para position, as well as their salts.

6. The products of formula (I) as defined in any one of claims 1 to 5 in which $R_1$ represents one of the following radicals:

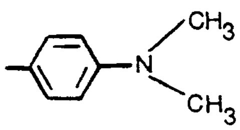 ; 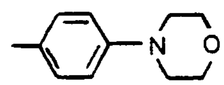 ; 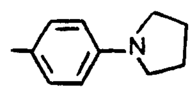 ; 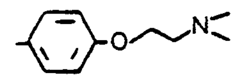

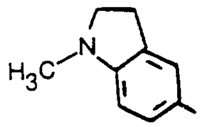 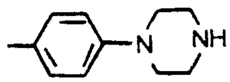 ; 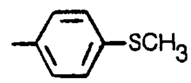 and 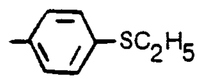

as well as their salts.

7. The products of formula (I) as defined in claim 6, in which $R_1$ represents a

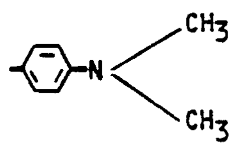

radical or a

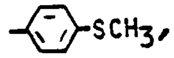

radical, as well as their salts.

8. The products of formula (I) as defined in any one of claims 1 to 7 in which the substituent $R_2$ represents a methyl radical in alpha or beta position or an ethyl radical in beta position, as well as their salts.

9. The products of formula (I) as defined in any one of claims 1 to 8, the names of which follow:

(17R)-4',5'-dihydro-11-beta-[4-(dimethylamino)-phenyl]-spiro-(estra-4,9-dien-17,2'-(3H)-furan)-3-one,
(17R)-11-beta-[4-(dimethylamino)-phenyl]-spiro-(estra-4,9-dien-17,2'-(5H)-furan)-3-one,
(17R)-11-beta-[4-(methylthio)-phenyl]-spiro-(estra-4,9-dien-17,2'-(5H)-furan)-3-one,
(17R)-4',5'-dihydro-11-beta-[4-(methylthio)-phenyl]-spiro-(estra-4,9-dien-17,2'-(5H)-furan)-3-one,
(17R)-11-beta-[4-(1-pyrrolidinyl)-phenyl]-spiro-(estra-4,9-dien-17,2'-(5H)-furan)-3-one,
(17R)-4',5'-dihydro-11-beta-[4-(1-pyrrolidinyl)-phenyl]-spiro-(estra-4,9-dien-17,2'-(5H)-furan)-3-one,
(17R)-4',5'-dihdyro-11-beta-[(2,3-dihydro-1-methyl)-(1H)-indol-5-yl-spiro-(estra-4,9-dien-17,2'-(3H)-furan)-3-one,
(17R)-11-beta-[4-(ethylthio)-phenyl]-spiro-(estra-4,9-dien-17,2'-(5H)-furan)-3-one, as well as their salts.

10. Preparation process of products of general formula (I) as defined in claim 1 characterized in that:

a) to prepare the products of formula ($I_A$):

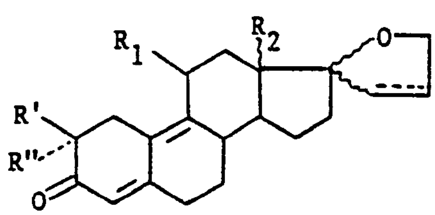

($I_A$)

in which $R_1$ and $R_2$ retain the same meaning as in claim 1, R' and R'' each represents a hydrogen atom or an alkyl radical or one represents a hydrogen atom and the other represents an alkyl radical, *either* a product of formula (II):

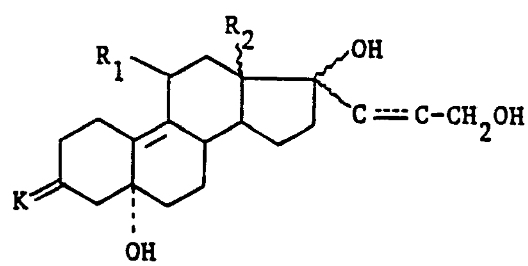

(II)

*or* a product of formula (III):

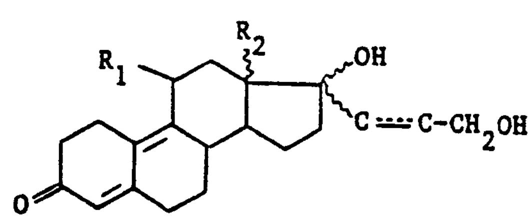

(III)

in which products $R_1$ and $R_2$ have the meaning indicated in claim 1 and K represents a protector group of the ketone radical, are subjected to the action of a cyclization reagent so as to obtain respectively, *either* the products of formula (IV):

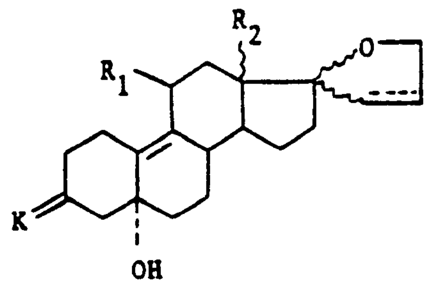

(IV)

which products of formula (IV) are subjected to the action of a dehydration reagent which is also able to free the ketone function so as to obtain the products of formula ($I_A$), in which R′ and R″ represent a hydrogen atom, *or* said products of formula ($I_A$) and that, if desired, the products of formula ($I_A$) in which $R_1$ consists of a sulphur or nitrogen atom are subjected to an oxidation so as to obtain the products in which $R_1$ consists of an sulphur atom oxidized to sulphoxide or sulphone or a nitrogen atom oxidized to N-oxide and that, if desired, the products of formula ($I_A$) are subjected to a salification, or, if desired, the products of formula ($I_A$) are subjected to the action of a strong base then of an alkyl halide so as to obtain a product of formula ($I_A$) in which R′ and/or R″ represent an alkyl radical having 1 to 4 carbon atoms;

b) to prepare the products of formula ($I_B$):

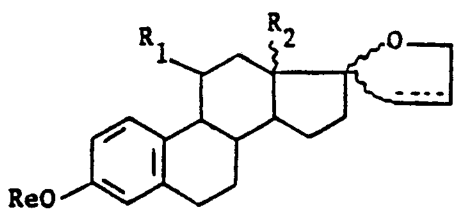

($I_B$)

in which $R_1$, $R_2$ and Re retain the same meaning as in claim 1, a product of formula ($I'_A$):

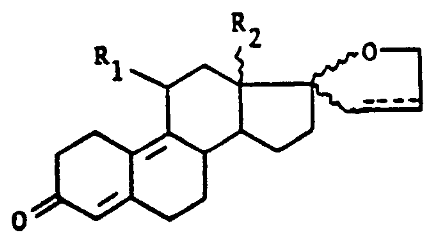

($I'_A$)

in which $R_1$ and $R_2$ have the meaning already indicated, is subjected to the action of an aromatization agent then, if appropriate, to the action of a saponification agent and finally, if desired, the product of formula ($I_B$) in which Re is a hydrogen atom is subjected to an alkylation reagent;

c) to prepare the products of formula ($I_C$):

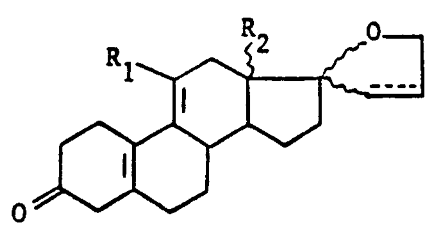

($I_C$)

in which $R_1$ and $R_2$ retain the same meaning as in claim 1, *either* a product of formula (V):

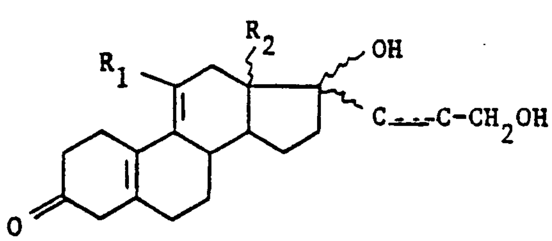

(V)

is subjected to the action of a cyclization agent, *or* a product of formula ($I'_A$) is subjected to the action of an acylation agent then to a saponification agent;

d) to prepare the products of formula ($I_D$):

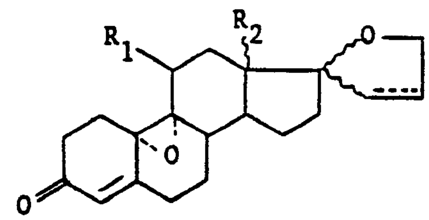

($I_D$)

in which $R_1$ and $R_2$ retain the same meaning as in claim 1, a product of formula ($I'_A$) is subjected to the action of an epoxidation agent;

e) to prepare the products of formula ($I_E$)

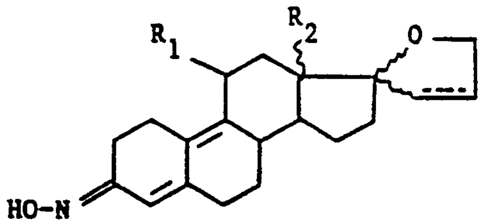

($I_E$)

in which $R_1$ and $R_2$ retain the same meaning as in claim 1, the hydroxylamine is reacted with a product of formula ($I'_A$).

11. Process according to claim 10 for the preparation of products of formula (I) in which $R_1$ represents an aryl or aralkyl radical substituted by a carboxy radical optionally esterified or salified, characterized in that a product of formula ($I_F$):

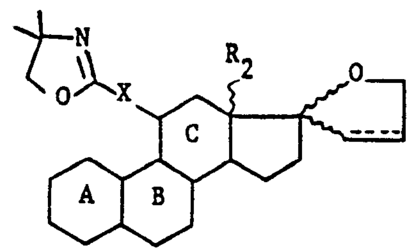

($I_F$)

in which A, B, C and $R_2$ have the meaning already indicated in claim 1 and X represents a carbocyclic or heterocyclic aryl or aralkyl radical, is subjected to an acid hydrolysis so as to obtain a product of formula ($I_G$):

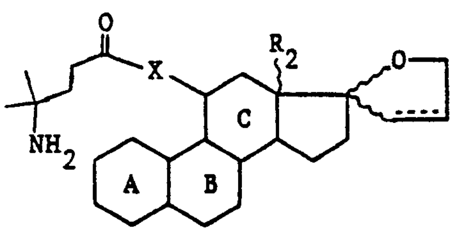

($I_G$)

which if appropriate, is subjected to the action of a basic agent so as to obtain a product of formula ($I_H$):

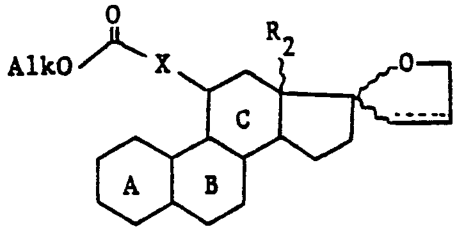

($I_H$)

in which alk represents an alkyl radical having 1 to 6 carbon atoms, which if appropriate, is saponified so as to obtain a product of formula ($I_J$):

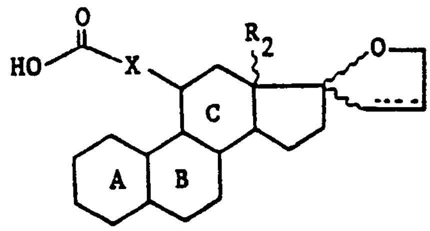

(IJ)

which, if appropriate, is esterified or salified.

12. Process according to claim 10 for the preparation of products of formula (I) characterized in that: the cyclization reagent which is reacted with the products of formula (II), (III) or (V) is tosyl chloride in the presence of pyridine.

13. As medicaments, the products of general formula (I) as defined in claim 1, as well as their pharmaceutically acceptable salts.

14. As medicaments, the products of general formula (I) as defined in claim 2, as well as their pharmaceutically acceptable salts.

15. As medicaments, the products of general formula (I) as defined in any one of claims 3 to 8, as well as their pharmaceutically acceptable salts.

16. As medicaments, the products of general formula (I) as defined in claim 9, as well as their pharmaceutically acceptable salts.

17. Pharmaceutical compositions containing as active ingredient at least one of the medicaments accordiong to any one of claims 13 to 16.

18. As new industrial products, the products of general formula (IV):

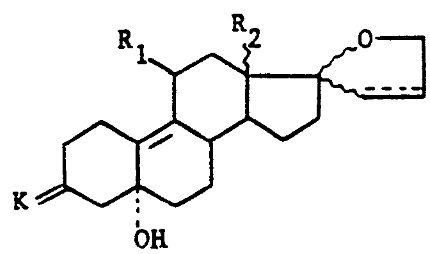

(IV)

in which $R_1$ and $R_2$ retain the same meaning as in claim 1 and K has the same meaning as in claim 10.